(19) Europäisches Patentamt
European Patent Office
Office européen des brevets

(11) EP 2 700 358 A2

(12) EUROPEAN PATENT APPLICATION

(43) Date of publication:
26.02.2014 Bulletin 2014/09

(51) Int Cl.:
A61B 5/1455 (2006.01)     A61B 5/00 (2006.01)

(21) Application number: 13181529.2

(22) Date of filing: 23.08.2013

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 24.08.2012  US 201261692904 P

(71) Applicant: Meditasks LLC
Tenafly, NJ 07670 (US)

(72) Inventor: Andrijauskas, Audrius
06112 Vilnius (LT)

(74) Representative: ABG Patentes, S.L.
Avenida de Burgos, 16D
Edificio Euromor
28036 Madrid (ES)

(54) **Dilution guided correction (DGC) method and apparatus for adjusting noninvasively measured total hemoglobin measurements and method for evaluating hydration state of a subject using the same**

(57) Methods and devices are provided for real time adjustment of noninvasively measured capillary hemoglobin concentration measures for a prediction of invasively measured hemoglobin concentration in large vessels and evaluating and modifying the state of interstitial hydration of an individual by the mVLT method. Implication of mVLT was refined by adding new diagnostic criteria and new variables, also expanding its noninvasive applicability. That opens application in the automated clinical decision support systems, semi-closed and closed loop infusion systems for optimisation of hydration, blood circulation and tissues perfusion, also optimizing blood transfusions.

Fig. 20

$GAP = aHb - cHb$    $\Delta GAP_1 = GAP_1 - GAP_0 = 0$
**POSITIVE GAP**
(a)

$GAP = aHb - cHb$    $\Delta GAP_1 = GAP_1 - GAP_0 = 0$
**NEGATIVE GAP**
(b)

EP 2 700 358 A2

**Description**

**CROSS-REFERENCE TO RELATED APPLICATIONS**

This application claims priority of U.S. Provisional Application No. 61/692,904, filed August 24, 2012, the contents of which is hereby incorporated by reference.

Throughout this application, certain publications are referenced by citation or number in parentheses. Full citations for these publications may be found immediately preceding the claims. The disclosures of these publications in their entireties are hereby incorporated by reference into this application in order to describe more fully the state of the art to which this invention relates.

**Abbreviations**

Hb -- hemoglobin concentration,

IV - intravenous,

mVLT -- mini volume loading test,

PI -- perfusion index,

cHb - noninvasively measured capillary hemoglobin concentration, also referred to as SpHb when it is measured by devices from Masimo Corporation, Irvine, CA, USA,

SpHb - noninvasively measured capillary hemoglobin concentration by using devices from Masimo Corporation, Irvine, CA, USA,

GAP -- difference between hemoglobin concentration in large blood vessels and capillaries,

aHb -- arterial hemoglobin concentration,

vHb -- venous hemoglobin concentration,

Hct - hematocrit,

*f*-ratio -- ratio between hematocrit or hemoglobin concentration in smaller and larger blood vessels,

FAR -- transcapillary fluid filtration-absorption ratio,

PD -- plasma dilution; aPD - arterial, vPD - venous and cPD - capillary,

VLT -- volume loading test,

PDE -- plasma dilution efficacy (defines the ability of a fluid challenge to increase plasma dilution from a preceding fluid challenge); aPDE - arterial, vPDE - venous and cPDE - capillary,

ABP -- arterial blood pressure,

cGAP -- difference between the hemoglobin concentration in an artery and the mean hemoglobin concentration from a mixture of capillaries,

sHb -- micro-capillary hemoglobin concentration,

sGAP -- difference between the hemoglobin concentration in an artery and the mean hemoglobin concentration in micro-capillaries.

mHb -- macro-capillary hemoglobin concentration,

mGAP -- difference between the hemoglobin concentration in an artery and the mean hemoglobin concentration in macro-capillaries.

tGAP -- the tissue perfusion index dependent GAP, which is related to the Fahraeus effect.

fGAP -- the transcapillary fluid filtration absorption ratio (FAR) related GAP.

paHb - the predicted arterial capillary hemoglobin concentration also labelled as the *PI-adjusted.*

ROG -- ratio of GAPs, which is the ratio between the GAP values in consecutive fluid challenges or parameter shifts,

ROP -- ratio of perfusion indexes which is the ratio between the PI in consecutive fluid challenges or parameter shifts,

ROF -- ratio of FAR which is the ratio between the FAR values in consecutive fluid challenges or parameter shifts,

pGAP -- the predicted GAP, which is the difference between the measured capillary hemoglobin concentration (cHb) and predicted arterial hemoglobin concentration (paHb) or venous (pvHb).

pPDE -- the predicted arterial plasma diution efficacy (calculated from predicted aHb).

key-3 -- a set of simultaneously measured capillary hemoglobin concentration, perfusion index and arterial hemoglobin concentration or venous hemoglobin concentration instead of the latter,

key-2 -- a set of simultaneously measured capillary hemoglobin concentration and perfusion index (PI) and arterial hemoglobin concentration or venous hemoglobin concentration instead of the latter.

## BACKGROUND OF THE INVENTION

Measuring blood hemoglobin concentration (Hb) is probably the most frequent routinely administered blood test in a variety of medical fields. Repeatedly administered Hb testing is a common practice during surgery and is indispensable in the management of many nonsurgical medical conditions. It is often a crucial component in subjects such as patients undergoing elective, urgent or emergent surgical or obstetrical procedures, patients undergoing treatment in toxicology units, critically-ill patients, and so on. Intravenous (IV) fluid resuscitation is an integral part of modem medicine practice in a variety of medical fields.

## BRIEF SUMMARY OF THE INVENTION

Fluid administration aims for optimization of the body fluid status. Methods such as mini Volume Loading Test (mVLT) and volume kinetics that are currently used to monitor the intravenous fluid infusion induced changes in body hydration status and distribution of the infused fluid between the compartments of the human body require numerous Hb measures in large blood vessels during the infusions and for some period thereafter. The use of noninvasive point-of-care devices may increase the applicability of these methods and the quality of patient care as a whole. Some of the potential benefits of noninvasive Hb monitoring include hastening the optimal administration if intravenous fluids and diuretic medication, also detecting imminent edema during fluid therapy and monitoring its resorbtion, detection and evaluation of bleeding, transfusion-decision making, preventing the over-transfusion of blood products, reducing phlebotomy-induced anemia in the intensive care area, and increasing patient safety and comfort in all care areas where Hb testing is done. The simultaneous noninvasive real-time monitoring of Hb in both arteries and capillaries can improve the methodology of the mVLT also enabling its applicability in closed or semi-closed loop infusion systems.

Using existing processes, Hb measurement is a routine procedure. It is probably the most frequently obtained blood test in both outpatients and inpatients. Several spectrophotometric techniques - invasive and noninvasive - have conventionally been used for measuring Hb. The CO-Oximeters used in a laboratory require 3 ml of blood, while point-of-care devices need only a drop which is commonly obtained from a fingertip blood draw after a pinprick. Development of spectrophotometric technique on the basis of conventional pulseoximetry enabled noninvasive measuring of Hb in the circulation under a finger nail, a technique named as *Pulse* CO-Oximetry.

**Noninvasive assessment of blood hemoglobin concentration**

The focus of the present invention is the noninvasive measurement of Hb and tissue perfusion index (PI). **We labelled the noninvasively measured Hb as capillary (cHb).** Pulseoximetry is widely used for the monitoring of hemoglobin oxygen saturation, as well as pulse rate and heart rhythm. The pulse oximeter is one of the most commonly used monitors for the measuring of vital signs. Development of this technique involved an increase in the number of light wavelengths {1}. The use of multiple wavelengths enabled the spectrophotometric noninvasive measuring of Hb in the circulation under a finger nail {2}. That technique is based on the modified Lambert-Beer's law which originally describes the absorption of monochromatic light when it passes through a substance. The modification was required because the tissues are not homogenous, and, in contrast to *in vitro* measurements of Hb by invasive techniques, the optical path length changes during noninvasive *in vivo* measurements of Hb by Pulse CO-Oximetry {3}. Aiming for better accuracy and precision of noninvasive measures, the number of wavelengths used in Pulse CO-Oximetry was increased from 2 to as many as 12 in the last decade.

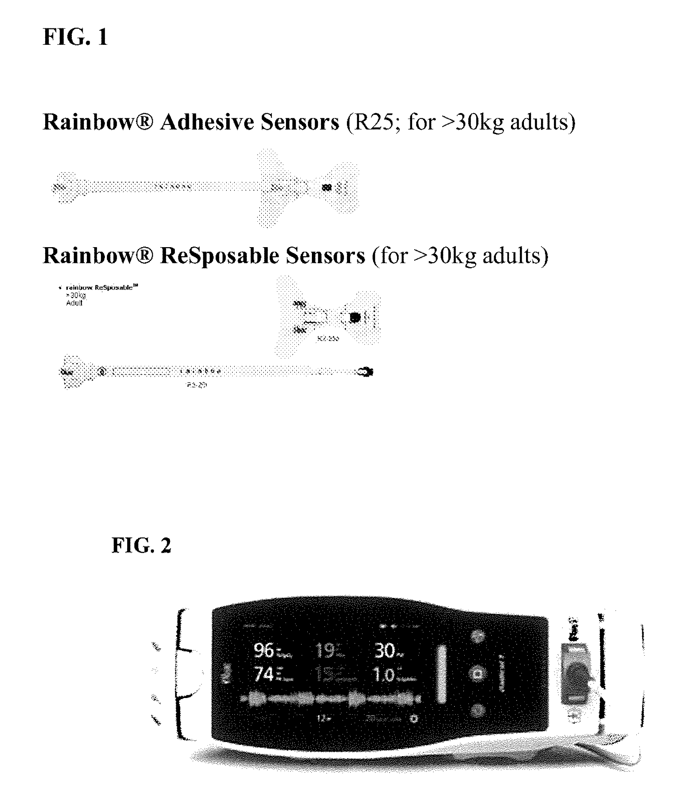

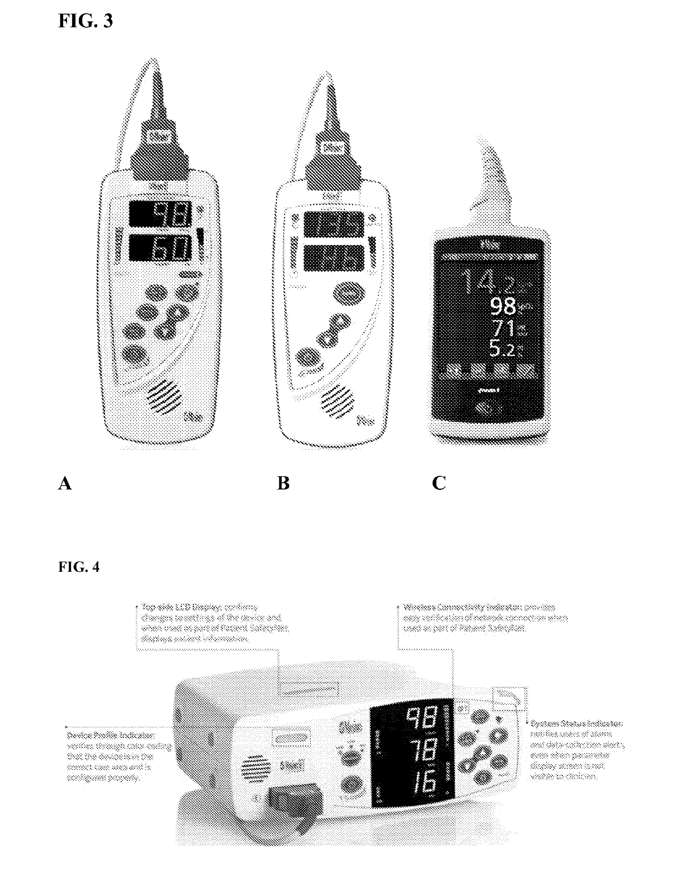

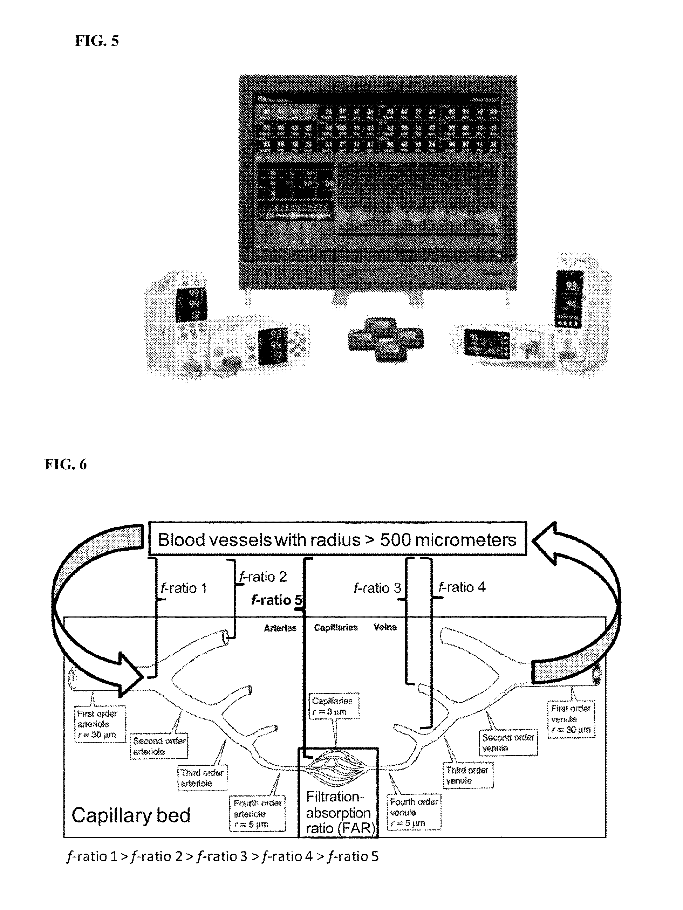

The first commercially available Pulse CO-Oximeter operated on the basis of Rainbow SET® Pulse CO-Oximetry technology, namely the Radical-7® monitor (a registered trademark of Masimo Corporation, Irvine, CA). It allowed non-invasive and continuous measurement of multiple blood constituents, fluid responsiveness, and other physiological parameters that previously required invasive procedures. Based on the Masimo SET® technology (a registered trademark of Masimo Corporation, Irvine, CA), the noninvasive sensor technology used more than 7 wavelengths of light to acquire blood constituent data based on light absorption. Sensors attached to the finger nail are used for continuous noninvasive real time monitoring of PI, cHb and other variables [FIG.1]. The cHb is also referred to as SpHb if it is measured by devices from Masimo Corporation, Irvine, CA, USA (SpHb®, a registered trademark of Masimo Corporation, Irvine, CA). From the introduction of Radical-7 monitor for measuring these variables in 2005, the digital processing algorithms, arterial pulsation filters, light and radiofrequency shielded sensors were continuously improved {2} {4} {5}. Advanced signal processing algorithms and unique adaptive filters work together to isolate, identify and quantify various hemoglobin species. Blood measurement results and other variables are then displayed numerically [FIG.2]. There are several siblings of the Radical-7 monitor which also measure cHb (namely SpHb™) and PI. The upgradable Rainbow® (a registered trademark of Masimo Corporation, Irvine, CA) technology is used in a versatile handheld easy-to-use bedside monitors such as Rad-57®, Pronto® and Pronto-7® [FIG.3], and also the Rad-87® [FIG.4] from Masimo Corporation, Irvine, CA. Many have a built-in radio which allows wireless communication with a remote monitoring and clinician notification system, for example, the Masimo Patient SafetyNet® (a registered trademark of Masimo Corporation, Irvine, CA) [FIG.5].

Other currently available noninvasive multi-wavelength hemoglobinometers include the NBM-200MP™ (a registered trademark of OrSense, Israel) and Haemospect® (a registered trademark of MBR Optical Systems, Germany). The NBM-200MP is based on Occlusion Spectroscopy™ technology (a registered trademark of OrSense, Israel). A ring-shaped sensor fitted on the finger intermittently compresses it with pressure greater than systolic pressure, thus preventing arterial pulsation related errors. NBM-200MP and Haemospect are much less investigated than Radical-7 and other similar devices from Masimo Corporation, but several large studies have demonstrated their similar performance {6} {7}.

Despite technological differences, the three commercially available noninvasive hemoglobinometers - the Pulse CO-Oximeter from Masimo Corporation (Irvine, CA), NBM-200MP™ from OrSense (Nes Ziona, Israel) and Haemospect® from MBR Optical Systems (Germany) - were compared and reported to be similar in more or less clinically *acceptable* accuracy and *unacceptable* precision when the invasively obtained arterial and venous Hb determined by common laboratory analysis (CO-Oximetry) were used as references {6} {7} {10}. The assessment and comparison of accuracy between Pronto-7™ and NBM-200MP reported in 2012 deserves special emphasis {6}. The two monitors were applied in an emergency room on two occasions (2 arms of investigation) in two 'comparable' patient groups consisting of 300 subjects each. In such a relatively big number of observations (1800 paired measures in 600 patients), the accuracy and precision were similar despite differences in technology. Literature reports insist that discrepancy between the invasive and noninvasive measures of Hb may be eliminated by further technological improvements {11}. However, there is also evidence that extrinsic factors such as oxygenation of blood may lead to instability of noninvasive Hb measures {12}. Also, in the evaluation of accuracy and precision of cHb measures, the appropriateness of using the invasively determined large vessel Hb by CO-Oximetry as a reference method raises concerns. That is because there is an inherent physiologic difference between Hb in large and small blood vessels (GAP), and the sensor for measuring cHb is typically only used to examine the capillary beds of derma under the finger nail.

However, the literature reports of more or less clinically accuracy and 'unacceptable' precision of the cHb measures in predicting the large vessel Hb are challenging the measured parameters' clinical reliability, and especially for evaluation of blood loss and transfusion decision making {4} {5}. Thus, despite continuous improvement of the digital processing algorithms, arterial pulsation filters, light and radiofrequency shielded sensors, the unpredictable discrepancy between invasive and noninvasive Hb measures remains a serious limitation in the use of noninvasive measures for the prediction of large vessel Hb values. As a result, there is a continuing search for a method of minimizing the GAP.

Both invasive and noninvasive Hb measurements have inherent variability. Additionally, there is a variety of physiologic and methodologic factors that can significantly influence Hb in the body. Physiologic factors such as the blood source (venous or arterial), site and time of blood draws, and patient body position are recognized in the clinical literature to add variability to hemoglobin levels. Vascular physiology affects Hb measurements. Specifically, measurements vary depending on whether the measurement is taken from venous or arterial blood or whether the measurement is taken from a capillary bed in a patient's finger. This is because the physiology of the capillaries will affect the Hb measurement in a way that is not present in a venous or arterial blood draw. Thus, noninvasive Hb measurement will not exactly match the measurement taken from a blood draw because the noninvasive devices are measuring the Hb in blood pulsing through a capillary bed, usually under a finger nail. As discussed above, Hb measurements through a capillary bed will differ, sometimes significantly, from Hb measurements taken from venous or arterial blood. Thus, a laboratory Hb analyzer can have a different reading from the noninvasive device and yet both devices could be entirely correct. The difference in the readings could be due to the fact that the devices are analyzing blood from different vascular types (arterial vs. capillary) and thus would report different readings for the described physiological reasons. It should be noted, that the controlled exchange of blood electrolytes, water and proteins with the surrounding tissues takes place in capillaries, and fluxtuations in noninvasively measured Hb values reflect the transcapillary balance of these constituents. The laboratory analysis of Hb in the blood obtained from a microcuvette after a pinprick in a fingertip can also show a different value as compared to venous or arterial Hb obtained via a standard blood draw from an artery or vein. This is possible because a local vasomotor response of a capillary bed to a pinprick is the stimulation of arteriolar sphincters which results in an immediate restriction of blood flow to the smallest capillaries that have the lower Hb as compared to larger vessels. This is why the blood that forms a blood drop obtained from a fingertip which enters the microcuvette lacks the fraction with the highest Hb difference with respect to large vessel Hb, which is present in cHb measurements. Moreover, to facilitate the formation of a blood drop, manual squeezing of arteries on both sides of the finger (also known as "milking") is commonly done, and thus more blood from relatively large vessels enter the blood drop. Thus, the GAP can be due to the physiology of the vasculature system or even error in the blood analysis devices, which have their own error ranges.

Recently, the discrepancy between SpHb and arterial (aHb) or venous (vHb) hemoglobin concentration was explained by the Fahraeus effect {13}. It implies that in blood vessels with diameters less than 500 micrometer the hematocrit (Hct) decreases with a decreasing capillary diameter {14} {15}. The Fahraeus effect affects Hct and Hb similarly when mean cell hemoglobin content (MCH) is constant. The ratio between Hct or Hb in smaller and larger blood vessels is defined as *f*-ratio. Thus, depending on the inner radius of a blood vessel [FIG.6], the Hb in different size capillaries under the SpHb sensor may have individual negative deviation from Hb in larger vessels (positive GAP). This explains a tendency of SpHb to underestimate the invasively measured aHb and venous Hb. The GAP decreases during hemodilution and loss of red cells, because Hct in smallest capillaries is already critically low and thus cannot decrease when Hct in large vessels decreases. That explains the findings of increased accuracy of SpHb measures at low Hb levels when invasive Hb measures are used as a reference method {11}. However, the Fahraeus effect does not explain why there is a tendency of overestimation reported at low arterial Hb levels {18}. Reference {19} explained how the cHb can be affected by transcapillary fluid filtration-absorption ratio (FAR) in the capillary beds under the sensor, and proposed a method - the mini volume loading test (mVLT) - to assess and optimise the body hydration status by evaluating changes of Hb in response to consecutive *mini fluid challenges* - relatively small volume intravenous crystalloid boluses (1.5 - 2.5 ml kg$^{-1}$) followed by 5 min periods without fluid. The method requires serial measures of arterial or at least venous Hb, and especially cHb. A Clinical study to validate the mVLT method showed the significantly increasing gap between the deviations of SpHb and arterial Hb during 20 min. period without fluid after the last (third) pre-operative fluid challenge, and hypothesized that it was caused by the decrease of FAR in the capillary bed under the sensor {20}. However, the Fahraeus effect related changes in cHb were ignored by that new method. Moreover, the PI can have a significant impact on the cHb measures.

The PI is the ratio of the pulsatile blood flow to the non-pulsatile or static blood in peripheral tissue. Thus, PI represents a noninvasive measure of peripheral perfusion that can be continuously and noninvasively obtained from a pulse oximeter. It is an assessment of the pulsatile strength at a specific monitoring site (e.g. the hand, finger or foot), and as such PI is an indirect and noninvasive measure of peripheral perfusion. It is calculated by means of pulseoximetry by expressing the pulsatile signal (during arterial inflow) as a percentage of the non-pulsatile signal, both of which are derived from the amount of infrared (940 nm) light absorbed {2}. The measurement of PI is independent of other physiological variables such as heart rate variability, SaO$_2$ or oxygen consumption. The PI trend reveals often subtle changes in perfusion that are otherwise missed by static displays. These subtle changes captured by the trend provide immediate clinical feedback of the efficacy of anesthesia, analgesia, and/or therapeutic intervention {8}. Changes in PI can also occur as a result of local vasoconstriction (decrease in PI) or vasodilation (increase in PI) in the skin at the monitoring site. These changes occur with changes in the volume of oxygenated blood flow in the skin microvasculature {9}. Obviously, the changes of PI affect the cHb measures even because of its sensitivity to changes in capillary radius and consequently *f*-ratio [FIG.6] which in turn leads to changes in the Fahraeus effect related gap between Hb in large and small vessels. Thus, there is no surprise that during crystalloid infusion in healthy volunteers the bias of SpHb measurement decreased with increasing PI and *vice versa* when the invasively measured venous Hb was used as a reference method {18,21}.

Aiming to increase the accuracy and especially precision in predicting arterial and venous Hb from noninvasive Hb measures, the focus has recently shifted from improving the measuring techniques to the mathematical adjustment of cHb values. A simplistic solution namely the *In Vivo* Adjustment of SpHb implies entering the invasively measured Hb value was introduced on the Radical-7 in year 2012 {16}. According to a single related report in literature so far {17}, such calibration has lowered a gap between SpHb and arterial Hb, and it decreased further with decreasing Hb values during durgery. However, as described by the Fahraeus effect earlier in the text, the gap could have decreased because of hemorrhage and hemodilution. Morover, the *In Vivo* adjustment cannot eliminate the impact of unpredictably changing transcapillary fluid equilibration that affects haemodilution in capillaries and consequently makes the arterio-capillary and veno-capillary Hb differences unstable. The transcapillary fluid equilibration is an extremely intense and dynamic process because about 95% of fluid exchange between the blood and body tissues takes part in capillaries while only about 3 - 5% of the body blood volume is in capillaries. That causes the difference between arterial and venous Hb shift from positive to negative during and immediately after the intravenous infusion of a crystalloid {22}. For the same reason, an option of *arterial* or *venous* mode of measuring SpHb in the Radical-7 monitor is misleading, because it is based on the mathematical conversion of arterial Hb into venous and *vice versa.* That conversion is based on the preliminary findings by Mokken et. al. {23} and Yang ZW et. al. {24} that arterial Hb measurements can be expected to be, on average, 0.7 - 1.0 g/dL less than the Hb measurements derived from venous blood.

Mathematical optimization is a common technique used for the improvement of the accuracy and presission in various parameters measuring devices. As big as possible pool of key variables obtained in as big as possible pool of observations and number of individuals should be used for calculating the coefficients of the polynomials so that they become more 'universal'. Following the objective to investigate if the mathematical optimization works for SpHb values when polynomials are derived from a relatively small pool of statistical data, we developed the *statistically biased calibration method* {25}. We have put that method into test by applying it retrospectively to SpHb, PI and arterial Hb data from our ongoing randomized clinical trial in total knee athroplasty patients who received perioperative stepwise crystalloid infusions. That method efficaciously reduced the gap between the invasive arterial Hb and SpHb. However, although mathematical optimisation reduces the individually occuring gap between the invasive and noninvasive Hb measures, there will always remain a space for individual incompliance with the established average statistical behaviour because of the inherently unpredictable and sometimes extremely severe physiological perturbations that occur in microcirculation, and especially during major surgery and critical illnesses.

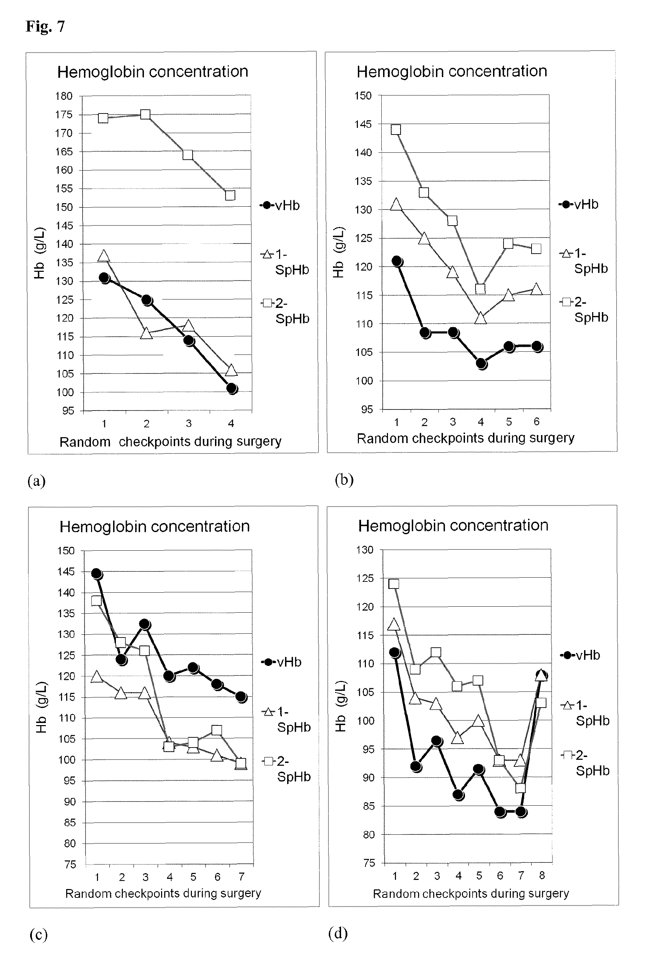

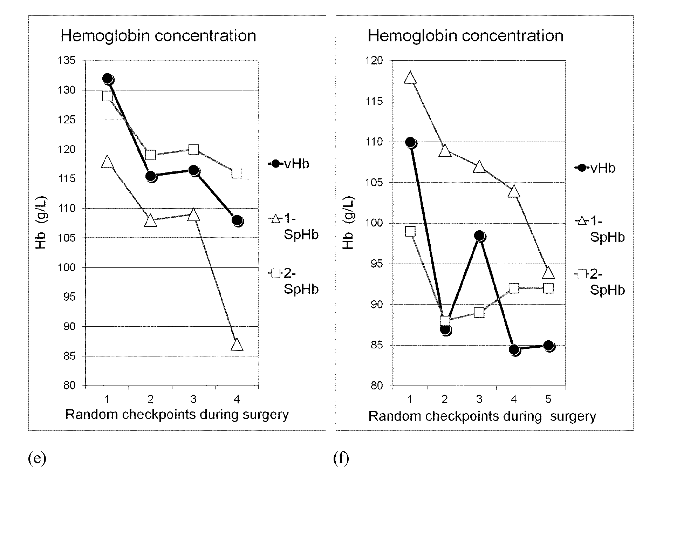

A kind of mathematical optimisation was presumably applied in the development of the new generation of SpHb sensors - namely the *k* version - where the statistical average gap between SpHb and invasive Hb measures, also the relationship between changes in PI and SpHb could probably have been taken into account. These sensors came into market in 2013. In our pilot clinical observations [FIG.7] we compared the venous Hb measured from a venous blood draw by a point-of-care device (HemoCue®, Sweden) and SpHb that was measured by using two versions of sensors - the ReSposable™ R2-25 (Masimo Inc., Irvine, CA). These sensors were attached to the nails of adjacent fingers in patients undergoing major elective orthopedic surgery. Sensor versions *k* (1-SpHb; most recent edition, 2013; software version 7.9.1.0) and g (2-SpHb; earlier edition, 2012 software version 7.6.2.1) were connected to separate Radical-7 Pulse CO-Oximeters (Masimo Inc., Irvine, CA). The *in vivo* adjustment mode for the calibration of Radical-7 devices was not deployed. Invasive measures were performed in duplicates, and the mean value is shown in the trends. Looking at the individual patient records, sometimes sensor version *k* appears to be clinically acceptably accurate and precise (deviation from invasive value is < 10 g l$^{-1}$ in all checkpoints; FIG. 7a,d) while version g is not. However, sometimes it is on the opposite, and version g appears to be clinically acceptably accurate and precise [FIG.7e] while version *k* is not. Although in other patients version *k* appears to be more accurate and precise than version g [FIG.7a,b] or similar [FIG.7c,d,f], both versions are not clinically acceptably accurate and precise in these cases taking into account all checkpoints that belong to the same patient.

In conclusion, there is a justified necessity to draw the clear separating line between the Hb in large blood vessels and in the micro-vasculature under the noninvasive sensor {13}. The individual minimization of Hb gap between the invasive and noninvasive measures can probably be most efficient by applying the mathematical modeling of the anatomically and physiologically defined difference between Hb in capillaries and large vessels {19,26}. Aside from speculations about the possible statistically-derived mathematical adjustment of SpHb measures in the latest edition of Masimo's sensors, our own example of mathematical optimisation {25} suggests that individual adjustment of SpHb values for the better prediction of large vessel Hb should be focused on individual calibration of a device based on the evaluation of actual relationship between cHb and PI during their spontaneous or induced simultaneous deviations, e.g. during deviations induced by a small IV fluid bolus.

However, despite continuous improvement of techniques the unpredictable discrepancy between the invasive and noninvasive hemoglobin measures remains a serious limitation in the use of noninvasive measures for the prediction of large vessel hemoglobin values. That is because: (a) Noninvasive devices are measuring the hemoglobin in blood pulsing through a capillary bed, usually under a finger nail. Vascular physiology affects the capillary hemoglobin concentration in a way that is not present in larger vessels. Thus, noninvasively measured values will not exactly match the measurement made from a blood sample from larger vein or artery. (b) The discrepancy between invasive and noninvasive hemoglobin concentration measures exists due to Fahraeus effect, also because hemodilution in capillary beds under the noninvasive sensor is unstable due to constantly changing transcapillary fluid filtration absorption ratio and fluctuation of capillary radiuses (reflected by perfusion index). (c) Although mathematical optimisation which is a common technique used for the improvement of the accuracy and precision in measuring devices, reduces the individually occurring gap between the invasive and noninvasive hemoglobin measures, there is an individual incompliance with the established average statistical behaviour because of unpredictable and sometimes severe physiological perturbations that occur in microcirculation under the noninvasive sensor. (d) All currently commercially available devices for noninvasive hemoglobin measures are reported to be similar in more or less clinically acceptable accuracy and unacceptable precision when the invasively arterial and venous hemoglobin measures are used as references.

**Assessment of the body fluid status from the test fluid load induced changes in hemodilution**

Intravenous fluid therapy is part of the everyday clinical practice in many fields of medicine, and especially in the perioperative period. Administration of isoosmotic crystalloid solution (crystalloid), which is inherently the hydrating fluid {28}, may have different clinical goals. For example, crystalloid can be a first choice fluid, e.g., for the treatment of dehydration, or it can be a second choice fluid, e.g., for the volume recovery after hemorrhage. Crystalloid solution is also a media for delivery of intravenous medications such as cardiovascular drugs, antibiotics and anaesthetics to target tissues. Achieving and maintaining a target plasma dilution is of major importance in total intravenous anaesthesia and target controlled infusion where the constant target plasma concentration of a drug is crucial. However, the crystalloid infusion induced plasma dilution cannot be reliably predicted from the chemical and physical features of a solution and the physiology of body water balance and fluid distribution among functional fluid compartments. In addition to the balance of fluid intake and net elimination, intravascular fluid retention is affected by a transcapillary fluid filtration-absorption ratio (FAR) {29} and lymphatic influx into central veins {30}. The FAR is affected by changes of net transcapillary pressure, and the lymphatic flow is affected by changes of interstitial hydrostatic pressure {31}. Consequently, both pressures are affected by interstitial fluid accumulation. Interstitial expansion by fluids leads to increasing compliance and progressive fluid accumulation until the anatomical limits are reached. When the interstitial fluid compliance is maximized (virtually infinite in overhydration), the lymphatic flow does not increase anymore, because hydrostatic pressure remains constant despite the further accumulation of interstitial fluid with a threat of imminent edema. Thus, even if fluid elimination is not activated, the hemodilution efficacy of a crystalloid tends to decrease during crystalloid infusion because of increasing interstitial fluid compliance. If the infusion is targeted to reach and maintain proper hemodilution or improve hemodynamics, there is a threat of interstitial fluid overload (edema). Edema formation deteriorates the turnover of plasma albumin in the lymphatic loop as a result of interstitial trapping of fluids and proteins {32}. That lowers the plasma protein content, leading to a decrease in plasma volume {33}. Edema prevents the effective delivery of drugs to target cells in swollen tissue and deteriorates the elimination of drugs and metabolites. Thus, for efficacy and safety reasons, any goal directed crystalloid infusion should be accompanied by real time monitoring of interstitial fluid accumulation with the well established panic signs for the detection of imminent edema. However, only the extreme deviations from normal interstitial hydration are detectable in the current clinical practice {34}. That leads to the life-threatening complications such as pulmonary edema {35}.

Inadequate intravenous fluid administration is an ongoing clinical concern. Thus, there is an obvious need for a relatively simple and reliable bedside method to assess the body hydration status. Assessment of the body fluid status and fluid demands from the test fluid load induced changes in non-invasively measured target parameters is attractive. A range of static and dynamic flow related parameters such as cardiac stroke volume and its variation, respectively, is used for that purpose in conventional goal directed fluid therapy {27}. Static hemodynamic parameters specifically reflect the fluid status, while dynamic measures provide a prediction of cardiovascular response to the fluid load before it is administered.

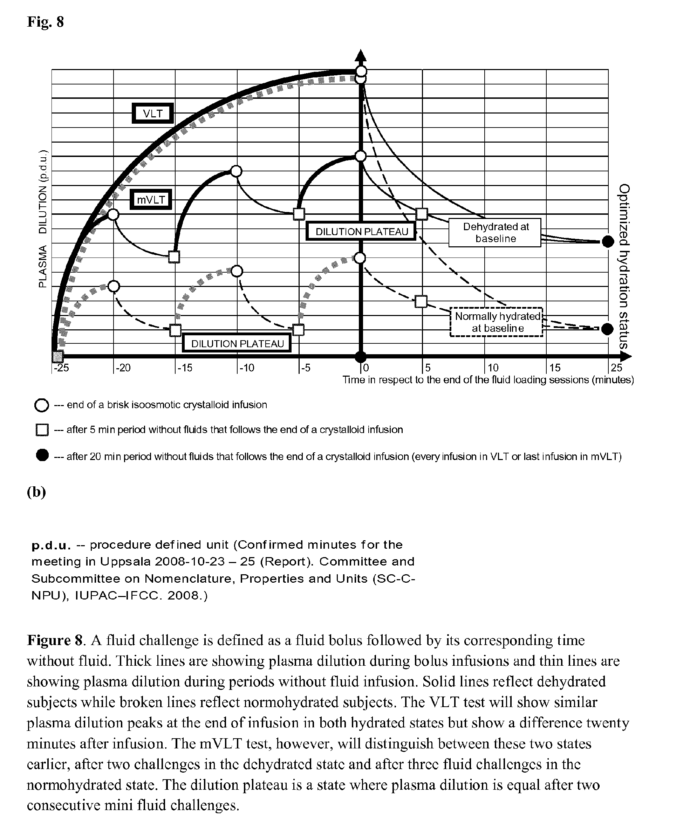

Two methods were proposed for the evaluation of fluid status by investigating the hemodilution response, which is the plasma dilution (PD) trend obtained during consecutive fluid challenges that consist of a crystalloid bolus followed by a period without fluid. It is used by the mVLT method {19} which is a development of a previously published method, the volume loading test (VLT) {19,36,37}. Systematic reviews of randomized clinical studies found no evidence to recommend one type of fluid therapy over another for the rational fluid administration applicable in most clinical settings {38}. Increasing patient safety concerns related to colloids suggests using crystalloids for the mVLT purposes. Both the mVLT and VLT are based on the concept that changes in PD induced by a crystalloid fluid challenge are more pronounced when the impact of net body fluid elimination and net interstitial fluid accumulation on intravascular fluid retention is low, such as when the patient is dehydrated [FIG.8]. The PD is calculated from a mini fluid challenge induced change in Hb.

Because we are considering dilution of plasma, we need to adjust for the hematocrit (Hct):

$$PD_i = (Hb \cdot Hbi_i^{-1} - 1) \cdot (1 - Hct)^{-1} \qquad (1)$$

where $PD_i$ is *plasma dilution* after the fluid challenge with number *i*, Hb is the *initial hemoglobin concentration* value obtained before the first fluid challenge, $Hb_i$ is the hemoglobin concentration value obtained after the fluid challenge number *i*, and Hct is the *initial hematocrit* value obtained before the first fluid challenge (since noninvasive hematocrit is not available, during noninvasive determination of PD, the initial hematocrit value is derived by dividing the noninvasive initial hemoglobin concentration by 330 which is the mean value of the normal range for mean cell hemoglobin concentration).

The main differences between the mVLT and the VLT are as follows: (a) the VLT uses crystalloid boluses larger than those used in the mVLT (7.5 vs. 2.5 - 5.0 mL·kg⁻¹), (b) the period without fluid following a bolus is at least four times longer in the VLT (20 min vs. 5 min), and (c) the mVLT allows for both evaluation and management of hydration status while the VLT is used for evaluation only. Despite different baseline hydration status, the VLT test yields similar PD at the peak but shows a difference 20 minutes after the end of infusion. This is because the peak PD is dependent on the volume status while residual PD after 20 minutes after the end of infusion is hydration status dependent {37}. Dehydrated subjects will show a higher plasma dilution 20 minutes after the end of infusion. In the mVLT test, similar PD differences will also be detected at the end of the experiment but differences can be detected earlier due to the repetitive boluses with 5-minute intervals without fluids. This is achieved by detecting a "dilution *plateau"* where the mini fluid challenge does not induce a further increase in PD. For those subjects with a higher hydration status (referred to as 'normally hydrated'), the dilution *plateau* is detected earlier, in this figure [FIG.8] it was arbitrary detected after the second mini fluid challenge. For those subjects with a lower baseline hydration status (arbitrarily referred to as 'dehydrated') this occurs during the third fluid challenge. A difference between aPD measurements before and after the mini fluid challenge also the related aPDE variable reflects the rate of intravascular fluid retention during the acute phase of fluid distribution and elimination in the entire body. Thus, we refer to this as the whole-body hydration responsiveness. Clinical studies to validate the mVLT method showed its ability to discriminate between different states of body hydration in healthy volunteers {39} and patients {40}. There is some theoretical and clinical evidence that noninvasively measured Hb should be interpreted as capillary hemoglobin with a potential application for monitoring the interstitial hydration status via changes in transcapillary fluid movement {26} {41}.

However, assessment of the body fluid status and the mVLT method has several drawbacks. The plasma dilution trends *per se* are not reliable for the detection of optimization during mVLT fluid protocol. The reliable mVLT application requires invasive Hb analysis. The noninvasive Hb measures are not suitable for monitoring changes in transcapillary fluid movement. That is because: (a) Comparison of PD after repetitive fluid boluses separated by only a few minutes cannot fully reflect differences in intravascular fluid retention because dilutions overlap. (b) The *dilution plateau* as marker of optimisation can be missed because its detection requires equal Hb and thus PD values before and after a mini fluid challenge and this requires an Hb measuring device with both high accuracy and precision. Also the dilution plateau can be missed even because it is an unstable transitory state. (c) The noninvasive Hb measures cannot reliably predict Hb in large vessels. Thus, reliable mVLT application requires invasive Hb analysis. (d) The noninvasive Hb measures are affected by both - the transcapillary fluid movement and the perfusion index. (e) Although referring to noninvasively measured Hb as capillary opens a theoretical possibility for monitoring the transcapillary fluid movement and detection of imminent edema, an impact of PI on capillary Hb readings has to be eliminated so that FAR would be the only unstable factor in the discrepancy between Hb in large vessels and capillaries. (f) The clinical interpretation of the noninvasively measured Hb should be based on referring to it as capillary hemoglobin.

**Clinical decision support devices and automated infusion systems**

Despite improvements in techniques and skills, the outcome of treatment after major surgery remains highly dependent on the overall perioperative optimisation of the patient. Optimisation procedures are labour-intensive, stressful to care providers and require simultaneous monitoring of multiple parameters. Nevertheless, optimisation targets may still be missed. Closed loop systems are the ultimate solution to ensure that optimal therapies are delivered in a timely manner {42}. The objectives such as keeping the perioperative arterial blood pressure (ABP) and optimised haemodilution in the preset safe limits can be achieved both automatically and semi-automatically. The mathematical modelling cannot account for all the environmental factors, but it may be helpful for clinicians in dealing with the complxity of decision-making and applying treatment in a timely manner. Integrating patient monitoring, fluid and drug infusion devices into one system and using a controller, which would be able to assess the requirements for a specific treatment according to patients' vital signs can make it possible to reduce the probability of hazards arising from the lack of appropriate timely reaction. Controllers such as PID, Fuzzy logic, Artificial neural networks, Rule-based, Model-based and others can be used to fit the specific requirements.

Rational perioperative fluid management is estimated to contribute to annual prevention of 3,000,000 post-operative complications and 800,000 deaths worldwide for high-risk surgical patients {43}. Goal-directed fluid therapy has been shown to improve outcome but is only used in 16% of high-risk surgeries {44}. This is mainly because it requires administration of fluid challenges followed by immediate evaluation of the target parameter's deviation (response), which is labour-intensive, time-inefficient and stressful to personnel. Thus, there is an obvious need for an automated decision support systems {45} consisting of devices that measure vital parameters, analyse them and propose treatment on the basis of the preprogrammed clinical algorithms. Such is the vital signs monitor EV1000 from Edwards Lifesciences Corporation (http://www.edwards.com/products/mininvasive/Pages/ev1000.aspx). Such decsion support systems can be easily transformed into semi-closed loop and closed-loop systems, e.g. closed-loop system for the fluid management and hemodynamic optimization {42}. If hemodynamic and plasma dilution parameters are measured simultaneously and automatically analyzed in real time during fluid challenges in goal directed fluid management, the use of mVLT methodology would enable the evaluation of plasma dilution response in parallel to conventional evaluation of the hemodynamic response. That would provide a wider clinically relevant information about the appropriatness of fluid administration.

The most specific target parameters used in the conventional goal directed fluid management, e.g., cardiac stroke volume, have limited availability and applicability in the everyday clinical practice. Perioperative patient optimisation at minimum requires maintenence of normal ABP. This can be achieved by applying continuous ABP monitoring and administration of fluid infusion and medication in case of arterial hypotension. A closed loop vasopressor drug injection to treat arterial hypotension system has been recently put to the test during anaesthesia and surgery {46}. It used a continuous noninvasive arterial pressure measurement device (CNAP™; Draeger Medical, Germany) connected to a laptop computer. When CNAP fell below a pre-set limit, the computer automatically delivered injection commands to the two syringe pumps pre-filled with vasopressors. The applicability of mVLT in the decision support devices and automated or semi-automated infusion systems is dependent on the possibility to reliably monitor the plasma dilution by noninvasive devices.

However, clinical decision support devices and automated infusion systems have limited clinical applicability and, even using them, the patient optimization targets are frequently missed. That is because: (a) Automated clinical decision support systems are focused on hemodynamic optimization by IV fluid loading, usually implementing goal directed therapy algorithms, while the related changes of body hydration status are not monitored or reliably evaluated. (b) The most specific target parameters used in goal directed fluid management have limited availability and applicability in the everyday clinical practice. (c) Fluid administration guided by hemodynamic response can lead to the deleterious swelling of tissues (edema).

The present invention provides solutions to those deficiencies identified under the "Prior Art" in the BACKGROUND section of this application. The present invention is focused on the mathematical adjustment of the noninvasive capillary hemoglobin (cHb) measures by multi-wavelength spectrophotometric technique in soft tissues under the sensor for the improvement of its accuracy and precision for the prediction of hemoglobin concentration (Hb) in large vessels, and its use for the monitoring of changes in transcapillary movement of fluids under the sensor during the mini volume loading test (mVLT), also defining the applicability of mVLT in automated and semi-automated infusion systems by using the same.

The present invention may be relevant to testing and patient optimisation in, for example, patients undergoing major elective or emergency surgery and obstetrical procedures, major surgery or organ transplantation, patients with preexisting disorders of fluid turnover or acquired fluid deficiency states secondary to burns, bleeding or ileus and critically-ill patients.

The present invention applies to both inpatient and outpatient surgical or hematology settings, and to procedures performed in operating rooms, intensive care units and other locations (e.g., interventional radiology or surgery wards) where testing blood hemoglobin content and administration of fluid replacement are indicated. This new invention is directly applicable to the care administered by family doctors, hematologists, anesthesiologists, intensive care doctors, surgeons and individuals who deliver care under their medical direction or supervision.

Despite continuous improvement of noninvasive techniques, the unpredictable discrepancy between the invasive and noninvasive Hb measures remains a serious limitation in the use of noninvasive measures for the prediction of large vessel Hb values. That is because noninvasive devices are measuring the Hb in blood pulsing through a capillary bed, usually under a finger nail. Vascular physiology affects the capillary Hb in a way that is not present in larger vessels. Thus, noninvasively measured Hb values do not exactly match the Hb measurement made using a blood sample from a larger vein or artery. The discrepancy between invasive and noninvasive Hb measures exists due to Fahraeus effect, also because hemodilution in capillary beds under the noninvasive sensor is unstable due to constantly changing FAR and fluctuation of capillary radiuses (reflected by PI). Although mathematical optimisation which is a common technique used for the improvement of the accuracy and precision in various measuring devices, reduces the individually occurring Hb gap between the invasive and noninvasive measures, there is an individual incompliance with the established average statistical behaviour because of the unpredictable and sometimes severe physiological perturbations that frequently occur in microcirculation under the noninvasive sensor. All currently commercially available devices for noninvasive hemoglobin measures are reported to be similar in more or less clinically acceptable accuracy and unacceptable precision when the invasively arterial and venous Hb measures are used as references.

Assessment of the body fluid status and the mVLT method has several drawbacks. The PD trends *per se* are not reliable for the detection of optimization during mVLT fluid protocol. That is because comparison of PD after repetitive fluid boluses separated by only a few minutes cannot fully reflect differences in intravascular fluid retention because dilutions overlap. The *dilution plateau* as marker of optimisation can be missed because its detection requires equal Hb and thus PD values before and after a mini fluid challenge and this requires an Hb measuring device with both high accuracy and precision. Also the dilution plateau can be missed even because it is an unstable transitory state. The reliable mVLT application requires invasive Hb analysis because the noninvasive Hb cannot reliably predict the large vessel Hb. Noninvasive Hb measures are affected by both - the transcapillary fluid movement and the perfusion index. Thus, although referring to noninvasively measured Hb as capillary opens a theoretical possibility for the monitoring the transcapillary fluid movement and detection of imminent edema, an impact of PI on capillary Hb readings has to be eliminated so that FAR would be the only unstable factor in the discrepancy between Hb in large vessels and capillaries. This is because the Fahraeus effect related gap is constant.

Clinical decision support devices and automated infusion systems have limited clinical applicability and, even using them, the patient optimization targets are frequently missed. That is because automated clinical decision support systems are focused on hemodynamic optimization by IV fluid loading, usually implementing goal directed therapy algorithms, while the related changes of body hydration status are not monitored or reliably evaluated. The most specific target parameters used in goal directed fluid management have limited availability and applicability in the everyday clinical practice. Fluid administration guided by hemodynamic response can lead to the deleterious swelling of tissues (edema) that negatively affect the overall outcomes of treatment.

The present invention relates to a mathematical model for individual discrimination between the components of discrepancy between arterial hemoglobin concentration (aHb) and cHb where transcapillary fluid filtration-absorption ratio (FAR) component is on one side and tissue perfusion and Fahraeus effect related interferring components are on the other side, also assessment of changes in transcapillary fluid movement in response to administration of fluid or diuretic therapy, also providing guidelines for their individualized administration.

The present invention includes a method and apparatus for determining the predicted arterial hemoglobin concentration (paHb) value from noninvasive mesures of cHb, also determining the changes in transcapillary fluid movement from the dynamics of cHb and paHb after an intake or IV administration of fluid or a diuretic, or vasoactive medication such as norepinephrine, or during series of test volume loads of crystalloid solutions in mVLT. The apparatus can also automatically (closed loop system) or semi-automatically (semi-closed loop system) guide administration of fluids and diuretic medication targeted to achieve or maintain a specific pattern of transcapillary fluid movement.

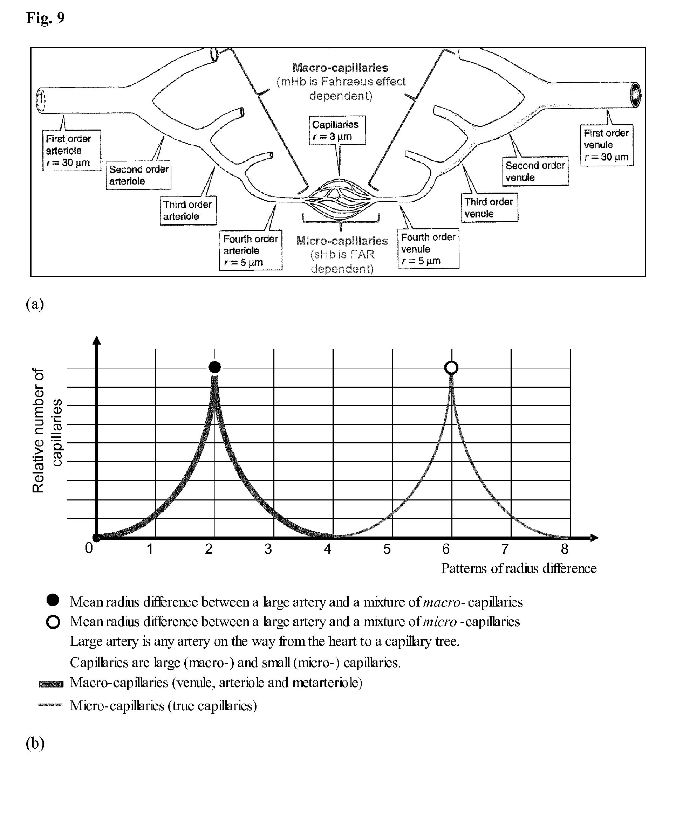

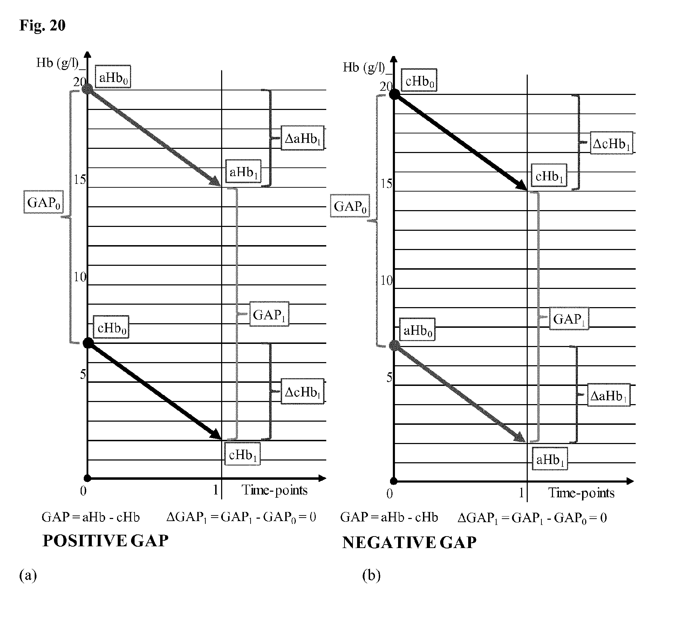

Addressing deficiencies within existing methods, the current invention proposes the **Dilution Guided Correction method (DGC method).** It consists of three models and the **calibration protocol.** A physiology based **dynamic gap model** is used as background for the mathematical modelling of changes in GAP defined by equations the **DGC-math model.** The dynamic gap model explains the anatomical and physiological origin of the discrepancy between the Hb in capillaries and large vessels [FIGS.9-19]. According to the present invention, the GAP consists of the systemic GAP which is related to the Fahraeus effect and the dynamic GAP which is related to changes of PI [FIGS.20-24]. The Hb and PI data obtained during the semi-invasive or noninvasive calibration protocol are then being processed by equations of the DGC-math. The semi-invasive calibration requires two noninvasive measures of cHb and PI and two invasive arterial or venous blood Hb measures for the calculation of actual GAP. This GAP is then used in the equations of DGC-math for the prediction of large vessel Hb in the upcoming monitoring period only for the same individual. Meanwhile, noninvasive calibration requires only two noninvasive measures of cHb and PI because the GAP value derived by statistical mathematical optimization method instead of actually measured will be used for the predictions of noninvasive readings. Elimination of the PI related deviation of cHb in the upcomming readings is achieved by using the mathematically determined individual relationship between simultaneous deviations of PI (ΔPI) and cHb (ΔcHb) made during the callibration. After eliminating the GAP and the impact of PI, the large vessel Hb is predicted from noninvasive measures with high precision and accuracy.

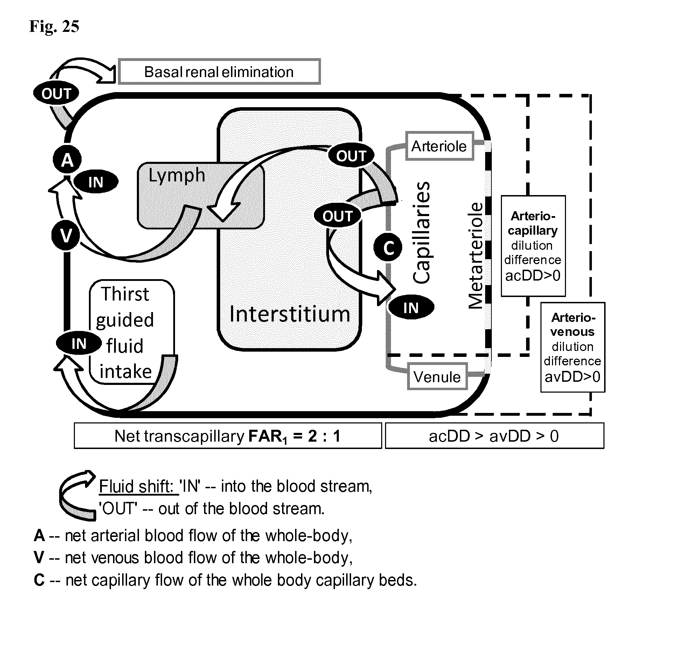

The minimization of the PI related fluctuations of cHb value enables the semi-invasive or noninvasive monitoring of changes in transcapillary fluid movement and detection of imminent edema during mVLT. The mVLT semi-invasively or noninvasively detects imminent edema during mVLT and other protocols of stepwise fluid infusion, also evaluates the efficacy of diuretic medication from changes of hemodilution in large vessels and capillaries. After the above described adjustment of cHb readings, the difference between the pre- and post adjustment cHb values is solely FAR dependent. Thus, changes in the difference between the cHb and adjusted cHb (paHb -- predicted arterial hemoglobin) reflects changes in FAR which in turn is a reflection of transcapillary fluid balance and tissue hydration status. The current invention proposes a **transcapillary reflux model** based on known physiology to explain the clinical relevance of variables derived from the above models [FIGS.25-27].

The DGC method deploys **calibration protocol** which is procedure applied for the calibration of a device configured to noninvasively measure cHb. The calibration needs to be applied only once if the sensors will stay in the securely fixed location during the cHb monitoring period thereafter.

The **semi-invasive calibration protocol** requires two noninvasive measures of cHb and PI and two invasive arterial or venous blood Hb measures. It implies evaluation of a single simultaneous change in PI, cHb and aHb or vHb that occurs spontaneously or is induced, e.g. by a mini fluid challenge during mVLT. A set of equations from the **DGC-math** is then used to evaluate the actual individual relationship between the deviations of the key-3 parameters measured during the calibration. The **non-invasive calibration protocol** requires only two noninvasive measures of cHb and PI. It implies evaluation of a single simultaneous change in PI and cHb that occurs spontaneously or is induced, e.g. by a mini fluid challenge during mVLT. That data are used thereafter to mathematically derive the PI-adjusted cHb value which is a prediction of aHb or vHb (paHb and pvHb, accordingly) in the period of observation observation after the calibration.

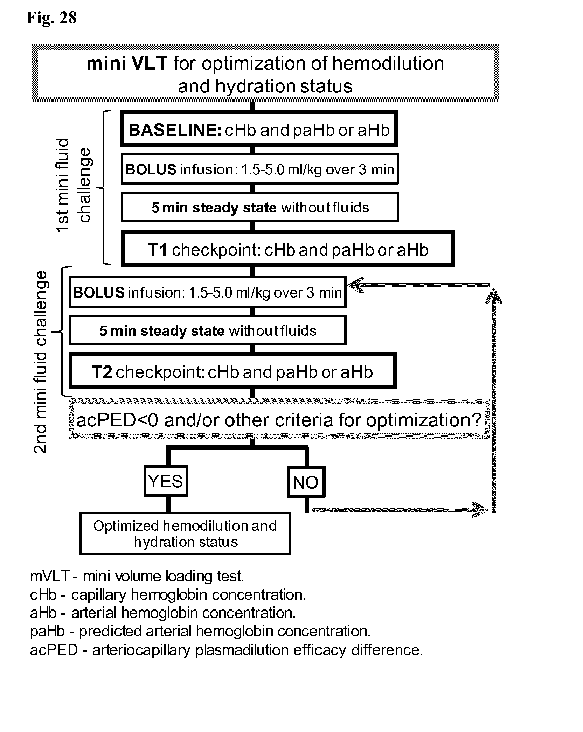

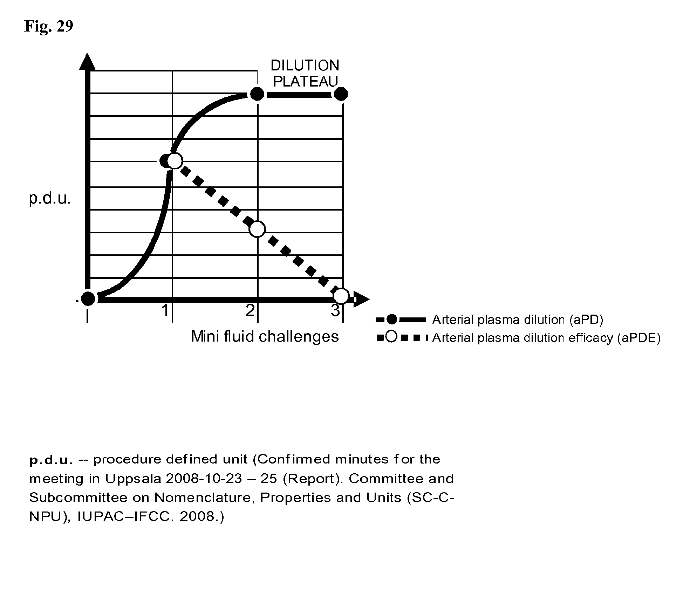

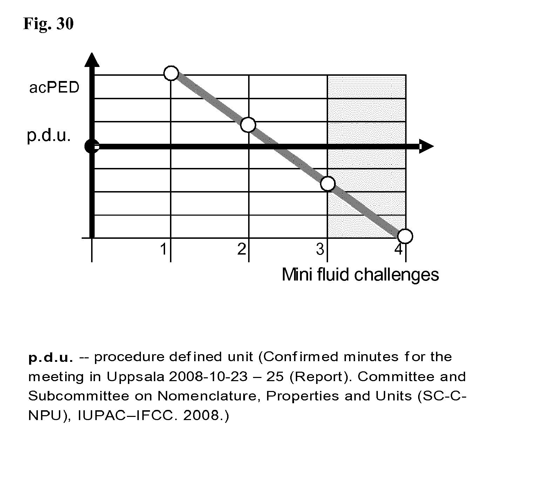

Addressing deficiencies within existing methods, the current invention **proposes new variables to the mVLT for the increased specificity and sensitivity in determining and optimizing the state of hydration.** Methodology is proposed for the evaluation of changes in tissue hydration and detection of imminent edema during mVLT and other fluid protocols or administration diuretics [FIGS.28]. Also, as previously proposed {26}, instead of monitoring PD trends and detecting *dilution plateau* during conventional mVLT algorithm, the monitoring of *plasma dilution efficacy* (PDE) of consecutive mini fluid challenges is proposed. The minimized arterial PDE [FIG. 29] would serve as a marker of optimized hydration status instead of hard to detect *dilution plateau.* The present invention also proposes assessment of mini fluid challenge induced changes in aPD and cPD, also aPDE and cPDE, and the difference of the latter - the arteriocapillary plasma dilution efficacy difference (acPED) by using the cHb values and the paHb or pvHb derived by the DGC method making the mVLT semi-invasive or noninvasive depending on the availability of invasive measures. The current invention **expands the set of diagnostic criteria for the optimisation of body hydration status and imminent edema** defining that state as a setting when at least one of the following criteria is met during a stepwise crystalloid infusion: (a) the aPDE or paPDE is negligible (close to zero), also the same for the venous variables when arterial are not available [FIG. 29], (b) the cPDE or predicted arterial PDE (paPDE) is similarly minimized (negligible decrease or increase or unchanged), (c) the acPED changes from positive to negative considering that paHb variable can be used instead of aHb for the calculation [FIG. 30], also venous valriables may be used instead, or (d) the decreasing ROF values during show the decreasing fluid movement from capillaries into tissues (decreasing FAR), and tissues are getting more and more expanded by fluids, and imminent edema may be present when ROF turns from positive to negative. The proposed transcapillary reflux model explains the clinical relevance of the variables. By means of the DGC method the mVLT becomes more specific and sensitive also more clinically applicable. The use of the DGC method expands the applicability of the mVLT method in the automated decision-support systems, also semi-closed or closed loop infusion systems.

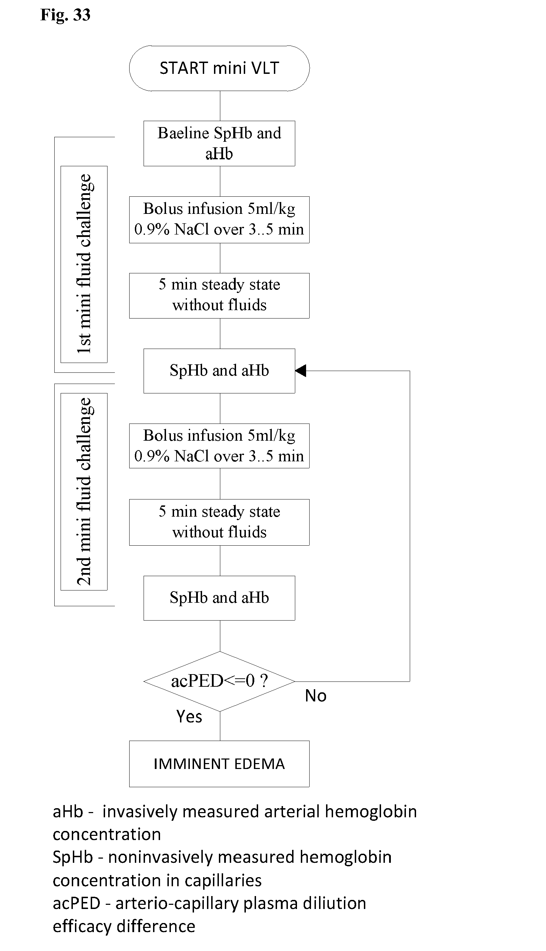

The use of the DGC method expands the applicability of the mVLT method in the **automated decision-support systems,** also **semi-closed or closed loop infusion systems.** The current invention proposes several algorithms for the possible use of the mVLT in the decision support systems and the semi-closed loop infusion system for the optimization of both hemodynamics and hydration status [FIG. 28, 31-34]. Any other target parameter can be used instead of arterial pressure which is used only for demo purposes.

A device or system is also provided comprising: a noninvasive blood hemoglobin concentration sensor attached to a computing apparatus so as to provide blood hemoglobin concentration input to the computing apparatus; an intravenous fluid pump controller which is attached to, and controlled by output from, the computing apparatus, wherein total hemoglobin measurements obtained by the noninvasive blood hemoglobin concentration sensor are adjusted by the computing apparatus according to the method of claim 1, and wherein the output is related to the input by the BIRD algorithm set forth in the specification and figures.

The DGC method applies to all devices which provide simultaneous measurements of cHb and PI via the same noninvasive sensor or via different sensors which measure cHb and PI. As used herein, SpHb and PI are referrences to cHb and PI which are measured at the same examination site and the synchronized - equal and simultaneous - averaging time is applied to these measures to calculate the variables that are displayed on the screen of the measuring device or can be obtained on-line. However, the currently available devices apply different averaging times for cHb and PI measures. That makes evaluation of relationship between simultaneous $\Delta$PI and $\Delta$cHb impossible. Presuming that this drawback is eliminated, the PI related adjustment of cHb could be implemented as an addition to the currently used in vivo adjustment. Modification of software in the noninvasive capillary Hb measuring devices is therefore required to provide the synchronized averaging of PI and cHb variables.

## BRIEF DESCRIPTION OF THE DRAWINGS

The invention is explained by way of example and with reference to the accompanying drawings:

**FIG. 1**: Most commonly used adhesive sensors for noninvasive continuous monitoring of perfusion index (PI), total hemoglobin (SpHb), Pleth Variability Index (PVI), $SpO_2$ and pulse rate by Pulse CO-Oximeter that operates on the basis of Rainbow SET Pulse CO-Oximetry technology, namely the monitor Radical-7 (Masimo Corporation, Irvine, CA).

**FIG. 2**: The 2012 Radical-7's upgradable rainbow SET platform features noninvasive and continuous monitoring of blood constituents that previously required invasive or complicated procedures, including total hemoglobin (SpHb), perfusion index (PI), oxygen content (SpOC™) and pleth variability index (PVI). It also features Masimo SET Measure-through Motion and Low Perfusion pulse oximetry for oxygen saturation ($SpO_2$) pulse and respiration rate through either acoustic sensor (RRa™) or through plethysmograph waveform (RRp™).

**FIG. 3:** The upgradable rainbow technology is used in a versatile handheld easy-to-use bedside monitors (A) Rad-57, (B) Pronto and (C) Pronto-7 with rainbow 4D™ technology for noninvasive and quick spot checking of total hemoglobin (SpHb®), $SpO_2$ pulse rate and perfusion index.

**FIG. 4:** The Rad-87 enables noninvasive continuous measuring of total hemoglobin (SpHb) and perfusion index (PI). It features a built-in 802.11 radio, allowing for bidirectional wireless communication with the remote monitoring and clinician notification system.

**FIG. 5:** Masimo Patient SafetyNet is the remote monitoring and clinician notification system that helps keeping patients safe on general care floors.

**FIG. 6:** The changes of blood hematocrit (Hct) during blood passage *via* micro-vessels (capillaries) with different radius in a capillary bed. The Hct in macro-capillaries (arteriole, metarteriole and venule) is affected solely by Fahraeus effect, while it is additionally affected by the transcapillary filtration-absorption ratio in micro-capillaries (true capillaries). The ratio between Hct in larger and smaller radius vessels is defined as *f*-ratio. The inner radius is above 500 in large vessels, and below 5 micrometers in micro-capillaries (true capillaries). Only the micro-capillaries are involved in transcapillary exchange of fluids. Their radius is too small to allow traceable changes in hematocrit in response to changing hemodilution in larger vessels because their radius allows entrance of one red cell at a time. Nevertheless, changes in transcapillary filtration-absorption ratio can change hematocrit in true capillaries by changing the distance between red cells in true capillaries. Thus, theoretically, measuring changes of hematocrit and hemoglobin concentration in capillaries would enable monitoring changes in transcapillary fluid movement.

**FIG. 7:** Our pilot clinical observations of the simultaneously measured hemoglobin values by using three different techniques during major elective orthopedic surgery. The venous Hb (vHb) was measured bedside from a venous blood draw (HemoCue, Sweden), while SpHb was measured noninvasively by using the two sensor versions - version *k* (1-SpHb; most recent edition, 2013) and g (2-SpHb; earlier edition, 2012) also appropriate software versions installed in two Radical-7 devices. All variables were recorded simultaneously at random checkpoints during surgery, infusion therapy and sometimes before and after intraoperative blood transfusions.

**FIG. 8:** A fluid challenge is defined as a fluid bolus followed by its corresponding time without fluid. Thick lines are showing plasma dilution during bolus infusions and thin lines are showing plasma dilution during periods without fluid infusion. Solid lines reflect dehydrated subjects while broken lines reflect normohydrated subjects. The VLT test will show similar plasma dilution peaks at the end of infusion in both hydrated states but show a difference twenty minutes after infusion. The mVLT test, however, will distinguish between these two states earlier, after two challenges in the dehydrated state and after three fluid challenges in the normohydrated state. The dilution plateau is a state where plasma dilution is equal after two consecutive mini fluid challenges.

**FIG. 9:** The arbitrary stratification of capillaries into macro- and micro-capillaries.

(a) Transcapillary fluid exchange exists only in micro-capillaries so changes in the transcapillary fluid filtration absorption ratio (FAR) can, therefore, lead to changes in micro-capillary hemoglobin concentration (sHb), while macro-capillary hemoglobin concentration (mHb) changes only in response to changes in large vessel Hb due to Fahraeus effect, (b) the sketch of arbitrary mean radius difference between the large artery and the two types of capillaries.

**FIG. 10:** The relationship between inner radius and Hb differences between a large artery and capillaries

**FIG. 11:** The mean radius difference between a large artery and a mixture of two types of capillaries.

**FIG. 12:** The origin of a positive GAP between aHb and cHb (cGAP).

**FIG. 13:** Relationship between the tissue perfusion index (PI) and the light absorption in micro- and macro-capillaries.

**FIG. 14:** A decrease in the gap between cHb and aHb (cGAP) due to an increase in perfusion index (PI) and decrease of mean radius difference between large artery and macro-capillaries (mGAP). Solid lines are for trends before the shift, while dashed lines are before the shift.

**FIG. 15:** A decrease in the gap between cHb and aHb (cGAP) due to an increase in the transcapillary fluid filtration-absorption ratio (FAR). Solid lines are for trends before the shift, while dashed lines are before the shift.

**FIG. 16:** A decrease in the gap between cHb and aHb (cGAP) due to a decrease in arterial hemoglobin concentration (aHb) in large arteries. Solid lines are for trends before the shift, while dotted and dashed lines are before the last and its previous shifts.

**FIG. 17:** A decrease in the gap between cHb and aHb (cGAP) due to a significant decrease in aHb. The Solid lines are for trends before the shift, while dotted-dashed and dashed lines are before the last and its previous shift.

**FIG. 18:** A decrease in the gap between cHb and aHb (cGAP) due to an increase in the perfusion index (PI).

**FIG. 19:** The relationship between the positioning (markers shown as CS1-4) of the cHb sensor above a capillary bed and the transcapillary fluid filtration-absorption ratio patterns affecting the cHb readings.

**FIG. 20:** The difference between between arterial (aHb) and capillary (cHb) concentration - the GAP - can be constant during changes in arterial hemoglobin (e.g. during dilution), but it may be (a) positive and (b) negative.

**FIG. 21:** Arbitrary data as an example of fluid infusion induced shifts where the GAP can be both (a) positive and (b) negative.

**FIG. 22:** The ratio between the transcapillary fluid filtration absorption ratio (FAR) related GAP (fGAP) and the tissue perfusion index dependent GAP (tGAP) may be lower in the GAP before the fluid infusion ($GAP_0$) than after the 5 minutes after its end ($GAP_1$).

**FIG. 23:** The relationship between the deviations of PI dependent GAP ($\Delta$tGAP) and the FAR dependent GAP ($\Delta$fGAP) as fractions of the GAP deviation ($\Delta$GAP) during the shift of aHb (or vHb), cHb and PI, e.g. during the *calibration protocol.*

**FIG. 24:** If the GAP is positive and PI decreases, the cHb value decreases and the GAP increases. However, if the GAP is negative, when PI decreases the cHb value decreases and the absolute value of the GAP decreases, although GAP remains negative. The deviation in FAR is synergistic - it affects the cHb and GAP in the same way as PI. The Fahraeus effect related GAP increases with increasing radius difference between the large vessels and capillaries.

**FIG. 25:** Net arterio-venous and arterio-capillary dilution differences and net interstitial fluid expansion during crystalloid infusion and the initial post-infusion period. The arrows in the figures represent equal volumes of fluid. The net whole-body interstitial fluid volume remains constant when the net lymphatic flux into the central veins is equal to the net volume difference between transcapillary fluid filtration and absorption. The transcapillary fluid filtration absorption ratio (FAR) is arbitrarily 2:1 and results in positive net arterio-venous (avDD) and arterio-capillary (acDD) dilution differences. Thirst-guided fluid intake is equal to the basal renal elimination.

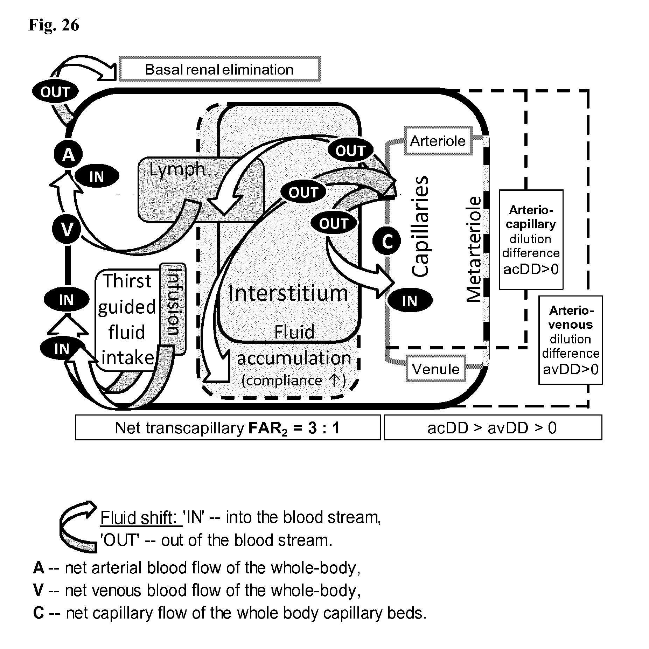

**FIG. 26:** Net arterio-venous and arterio-capillary dilution differences and net interstitial fluid expansion during crystalloid infusion and the initial post-infusion period. The arrows in the figures represent equal volumes of fluid. Intravenous fluid infusion causes interstitial fluid accumulation and an increase in both avDD and acDD. This occurs because an increase in net transcapillary pressure leads to an increase in FAR from 2:1 to 3:1, an increase in FAR leads to an increase in interstitial fluid volume and compliance, and an increase in compliance maintains both stable interstitial hydrostatic pressure and a stable lymphatic flux. Because the lymphatic flux remains unchanged, it becomes lower than the net volume difference between transcapillary fluid filtration and absorption. When compliance reaches its physiological maximum, all of the infused fluid can be trapped in the interstitium and renal elimination may remain at basal levels. This point might represent hydration non-responsiveness during the mVLT.

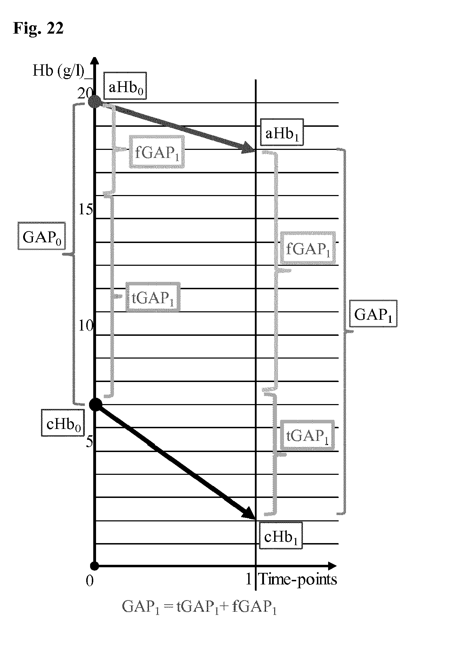

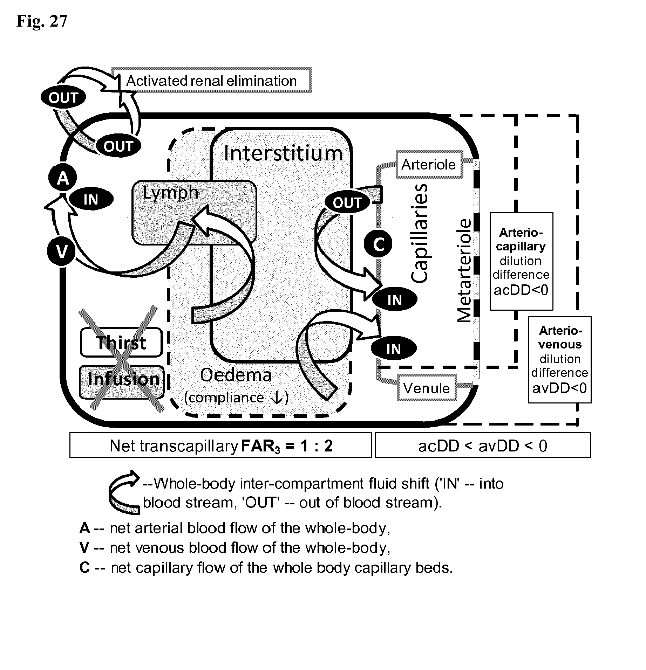

**FIG. 27:** Net arterio-venous and arterio-capillary dilution differences and net interstitial fluid expansion during crystalloid infusion and the initial post-infusion period. The arrows in the figures represent equal volumes of fluid. Following the end of an infusion that causes interstitial edema, the release of excessive fluid into the capillaries and a change in avDD and acDD from positive to negative is a result of a decrease in net transcapillary pressure leading to an arbitrary decrease in FAR from 3:1 to 1:2, a decrease in both interstitial fluid volume and compliance, and activated renal elimination that matches the excess interstitial fluid being released into the circulation. Consequently, arterial blood that enters the capillaries is less dilute than capillary and venous blood. The lymphatic flux facilitates fluid release from the edema because lymphatic flux is maintained by the accumulated excessive interstitial fluid, not by the transcapillary fluid influx shown in Fig. 22.

**FIG. 28:** The flowchart describing the basic algorithm for the optimisation of fluid status by using the mini Volume Loading Test (mVLT) fluid protocol.

**FIG. 29:** A theoretical sketch of the aPD and aPDE of three mini fluid challenges. The aPDE after the first mini fluid challenge decreases and becomes negligible after the third fluid challenge. This is an indication that net fluid extravasation is equal to net volume of infused fluid entering the circulation. The fluid infusion is no longer efficaciously diluting the blood at this point.

**FIG. 30**: The arterio-capillary plasma dilution efficacy difference (acPED) changes from positive to negative. This is a confirmation of the transcapillary fluid reflux.

**FIG. 31:** The flowchart describing the use of the mini Volume Loading Test (mVLT) in the clinical algorithm for the combined optimization of hemodynamic and hydration status.

**FIG. 32:** The flowchart describing the use of the mini Volume Loading Test (mVLT) in the decision support system or semi-closed loop infusion system for the combined optimization of circulation and perfusion status (flow-related parameters), hydration status (hemodilution) and transfusion decision-making (increasing the anemia tolerance).

**FIG. 33:** The flowchart describing the use of the mini Volume Loading Test (mVLT) in the software for a decision support system for the simplistic combined optimization of hemodynamic and hydration status and optimization of blood transfusions.

**FIG. 34**: The flowchart describing the use of the mini Volume Loading Test (mVLT) in the software for a decision support system or semi-closed loop infusion system for the combined optimization of circulation and perfusion status (flow-related parameters), hydration status (hemodilution) and transfusion decision-making (increasing the anemia tolerance).

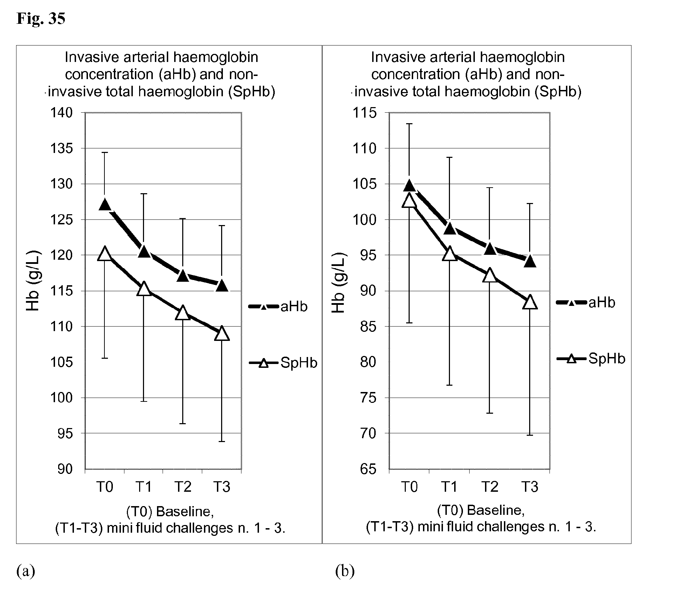

**FIG. 35:** Change in arterial Hb and SpHb during the two fluid protocols. (a) Preoperative, and (b) postoperative fluid protocols. Data point T0 was at baseline before the first bolus, and data points T1-T3 were after the 5 minute periods without fluids that followed each of three 5 ml kg$^{-1}$ boluses of acetated Ringer's solution in 3 mini fluid challenges. Data presented as mean $\pm$ SD.

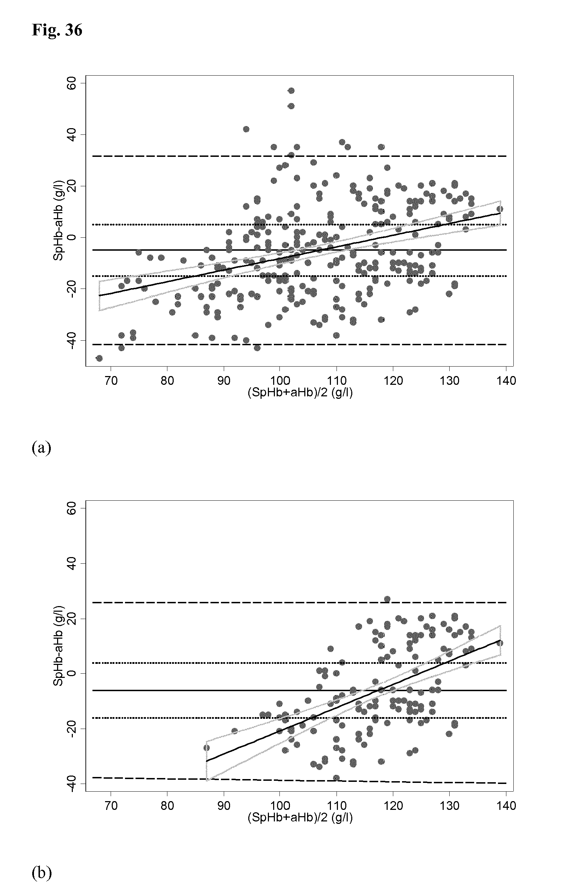

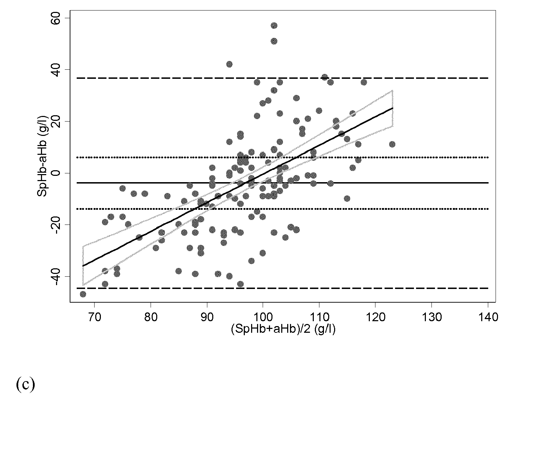

**FIG. 36**: Bland-Altman plots showing the agreement between SpHb and arterial Hb for all data sampling points in two fluid experiments. Vertical axis is the difference between the measurements. Horizontal axis is the mean of arterial Hb and SpHb. Bias is a solid line, limits of agreement (bias $\pm$ 2*SD) - dashed lines, outlier limits (bias $\pm$ 10 g l-1) - dotted lines, and linear correlation projection - light lines. (a) Overall 288 peri-operative paired values (144 preoperative + 144 postoperative). (b) Preoperative 144 paired values. (c) Postoperative 144 paired values.

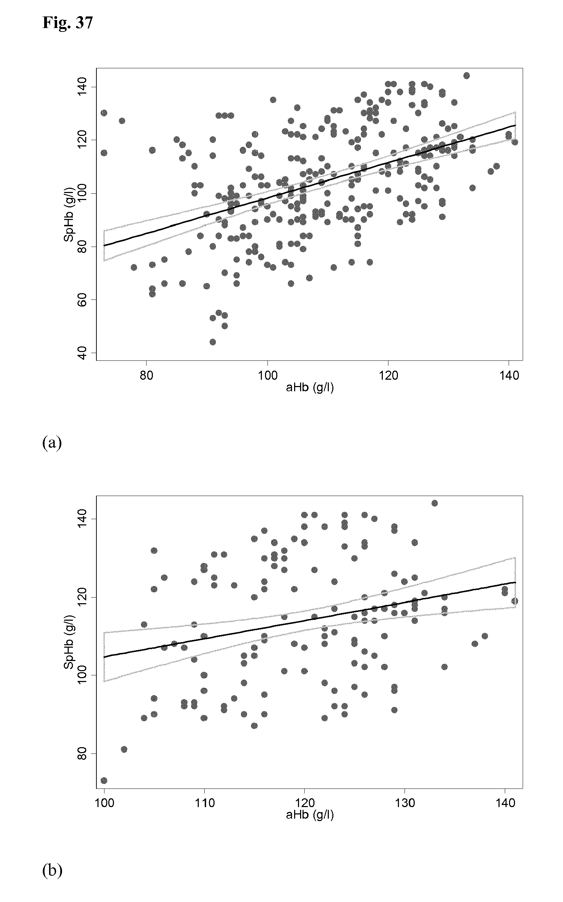

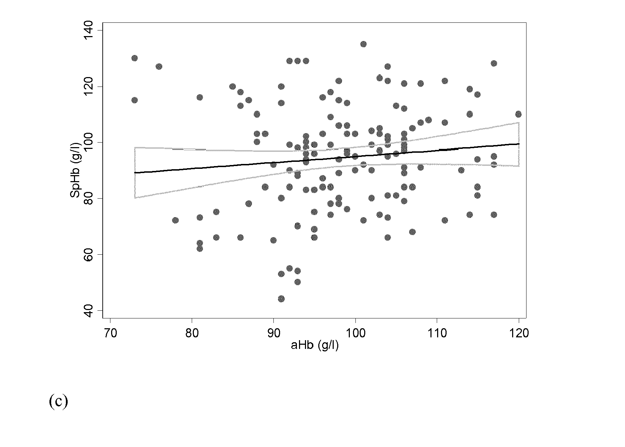

**FIG. 37:** The linear regression between noninvasive (SpHb) and invasive arterial (aHb) haemoglobins. Regression line (heavy line) and 95% CI lines (light lines). (a) Overall peri-operative correlation between 288 SpHb and 288 aHb measures. (b) Pre-operative correlation between 144 SpHb and 144 aHb measures. (c) Post-operative correlation between 144 SpHb and 144 aHb measures.

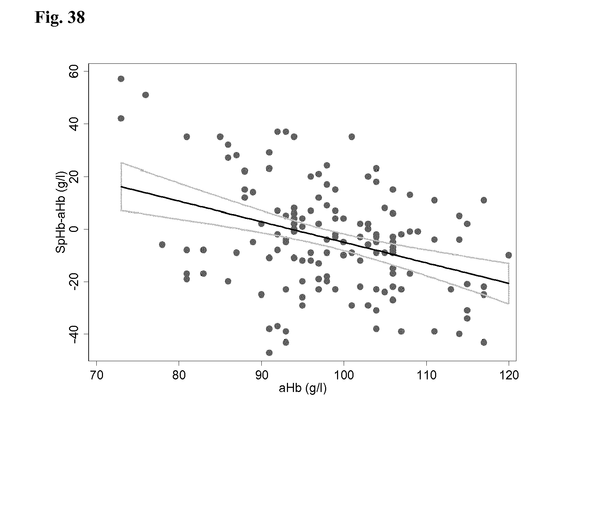

**FIG. 38**: The bias of the SpHb measurement versus the invasive arterial Hb for all data sampling points in the post-operative fluid experiment. The risk of misjudging postoperative aHb by relying on SpHb is when aHb is below 100 g l$^{-1}$.

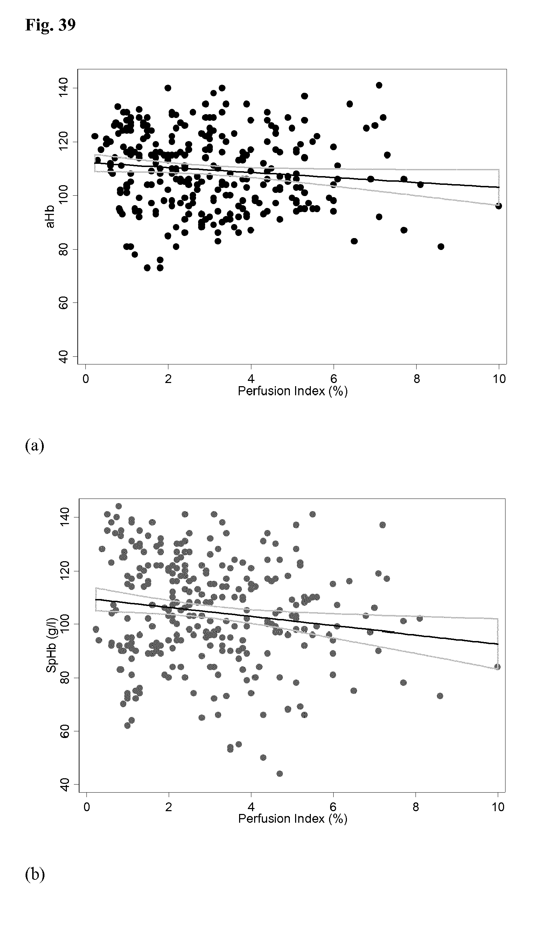

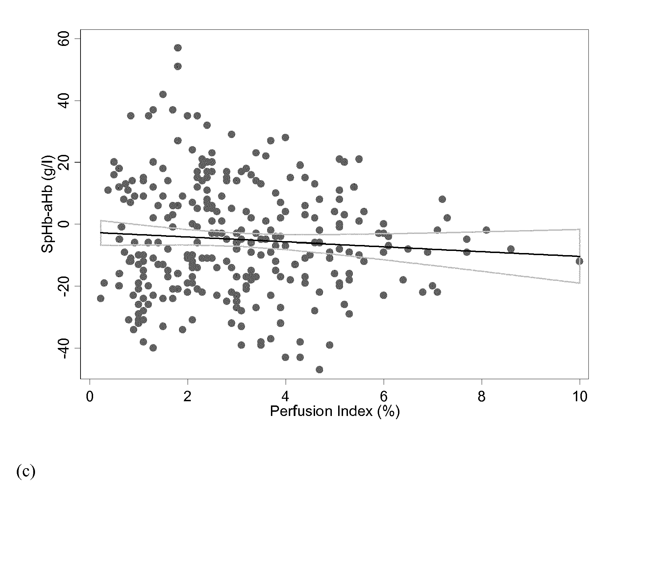

**FIG. 39:** Correlation between the perfusion index and haemoglobin concentration in 288 perioperative data measuring points (144 preoperative + 144 postoperative). (a) Correlation between the perfusion index and aHb. (b) Correlation between the perfusion index and SpHb. (c) Correlation between the perfusion index and the difference between SpHb and aHb.

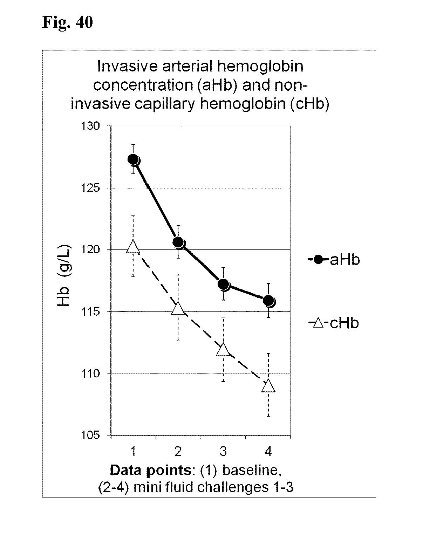

**FIG. 40:** The invasive aHb (arterial) and non-invasive cHb (capillary; SpHb™) variables determined at four data points. Data point 1 was at the baseline before the first bolus, and data points 2-4 were after the 5 minute periods without fluids that followed each of the three 5 ml·kg$^{-1}$ crystalloid boluses in the three mini fluid challenges. The data is presented as the mean ± SEM.

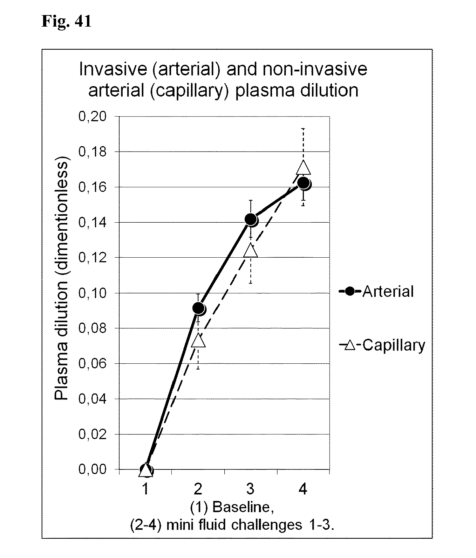

**FIG. 41:** The plasma dilution: invasive aPD (arterial) and non-invasive cPD (capillary) variables determined at four data points. Data point 1 was at the baseline before the first bolus, and data points 2-4 were after the 5 minute periods without fluids that followed each of the three 5 ml·kg$^{-1}$ crystalloid boluses in the three mini fluid challenges.

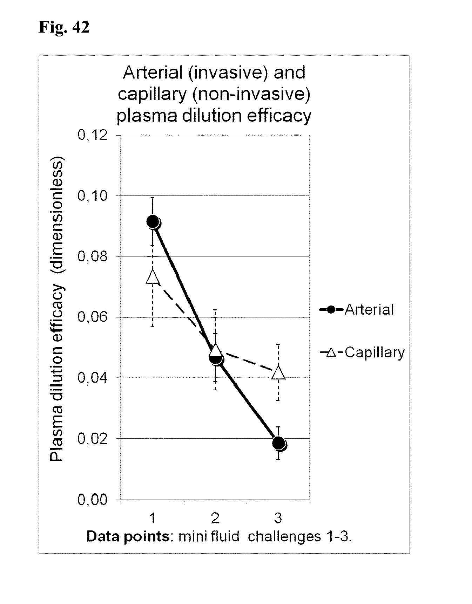

**FIG. 42:** The plasma dilution efficacy: invasive aPDE (arterial) and non-invasive cPDE (capillary) variables determined at four data points. The data points 1-3 were after the 5 minute periods without fluids that followed each of the three 5 ml·kg$^{-1}$ crystalloid boluses in the three mini fluid challenges. The data is presented as the mean ± SEM.

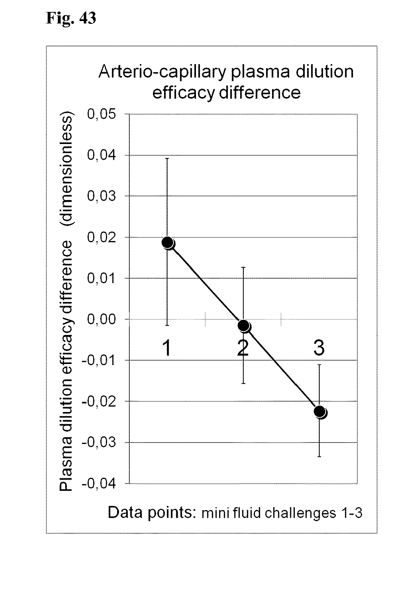

**FIG. 43:** The arterio-capillary plasma dilution efficacy difference (acPED) changes from positive to negative serving as confirmation of transcapillary fluid reflux. The data points 1-3 were after the 5 minute periods without fluids that followed each of the three 5 ml·kg$^{-1}$ crystalloid boluses in the three mini fluid challenges. **(a)** Invasive aHb (arterial) and non-invasive cHb (capillary). **(b)** Plasma dilution: invasive aPD (arterial) and non-invasive cPD (capillary). **(c)** The plasma dilution efficacy: invasive aPDE (arterial) and non-invasive cPDE (capillary). **(d)** The arterio-capillary plasma dilution efficacy difference (acPED) changes from positive to negative serving as confirmation of transcapillary fluid reflux. The data is presented as the mean ± SEM.

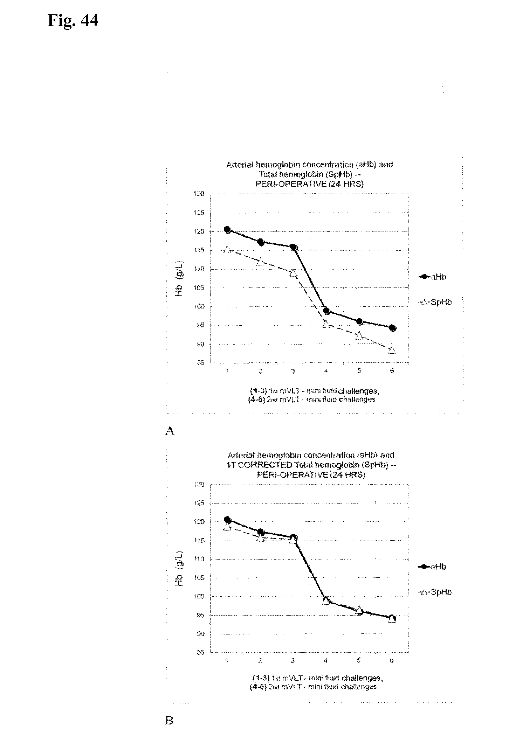

**FIG. 44:** The decreasing ratio of FAR in consecutive mini fluid challenges (ROF) during mVLT shows that tissues are getting more and more expanded by fluids, and imminent edema may be present when ROF becomes negative.

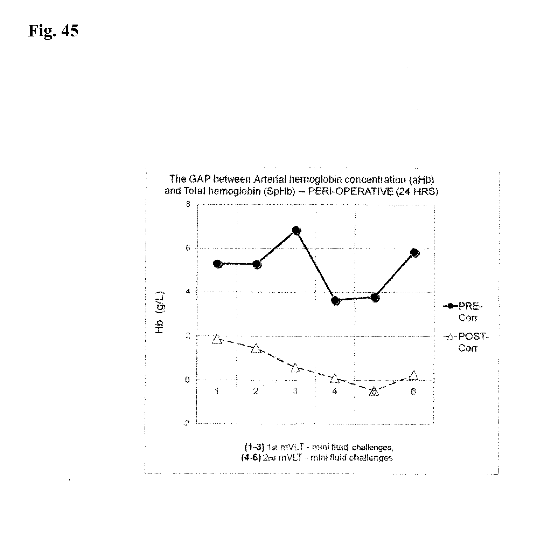

**FIG. 45:** The actually measures arterial Hb and noninvasively measured SpHb (Radical 7 from Masimo Corp.) also the PI-adjusted noninvasive capillary hemoglobin (paHb) in mVLT after the semi-invasive calibration protocol (data from calibration is not included in the chart).

**FIG. 46**: A block diagram that illustrates a computer system upon which an ambodiment of the invention may be implemented.

**FIG. 47**: A diagram illustrating a semi-closed loop system infusion system consisting of ABP and Hb measuring devices, algorithmic data analysis and generating the commands for the pumps to perform the vasopressor injection and infuse fluids and blood, according to an embodiment.

**FIG. 48:** A flow chart demonstrating a system that implements a calibration protocol according to the statistically biased calibration method, according to an embodiment.

## DETAILED DESCRIPTION

The present invention is focused on the **mathematical adjustment** of the noninvasive capillary hemoglobin (cHb) measures by multi-wavelength spectrophotometric technique in soft tissues under the sensor for the improvement of its accuracy and precision for the prediction of hemoglobin concentration (Hb) in large vessels, and its use for the monitoring of changes in transcapillary movement of fluids under the sensor during the mini volume loading test (mVLT), also defining the applicability of mVLT in automated and semi-automated infusion systems by using the same.

The present invention provides a Dilution Guided Correction method (DGC method) to minimize a discrepancy between invasively measured arterial hemoglobin concentration (aHb) and noninvasively measured total hemoglobin, e.g. SpHb obtained by a Pulse CO-Oximetry module of the Rainbow SET® platform (Masimo Corporation, Irvine, CA, USA). The method applies to all devices which provide simultaneous measurements of capillary hemoglobin concentration (cHb) and perfusion index (PI) via the same noninvasive sensor or via different sensors which measure total hemoglobin and prefusion index at the same examination site. As used herein, SpHb and PI are referrences to total hemoglobin and perfusion index which are simultaneously (or near simultaneously) measured at the same examination site.

The Dilution Guided Correction method (DGC method) allows for the minimization of a difference (referred to herein as GAP, which may be positive or negative), in real time in some embodiments, between hemoglobin concentration (Hb) measurements in larger and lower radius vessels (supplied by the former).

The present invention may be relevant to testing and patient optimisation in, for example, patients undergoing major elective or emergency surgery and obstetrical procedures, major surgery or organ transplantation, patients with preexisting disorders of fluid turnover or acquired fluid deficiency states secondary to burns, bleeding or ileus and critically-ill patients.

The present invention applies to both inpatient and outpatient surgical or hematology settings, and to procedures performed in operating rooms, intensive care units and other locations (e.g., interventional radiology or surgery wards) where testing blood hemoglobin content and administration of fluid replacement are indicated. This new invention is directly applicable to the care administered by family doctors, hematologists, anesthesiologists, intensive care doctors, surgeons and individuals who deliver care under their medical direction or supervision.

Despite continuous improvement of noninvasive techniques, the unpredictable discrepancy between the invasive and noninvasive Hb measures remains a serious limitation in the use of noninvasive measures for the prediction of large vessel Hb values. That is because noninvasive devices are measuring the Hb in blood pulsing through a capillary bed, usually under a finger nail. Vascular physiology affects the capillary Hb in a way that is not present in larger vessels. Thus, noninvasively measured Hb values do not exactly match the Hb measurement made using a blood sample from a larger vein or artery. The discrepancy between invasive and noninvasive Hb measures exists due to Fahraeus effect, also because hemodilution in capillary beds under the noninvasive sensor is unstable due to constantly changing FAR and fluctuation of capillary radiuses (reflected by PI). Although mathematical optimisation which is a common technique used for the improvement of the accuracy and precision in various measuring devices, reduces the individually occurring Hb gap between the invasive and noninvasive measures, there is an individual incompliance with the established average statistical behaviour because of the unpredictable and sometimes severe physiological perturbations that frequently occur in microcirculation under the noninvasive sensor. All currently commercially available devices for noninvasive hemoglobin measures are reported to be similar in more or less clinically acceptable accuracy and unacceptable precision when the invasively arterial and venous Hb measures are used as references.

Assessment of the body fluid status and the mVLT method has several drawbacks. The PD trends *per se* are not reliable for the detection of optimization during mVLT fluid protocol. That is because comparison of PD after repetitive fluid boluses separated by only a few minutes cannot fully reflect differences in intravascular fluid retention because dilutions overlap. The *dilution plateau* as marker of optimisation can be missed because its detection requires equal Hb and thus PD values before and after a mini fluid challenge and this requires an Hb measuring device with both high accuracy and precision. Also the dilution plateau can be missed even because it is an unstable transitory state. The reliable mVLT application requires invasive Hb analysis because the noninvasive Hb cannot reliably predict the large vessel Hb. Noninvasive Hb measures are affected by both - the transcapillary fluid movement and the perfusion index. Thus, although referring to noninvasively measured Hb as capillary opens a theoretical possibility for the monitoring the transcapillary fluid movement and detection of imminent edema, an impact of PI on capillary Hb readings has to be eliminated so that FAR would be the only unstable factor in the discrepancy between Hb in large vessels and capillaries. This is because the Fahraeus effect related gap is constant.

Clinical decision support devices and automated infusion systems have limited clinical applicability and, even using them, the patient optimization targets are frequently missed. That is because automated clinical decision support systems are focused on hemodynamic optimization by IV fluid loading, usually implementing goal directed therapy algorithms, while the related changes of body hydration status are not monitored or reliably evaluated. The most specific target parameters used in goal directed fluid management have limited availability and applicability in the everyday clinical practice. Fluid administration guided by hemodynamic response can lead to the deleterious swelling of tissues (edema) that negatively affect the overall outcomes of treatment.

The present invention relates to a mathematical model for individual discrimination between the components of discrepancy between arterial hemoglobin concentration (aHb) and cHb where transcapillary fluid filtration-absorption ratio (FAR) component is on one side and tissue perfusion and Fahraeus effect related interferring components are on the other side, also assessment of changes in transcapillary fluid movement in response to administration of fluid or diuretic therapy, also providing guidelines for their individualized administration.

The present invention includes a method and apparatus for determining the predicted arterial hemoglobin concentration (paHb) value from noninvasive mesures of cHb, also determining the changes in transcapillary fluid movement from the dynamics of cHb and paHb after an intake or IV administration of fluid or a diuretic, or vasoactive medication such as norepinephrine, or during series of test volume loads of crystalloid solutions in mVLT. The apparatus can also automatically (closed loop system) or semi-automatically (semi-closed loop system) guide administration of fluids and diuretic medication targeted to achieve or maintain a specific pattern of transcapillary fluid movement.

Addressing deficiencies within existing methods, the current invention proposes the **Dilution Guided Correction method (DGC method).** It consists of three models and the **calibration protocol**. A physiology based **dynamic gap model** is used as background for the mathematical modelling of changes in GAP defined by equations the **DGC-math model.**

**The dynamic gap model**

Addressing deficiencies within existing methods, the current invention proposes an applied physiology based model of the dynamic gap - the **dynamic gap model** - for the mathematical modelling of changes in GAP. The dynamic gap model explains the anatomical and physiological origin of the GAP. The multi-wavelength spectrophotometry technique measures Hb in a mixture of macro-capillaries (arterioles, metarterioles, and venules) and micro-capillaries (also known as true capillaries) in the derma [FIG.6] [FIG.9a]. The Fahraeus effect means that the blood viscosity is reduced as the inner radius of a vessel decreases. Thus, there is an Hb difference (a gap) between a larger and a smaller blood vessel. The hematocrit (Hct) in macro-capillary is determined by the Hct in large artery and the Fahraeus effect, while the Hct in micro-capillary is determined by the inner radius of a vessel and the transcapillary filtration-absorption ratio. The Fahraeus effect does not apply to micro-capillaries, because their radius is too small to allow the passage of more than one erythrocyte at a time. Even more, red cell has to accommodate its shape and become more oval in order to pass these vessels. Obviously, the Hct in a micro-capillary does not react to Hct changes in a large artery. There is a linear relationship between the Hct and Hb when the mean cell haemoglobin and mean cell volume are stable. Thus, under such conditions, the Hb changes in parallel to Hct.

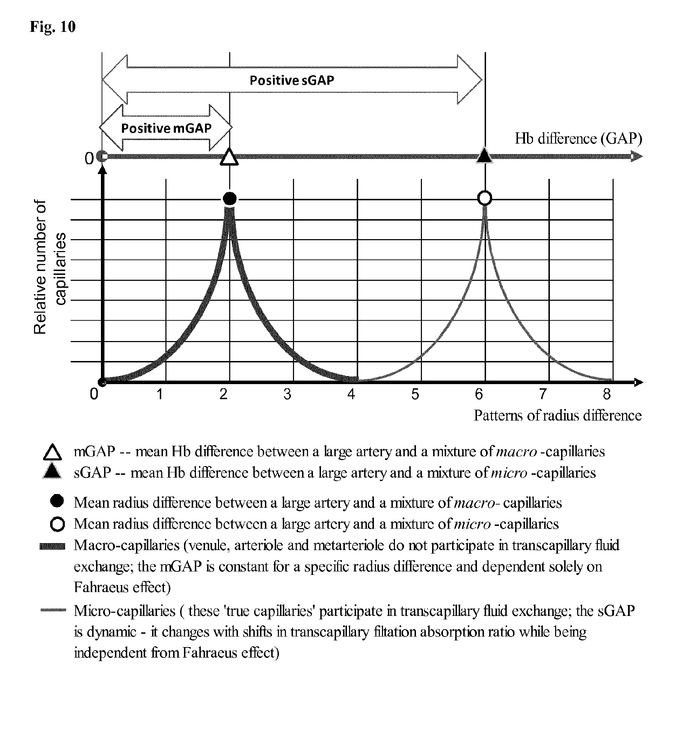

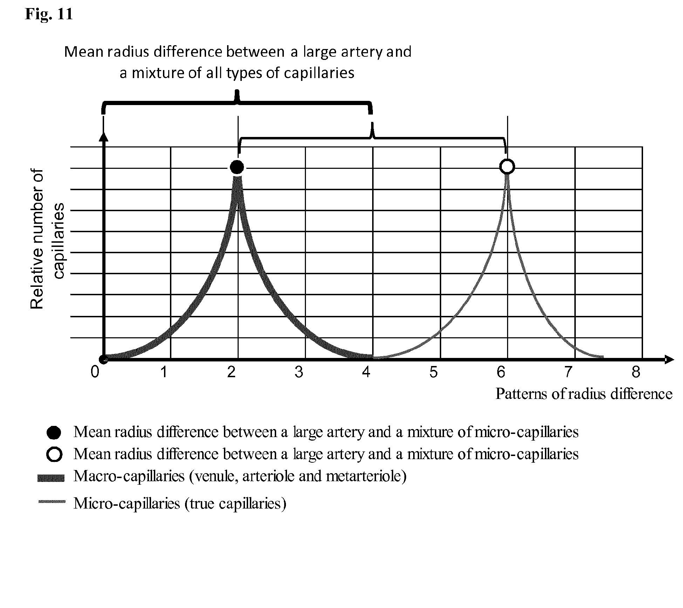

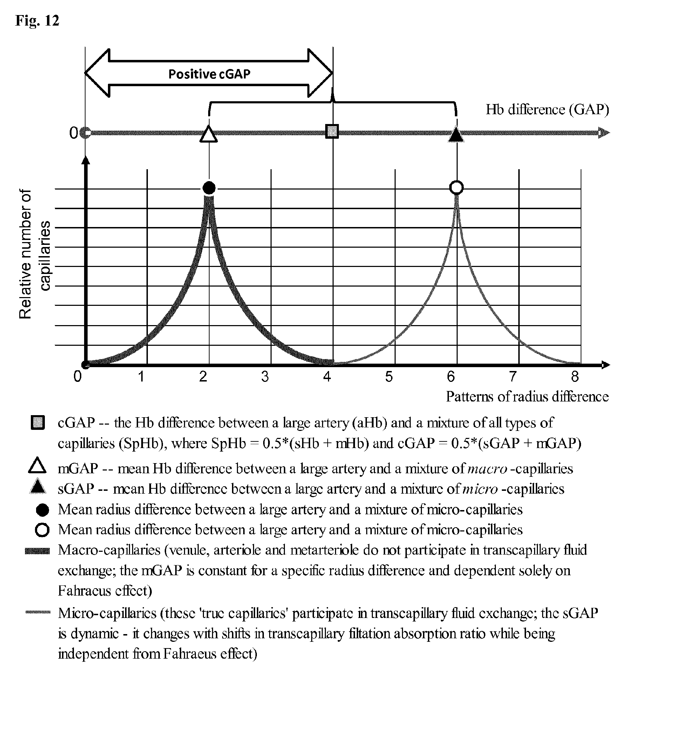

According to the model, under the cHb sensor, e.g. under the SpHb® measuring sensor from Masimo Corporation, Irvine, CA, there is an unpredictable mixture of capillaries with different radii [FIG.9a], and the relative number of capillaries with equal radii determines the mean radius difference between a large artery and a mixture of micro- or macro-capillaries [FIG.9b]. Consequently, the mean radius differences determine the Hb gap between a large artery and the two types of capillaries under the cHb sensor [FIG.10]. We refer to large artery as an artery where arterial blood samples for laboratory analysis of aHb are obtained. The mean value of inner radius differences defines the difference between a large artery and a mixture of all types of capillaries [FIG. 11]. According to the model, the cHb is the mean value of mean Hb values in the macro- (mHb) and micro- (sHb) capillaries. The difference between the aHb and cHb (cGAP) is the means of difference between the aHb and mHb (mGAP) and difference between the aHb and sHb (sGAP) [FIG.12].

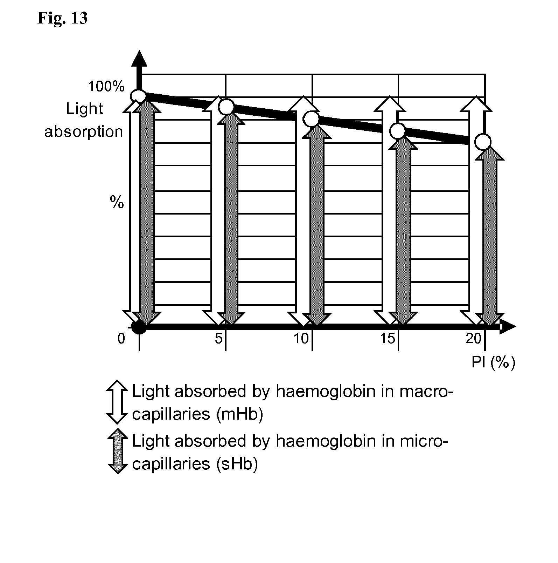

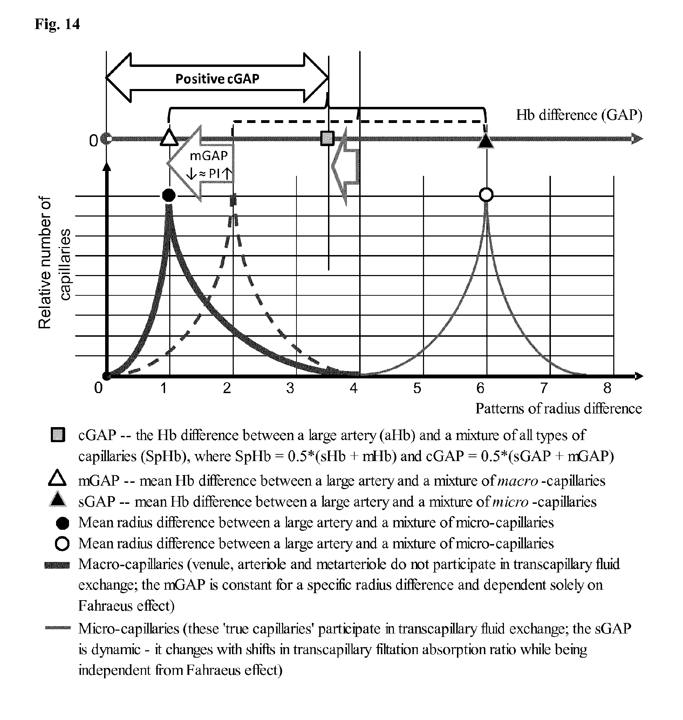

Perfusion index is calculated by expressing the pulsatile signal as a percentage of the non-pulsatile signal. According to the present invention the relatively frequent pleth oscillations (vasomotion) in micro-capillaries are overlapping with the less frequent heart rate dependent oscillations in macro-capillaries. Consequently, the infrared light absorption by hemoglobin in the overlapping zone determines the non-pulsatile part of extracted signal [FIG.13]. Thus, the PI increases when the blood flow in micro-capillaries decreases in respect to macro-capillaries and leads to the lower infrared light absorption therein [FIG.14]. The differences in anatomy and physiology determine the possibility of different Hb response in micro- and macro- capillaries to changes in PI. When the increase in PI reflects dilation of both types of capillaries, only macro-capillaries can respond by increasing the Hb here within and lower the cGAP. Even when dilating, micro-capillaries are too small to allow the passage of more than one red cell at a time, and thus, sHb remain stable. Obviously, the bias of cHb measurement decreases with increasing PI and vice versa. A decrease in PI would lead to an increasing cGAP because cHb decreases more than aHb. This can explain why a previously reported decrease in PI was associated with an increasing GAP during lactated Ringer's infusion in healthy volunteers {18} {47}. Moreover, the previously observed decrease in PI itself could not be reliably explained by vasoconstriction induced by the temperature of the infusion fluid because the fluid had been warmed before infusion {47}.

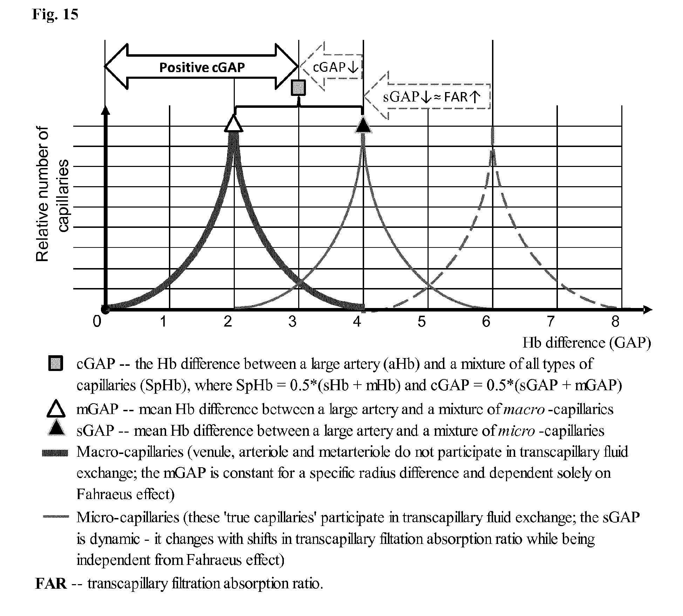

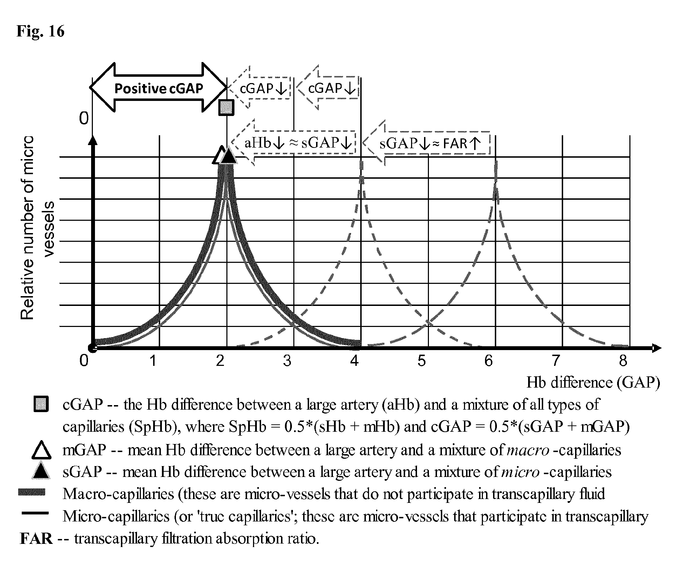

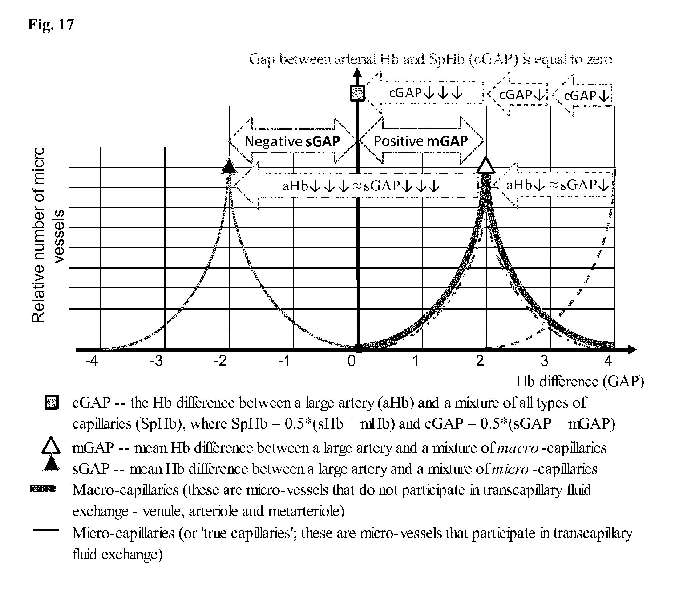

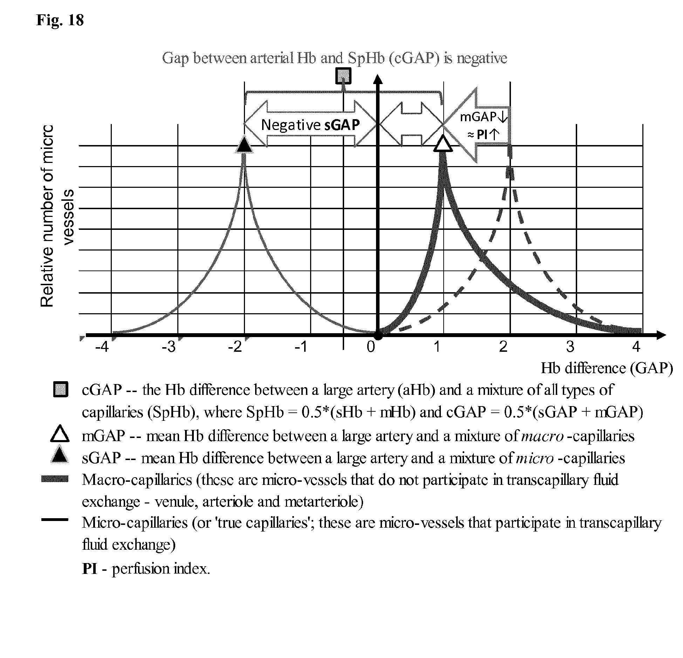

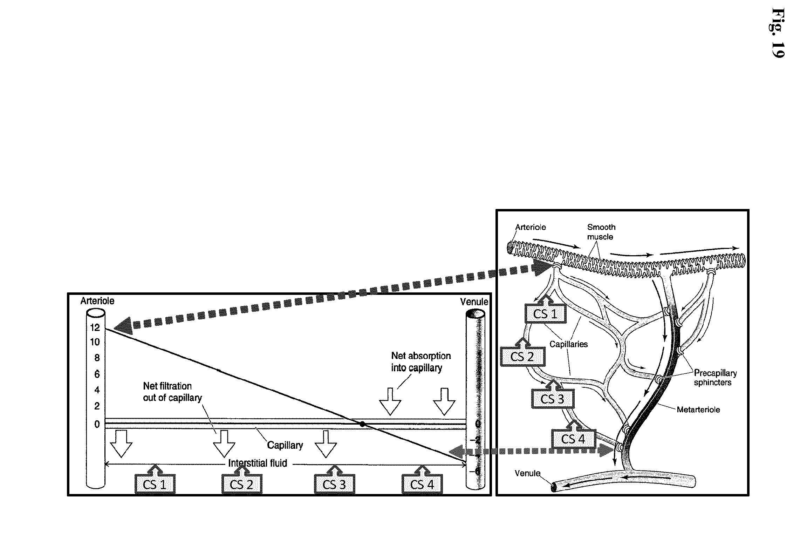

Transcapillary fluid exchange exists only in micro-capillaries so changes in the FAR can, therefore, alter the distance between adjacent red cells and lead to changes in sHb. Thus, an increase in the FAR decreases the cGAP [FIG.15]. If there is a loss of red cells, the situation changes. In macro-capillaries, mHb changes in a similar manner to aHb because the Fahraeus effect-dependent Hb gap between these vessels is constant. In contrast, as described earlier in the text, the sHb in micro-capillaries does not change when aHb changes. Consequently, the cGAP decreases during haemorrhage [FIG.16]. In case of significant loss of red cells, the previously positive cGAP becomes zero [FIG.17], and further loss of red cells or even an increase in PI can lead to a negative cGAP [FIG.18]. In a capillary bed, the transcapillary filtration is greatest on the arteriolar side and absorption is greatest on the venular side. Obviously, the cHb readings will depend on the prevailing section of a capillary bed being scanned - the cHb value will be higher if the arteriolar side of capillary tree is prevailing under the sensor [FIG.19].

**The DGC-math**

Dilution Guided Correction mathematical method (DGC-math) is proposed for the processing of invasively and noninvasively measured variables - Hb and PI - for the individual adjustment of cHb values aiming to predict the aHb or vHb values when they are not available, also for calculating the derivative variables for the purposes of monitoring the hydration status and transcapillary fluid movement.

**The mathematical postulations and equations are as follow:**

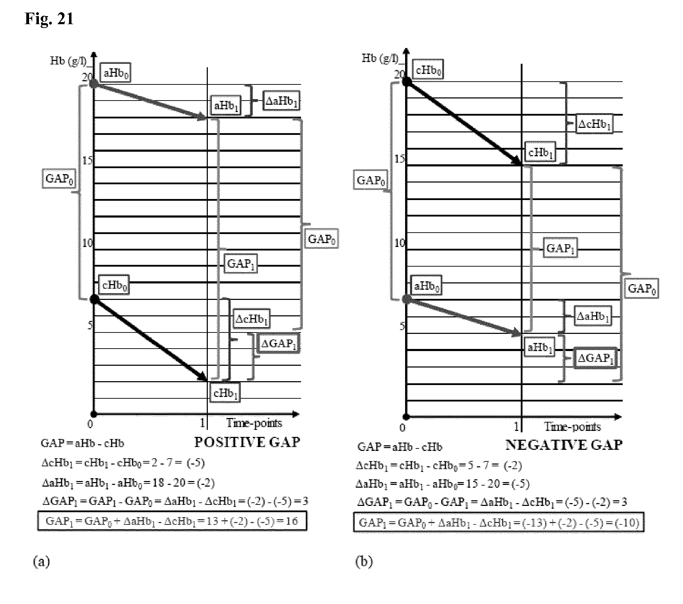

1. The GAP, which is a difference between aHb (or vHb) and cHb, can be positive or negative depending on the mixture of capillaries under the cHb measuring sensor. The GAP is positive when the prevailing capillaries are closer to the venular side of the capillary bed, but negative when they are closer to the arteriolar side [FIG.19]. The positive GAP may remain constant during changes in aHb [FIG.20a] or it may change [FIG.21a]. Similarly, the negative GAP may remain constant during changes in aHb [FIG.20b] or it may change [FIG.21b].

$$GAP = aHb - cHb \qquad\qquad [1]$$

where GAP is a difference between arterial and capillary hemoglobin concentrations, aHb is arterial hemoglobin concentration, cHb is capillary hemoglobin concentration. The same implies to the venous hemoglobin concentration instead of arterial.

2. The stability of the GAP is evaluated by looking at its changes, e.g. during the semi-invasive calibration of noninvasive Hb measuring devices. The deviation of GAP ($\Delta$GAP) is calculated by the following equation:

$$\Delta GAP_1 = GAP_1 - GAP_0 = \Delta aHb_1 - \Delta cHb_1 \qquad\qquad [2]$$

$$\Delta aHb_1 = aHb_1 - aHb_0 \qquad\qquad [3]$$

$$\Delta cHb_1 = cHb_1 - cHb_0 \qquad\qquad [4]$$

$$GAP_1 = GAP_0 + \Delta aHb_1 - \Delta cHb_1 \qquad\qquad [5]$$

where $AGAP_1$ is the deviation of GAP recorded during the calibration protocol, $GAP_1$ is the GAP at the end of the calibration protocol, $GAP_0$ is the GAP at the start of the calibration protocol (baseline value), $\Delta aHb_1$ is the deviation of aHb during the calibration protocol, $\Delta cHb_1$ is the deviation of cHb during the calibration protocol, $aHb_1$ is the aHb at the end of the calibration protocol, $aHb_0$ is the aHb recorded at the start of the calibration protocol (baseline value). The same implies to venous variables.

As an example what can happen to the positive GAP during the fluid infusion such as used for the calibration [FIG.21a] are as follows:

$$\Delta aHb_1 = 18 - 20 = (-2) \qquad\qquad [6]$$

$$\Delta cHb_1 = 2 - 7 = (-5) \qquad\qquad [7]$$

$$\Delta GAP_1 = (-2) - (-5) = 3 \qquad\qquad [8]$$

$$GAP_1 = GAP_0 + \Delta aHb_1 - \Delta cHb_1 = 13 + (-2) - (-5) = 16 \qquad\qquad [9]$$

As an example with negative GAP, data recorded during the calibration protocol [FIG.21b] are as follows:

$$\Delta aHb_1 = 18 - 20 = (-2) \qquad\qquad [10]$$

$$\Delta cHb_1 = 2 - 7 = (-5) \qquad\qquad [11]$$

$$\Delta GAP_1 = (-2) - (-5) = 3 \qquad\qquad [12]$$

$$GAP_1 = GAP_0 + \Delta aHb_1 - \Delta cHb_1 = (-13) + (-2) - (-5) = (-10) \qquad [13]$$

Note that in the latter example, although the deviation of GAP ($\Delta GAP_1$) is positive, it means that the absolute values of the GAP decreased simply because the GAP is negative.

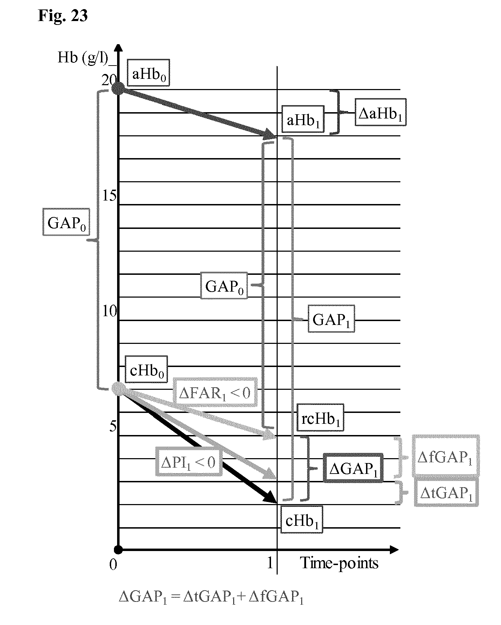

3. The GAP consists of two fractions: (a) the Fahraeus effect related deviation and (b) the FAR related GAP (fGAP). Since changes of the Fahraeus effect related deviations in cHb are reflected by changes in PI, we refer to the related GAP as tGAP. The ratio between the fGAP and tGAP may be changing over time, e.g. the ratio is lower in the GAP before the fluid infusion ($GAP_0$) than after the 5 minutes after its end ($GAP_1$) [FIG.22] Although there is no clinical method to determine their values or even ratio between the fGAP and tGAP, their deviations can be evaluated [FIG. 23] in the shifts that follow a *calibration protocol* proposed by the present invention.

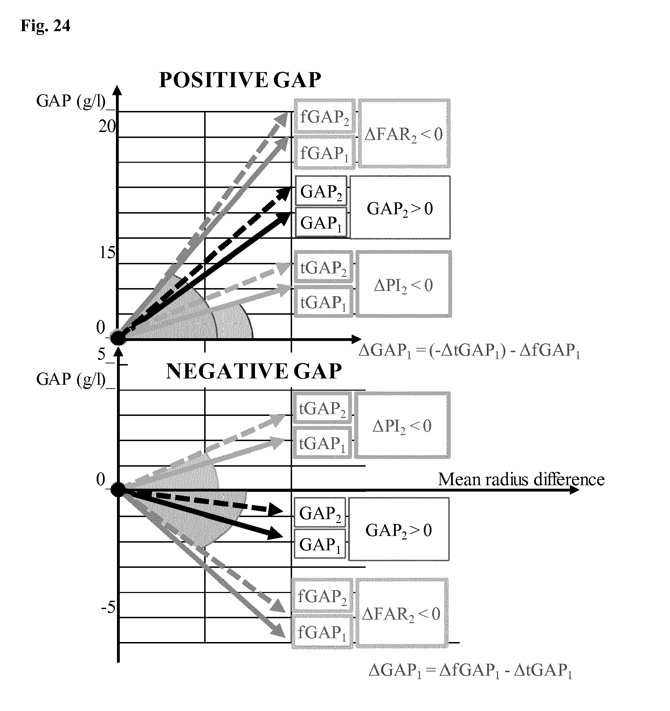

4. The changes in FAR cannot be measured and thus deviation of fGAP ($\Delta fGAP$) and the real capillary Hb (rcHb) cannot be determined even when the initial GAP is known, e.g. from the in vivo adjustment of cHb measures. meanwhile, the deviation of tGAP ($\Delta tGAP$) can be determined from the shift of PI. This is because the relationship between changes in PI ($\Delta PI$) and related changes in GAP ($\Delta GAP$) and cHb ($\Delta cHb$) is linear [FIG.13], and PI increase reflects an increase in the percentage of light absorbed by hemoglobin in macro-capillaries during spectrophotometric measures of cHb. Thus, when PI increases, the fraction of light absorbed in macro-capillaries under the sensor also increases. Since Hb in macro-capillaries (mHb) is closer to Hb in large vessels the increase in PI is allways related to a decrease of GAP [FIG.14]. The GAP is positive when the prevailing capillaries under the sensor are closer to the venular side of the capillary bed, but negative when they are closer to the arteriolar side [FIG.19]. Thus, if the GAP is positive and PI decreases, the cHb decreases and the GAP increases. However, if the GAP is negative, when PI decreases, the cHb value also decreases and the absolute value of the GAP decreases, although GAP remains negative. Simplistically, when PI increases, cHb values allways get closer to aHb or vHb values. The increasing FAR tends to increase the gap when it is positive [FIG.24a]. Its negative deviation ($\Delta FAR<0$) acts synergistically with the negative deviation of PI ($\Delta PI<0$). In contrast, the decreasing FAR and PI both tend to decrease the gap when it is negative [FIG.24b]. Therefore, a $\Delta GAP$ is a result of an interferring affect of $\Delta FAR$ and $\Delta PI$. However, since units of measure for the GAP (g/L), PI (%) anf FAR (ratio) are different, equation [$\Delta GAP = \Delta PI - \Delta FAR$] for the negative GAP and [$\Delta GAP = (-\Delta PI) - \Delta FAR$] for the positive GAP cannot be used to determine the [$\Delta GAP = \Delta tGAP - 4GAP$] and [$\Delta GAP = (-\Delta tGAP) - \Delta fGAP$], accordingly [FIG.23,24]. However, to avoid the conflict of units of measures, the ratios of these variables can be used to determine their relationship. The DGC-math equations are used as follows.

5. Since realtionship between the deviation of PI and cHb is linear, the deviation from that relationship is due to changes in FAR. Thus, a synergistic effect of PI and FAR on the deviation GAP can be determined during the calibration, when the $\Delta GAP_1$ is measured (see equations from 2 to 13).

6. In the **semi-invasive calibration protocol (DGC-1),** both invasive aHb (or vHb) and noninvasive cHb and PI measures during the calibration protocol are required to determine the deviation in FAR expressed in the variable labeled as Ratio of Filtration (ROF).

These are equations used for the calibration:

$$ROG_1 = GAP_1 \cdot GAP_0^{-1} \qquad\qquad [14]$$

where $ROG_1$ is the ratio of GAPs, which is the ratio between $GAP_1$ (the GAP value determined after a deviation of parameters in the calibration protocol) and $GAP_0$ (baseline GAP value in the calibration protocol).

$$ROP_1 = PI_1 \cdot PI_0^{-1} \qquad [15]$$

where $ROP_1$ is ratio of perfusion indexes, which is the ratio between $PI_1$ (the PI value after a deviation of parameters in the calibration protocol) and $PI_0$ (baseline PI value in the calibration protocol).

$$ROF_1 = FAR_1 \cdot FAR_0^{-1} \qquad [16]$$

where $ROF_1$ is ratio of FARs, which is the ratio between $FAR_1$ (the FAR value after a deviation of parameters in the calibration protocol) and $FAR_0$ (baseline FAR value in the calibration protocol).

Since the discrepancy between the $\Delta GAP$ and $\Delta PI$ are related to $\Delta FAR$, the latter equation can be applied:

$$ROF_1 = ABS(ROG_1) - ABS(ROP_1) \qquad [17]$$

The deviations of ROF during upcomming changes are very important - **the increase of ROF shows that the ratio between the fluid entering tissues from capillaries increases in respect the part of it that returns to capillaries**. It is deployed by the mVLT method for the detection of edema formation as explained by the transcapillary reluc model proposed by the present invention.

7. Same equations are used to calculate the above variables in time points after calibration, when noninvasive values are not available, but noninvasive cHb and PI measures are required.

8. **Calculating the PI-adjusted or predicted arterial hemoglobin concentration (paHb).** The predicted GAP (pGAP) needs to be calculated for the estimates of aHb (paHb) or vHb (pvHb) from cHb. First, the net impact of interfering changes in FAR and PI on the GAP has to be determined during the semi-invasive calibration protocol by using the equations as follow:

$$k = ROG_1/ROP_1 \qquad [18]$$

where $k$ is the PI-adjustment coefficient, $ROG_1$ is the ratio of GAPs, which is the ratio between $GAP_0$ (baseline GAP value in the calibration protocol) and $GAP_1$ (the GAP value determined after a deviation of parameters in the calibration protocol), and $ROP_1$ is ratio of perfusion indexes, which is the ratio between $PI_0$ (baseline PI value in the calibration protocol) and $PI_1$ (the PI value after a deviation of parameters in the calibration protocol).

The calculation of the pGAP in the observations after calibration:

$$pGAP_i = GAP_{i-1} + k*ROP_i \qquad [19]$$

where $k$ is the PI-adjustment coefficient, $pGAP_1$ is the predicted difference between the aHb (or vHb) and cHb at data point n.$i$ when invasive measures are not availbale; $GAP_{i-1}$ is the difference between the predicted aHb (or vHb) and cHb; $ROP_i$ is ratio of perfusion indexes, which is the ratio between $PI_i$ (PI value after the shift) and $PI_{i-1}$ (the PI value before the shift).

Calculating the PI-adjusted or namely the predicted aHb (paHb) is as follows:

$$paHb_i = cHb_i + pGAP_i \qquad [20]$$

Since our preliminary observations are made with Radical 7 that has very different averaging times for PI and SpHb, the efficacy of PI-adjustment cannot be fully revealed [see *Clinical cases* section for details].

9. The calibration needs to be applied only once if the sensors stays in the securely fixed location during the cHb monitoring period thereafter.

**The calibration protocol**

1. The **semi-invasive calibration protocol** requires two noninvasive measures of cHb and PI and two invasive arterial or venous blood Hb measures. It implies evaluation of a single simultaneous change in PI, cHb and aHb or vHb that occurs spontaneously or is induced, e.g. by a mini fluid challenge during mVLT. The calibration process begins with obtaining the three measured parameters referred to as **key-3.** That is noninvasively determined cHb and PI, and the invasively measured aHb or vHb when aHb is not available. The key-3 measurements are taken simultaneously. As previously discussed, cHb and PI measurements may be taken by the same sensor or by different sensors examining the same site. In one embodiment, the measurements are taken by the same sensor to ensure that the same microvascular site is the basis for the measurements. Also, the cHb and PI measurements have to be taken simultaneously after the synchronized same duration averaging process. After obtaining the key-3 prameters at the beginning of calibration, another set of key-3 is required for the completion of the calibration protocol. The requirement is that the second set includes at least one variable that has deviated from its baseline value. There are several options to induce a change in cHb or PI, or aHb or vHb or at least one of them: (a) wait untill there is a "naturally" occuring change in at least one variable, (b) induce a change in variables by oral fluid intake, (c) induce a change in variables by a relatively small intravenous infusion of a crystalloid, e.g. use the mini fluid challenge of the mVLT algorithms, (d) induce a change in variables by a diuretic, or (e) induce a change in variables by vasoactive agents such as epinephrine. A set of equations from the **DGC-math** is then used to evaluate the actual individual relationship between the deviations of the key-3 parameters during the calibration. That data are used thereafter for the mathematically derived predicted aHb or vhb values also labelled as PI-adjusted cHb value, from the actual measures of cHb and PI in the observation period after the calibration by using equations from the DGC-math model.

**The DGC method with semi-invasive calibration protocol comprises (DGC-1):**

a) quantifying a subject's initial baseline generic variable(s) (key-3 parameters); b) provoking a deviation of at least one generic variable, e.g by administering an intravenous relatively small test volume load - 2.5 ml per kg of subject's lean body mass of fluid to the subject - an isooncotic, isoosmotic non-cellular liquid is infused at the highest available and clinically appropriate rate with the target of infusing it over a period of 2-5 minutes *(test bolus* in mVLT); alternatively (1) wait untill there is a 'naturally' occuring change in key-3 parameters, or (2) induce a change in key-3 parameters by a diuretic, or (3) induce a change in key-3 parameters by vasoactive agents such as epinephrine, or other means of inducing the changes in at leat one of the key-3 parameters; c) quantifying the subject's key-3 parameters after end of step b)-(1) or b)-(2) or b)-(3) or after a period of 5 minutes from the end of step b), but before 6 minutes from the end of step b) without further intravenous administration of a liquid to the subject; d) referring to the processes in steps a), b) and c) as the calibration protocol; e) determining by DGC-math the deviations of the respective parameters from the two key-3 sets obtained in steps a) and b); f) determining the the predicted aHb or vHb values also labelled as PI-adjusted cHb value, from the actual measures of cHb and PI in the observation period after the calibration by using equations from the DGC-math model.

For the **noninvasive calibration protocol (DGC-2)** requires the same equations but the GAP needs to be derived by methods of statistical mathematical optimisation in a large pool of observations. It requires only two noninvasive measures of cHb and PI. It implies evaluation of a single simultaneous change in PI and cHb that occurs spontaneously or is induced, e.g. by a mini fluid challenge during mVLT. The calibration process begins with obtaining the two measured parameters referred to as **key-2.** That is noninvasively determined cHb and PI. The key-2 measurements are taken simultaneously. As previously discussed, cHb and PI measurements has to be taken by the same sensor or by different sensors examining the same site. In one embodiment, the measurements are taken by the same sensor to ensure that the same microvascular site is the basis for the measurements. Also, the cHb and PI measurements have to be taken simultaneously after the synchronized same duration averaging process. After obtaining the key-2 prameters at the beginning of calibration, another set of key-2 is required for the completion of the calibration protocol. The requirement is that the second set includes at least one variable that has deviated from its baseline value. There are several options to induce a change in cHb or PI, or at least one of them: (a) wait untill there is a "naturally" occuring change in at least one variable, (b) induce a change in variables by oral fluid intake, (c) induce a change in variables by a relatively small intravenous infusion of a crystalloid, e.g. use the mini fluid challenge of the mVLT algorithms, (d) induce a change in variables by a diuretic, or (e) induce a change in variables by vasoactive agents such as epinephrine. A set of equations from the **DGC-math** is then used to evaluate the actual individual relationship between the deviations of the key-2 parameters during the calibration. That data are used thereafter for the mathematically derived PI-adjusted cHb value and the predicted aHb or vHb (paHb and pvHb, accordingly) from the actual noninvasive measures of cHb and PI in the observation period after the calibration.

**The DGC method non-invasive calibration protocol comprises (DGC-2):** a) quantifying a subject's initial baseline generic variable(s) (key-2 parameters); b) provoking a deviation of at least one generic variable, e.g by administering an intravenous relatively small test volume load - 2.5 ml per kg of subject's lean body mass of fluid to the subject-an isooncotic, isoosmotic non-cellular liquid is infused at the highest available and clinically appropriate rate with the target of infusing it over a period of 2-5 minutes (*test bolus* in mVLT); alternatively (1) wait untill there is a 'naturally' occuring change in key-2 parameters, or (2) induce a change in key-2 parameters by a diuretic, or (3) induce a change in key-2 parameters by vasoactive agents such as epinephrine, or other means of inducing the changes in at leat one of the key-2 parameters; c) quantifying the subject's key-2 parameters after end of step b)-(1) or b)-(2) or b)-(3) or after a period of 5 minutes from the end of step b), but before 6 minutes from the end of step b) without further intravenous administration of a liquid to the subject; d) referring to the processes in steps a), b) and c) as the calibration protocol; e) determining by DGC-math the deviations of the respective parameters from the two key-3 sets obtained in steps a) and b); f) determining the the predicted aHb or vHb values also labelled as PI-adjusted cHb value, from the actual measures of cHb and PI in the observation period after the calibration by using equations from the DGC-math model.

**The transcapillary reflux model**

The current invention proposes a **transcapillary reflux model** based on known physiology to explain the clinical relevance of variables derived from the above models. One condition is that aPD is considered to reflect the distribution of infused fluid throughout the entire body while cPD reflects the local transcapillary fluid equilibration at the site of observation.

Fig.25 shows a steady-state situation. Fluid is consumed only orally as triggered by thirst and fluid leaves the system through basal renal elimination. The aPD exceeds both venous plasma dilution (vPD) (arterio venous dilution difference, avDD > 0) and cPD (arterio capillary dilution difference, acDD > 0) since the arbitrary transcapillary filtration absorption ratio (FAR) pattern N.1 ($FAR_1$) is 2:1. Transcapillary fluid exchange only occurs in the smallest of capillaries. Normally, FAR > 1.0 is necessary for lymph production. As shown, there is no interstitial fluid accumulation when the lymphatic flux into central veins matches the net volume difference between transcapillary fluid filtration and absorption.

Fig.26 shows that fluid infusion causes interstitial fluid accumulation and an increase in both avDD and acDD. An increase in net transcapillary pressure leads to an increase in FAR, here it arbitrarily increased from pattern N.1 ($FAR_1$=2:1 in figure 25) to pattern N.2 ($FAR_2$=3:1). Subsequently an increase in FAR leads to an increase in both - interstitial fluid volume and compliance. The increase in compliance maintains a stable interstitial hydrostatic pressure and lymphatic flux, respectively. Eventually, when the interstitial fluid compliance reaches its physiological maximum, all of the infused fluid can be trapped in the interstitium while renal elimination may remain unchanged. This situation might represent the point where hydration of tissues is not necessary any longer.

Fig.27 shows that avDD and acDD change from positive to negative and FAR arbitrarily decreases from pattern N.2 ($FAR_2$=3:1) to pattern N.3 ($FAR_3$=1:2) when the prevailing fluid extravasation from capillaries into tissues changes to prevailing fluid reflux from tissues into capillaries. In this scenario, renal elimination has increased to compensate for the increased fluid return from the interstitium, *the transcapillary reflux.* Consequently, aPD remains stable. This is equivalent to the dilution plateau described in a refined mVLT method proposed by present invention.

**Application of DGC method for performing the reined mVLT method**

A stepwise intravenous infusion according is used in conventional mVLT protocol [FIG.8, 28]. In brief, this consists of stepwise IV infusion. Specifically, the consecutive mini fluid challenges are performed. These arre relatively small 1.5-5.0 ml·kg$^{-1}$ boluses of crystalloid solution infused over 3-5 min followed by 5 min periods without fluid.

Addressing deficiencies within existing methods, the current invention **proposes new variables to the mVLT for the increased specificity and sensitivity in determining and optimizing the state of hydration.** The pcHb value and paHb or pvHb are proposed when invasive Hb measures are not applicable or available. Evaluating of the GAP between the pcHb and either aHb, paHb, vHb or pvHb is proposed for the evaluation of changes in FAR and detection of imminent edema during mVLT. Also, as previously suggested {26}, instead of monitoring PD trends and detecting *dilution plateau* during conventional mVLT algorithm, the monitoring of *plasma dilution efficacy* (PDE) of consecutive mini fluid challenges is proposed. The PDE can be calculated as follows:

$$PDE_i = (PD_i + 1) \cdot (PD_{i-1} + 1)^{-1} - 1 \qquad\qquad [5]$$

where $PDE_i$ is the *plasma dilution efficacy* of the fluid challenge number *i*, $PD_i$ is the plasma dilution at the end of fluid challenge number *i*, and $PD_{i-1}$ is the plasma dilution at the end of the preceding fluid challenge.

Fig. 29 shows an illustration of the aPD and aPDE of three mini fluid challenges. The aPDE after the first mini fluid challenge decreases and becomes negligible after the third fluid challenge. This is an indication that net fluid extravasation is equal to net volume of infused fluid entering the circulation. The fluid infusion is no longer contributes for diluting the blood at this point. By looking at aPDE it is, however, not possible to fully clarify where the fluid goes. A decreasing aPDE tells us that the fluid is moves out of circulation. The fluid can either be eliminated via the renal system or it can be accumulated in tissues, or a combination of both. That is why the measurement of cHb is necessary. The difference between aPDE and cPDE, acPED, and its change during stepwise fluid infusion, will clarify the impact of interstitial fluid accumulation on net fluid extravasation [FIG.27]. The minimized arterial PDE [FIG. 29] would serve as a marker of optimized hydration status instead of hard to detect *dilution plateau.* It would be the PDE value after the mini fluid challenge where, ideally, the *dilution plateau* is detected in the PD trend.

The difference between the PDE variables derived from different generic parameters, actually measured or predicted, is referred to as plasma dilution efficacy difference (PED), e.g. the difference between the PDE derived from aHb (aPDE) and PDE derived from cHb (cPDE) is referred to as arterio-capillary plasma dilution efficacy difference (acPED). The current invention **expands the set of diagnostic criteria for the optimisation of body hydration status and imminent edema** defining that state as a setting when at least one of the following criteria is met during a stepwise crystalloid infusion: (a) the aPDE or paPDE is negligible (close to zero), also the same for the venous variables when arterial are not available [FIG. 29], (b) the cPDE or predicted arterial PDE (paPDE) is similarly minimized (negligible decrease or increase or unchanged), (c) the acPED changes from positive to negative considering that paHb variable can be used instead of aHb for the calculation [FIG. 30], also venous valriables may be used instead, or (d) the decreasing ROF values during show the decreasing fluid movement from capillaries into tissues (decreasing FAR), and tissues are getting more and more expanded by fluids, and imminent edema may be present when ROF turns from positive to negative. The proposed transcapillary reflux model explains the clinical relevance of the variables. By means of the DGC method the mVLT becomes more specific and sensitive also more clinically applicable. The use of the DGC method expands the applicability of the mVLT method in the automated decision-support systems, also semi-closed or closed loop infusion systems.

The DGC method applies to all devices which provide simultaneous measurements of cHb and PI via the same noninvasive sensor or via different sensors which measure cHb and PI. As used herein, SpHb and PI are referrences to cHb and PI which are measured at the same examination site and the synchronized - equal and simultaneous - averaging time is applied to these measures to calculate the variables that are displayed on the screen of the measuring device or can be obtained on-line. However, the currently available devices apply different averaging times for cHb and PI measures. That makes reliable evaluation of relationship between simultaneous $\Delta PI$ and $\Delta cHb$ impossible. Presuming that this drawback is eliminated, the PI proposed adjustment of cHb could be implemented as an addition to the currently used in vivo adjustment. Modification of software in the noninvasive cHb measuring devices is therefore required to provide the variable synchronized averaging of PI and cHb variables.

**Application of the modified mVLT protocol in automated systems**

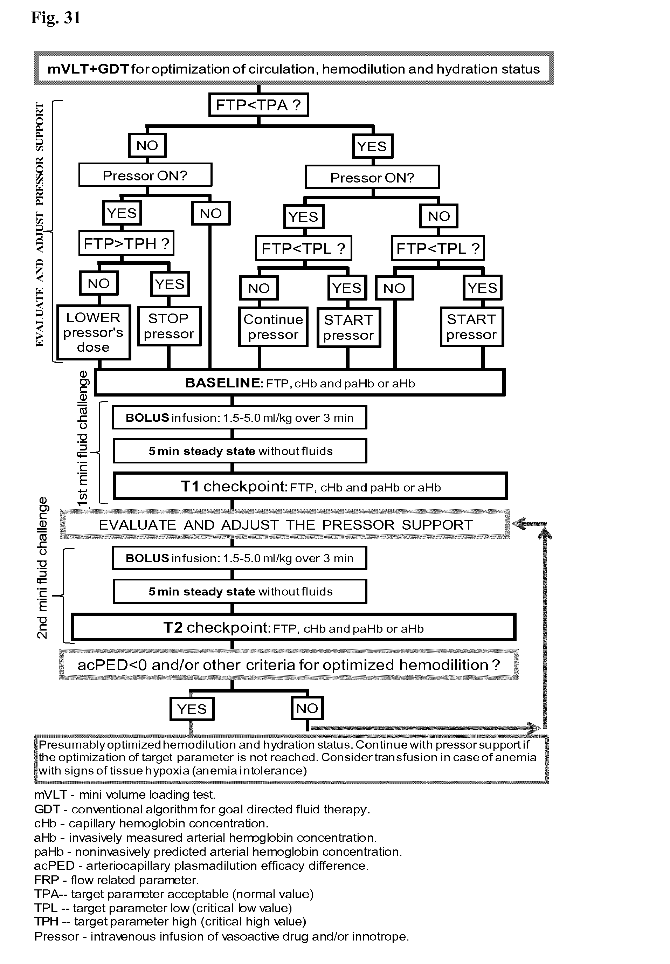

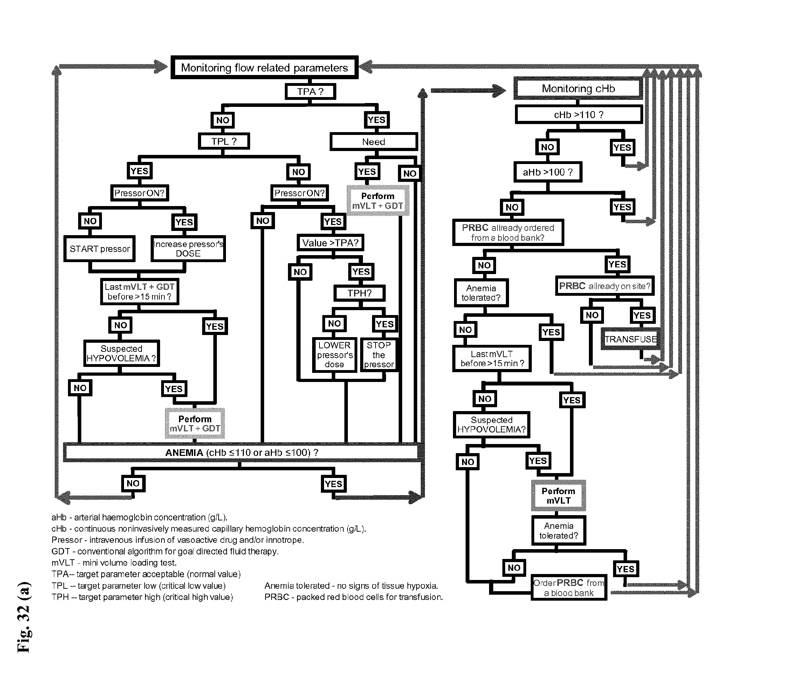

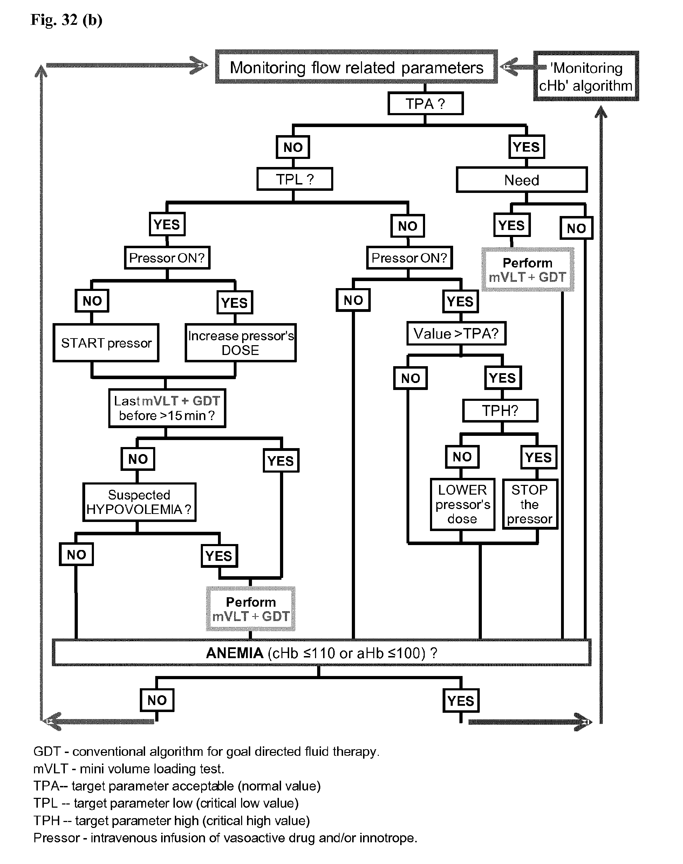

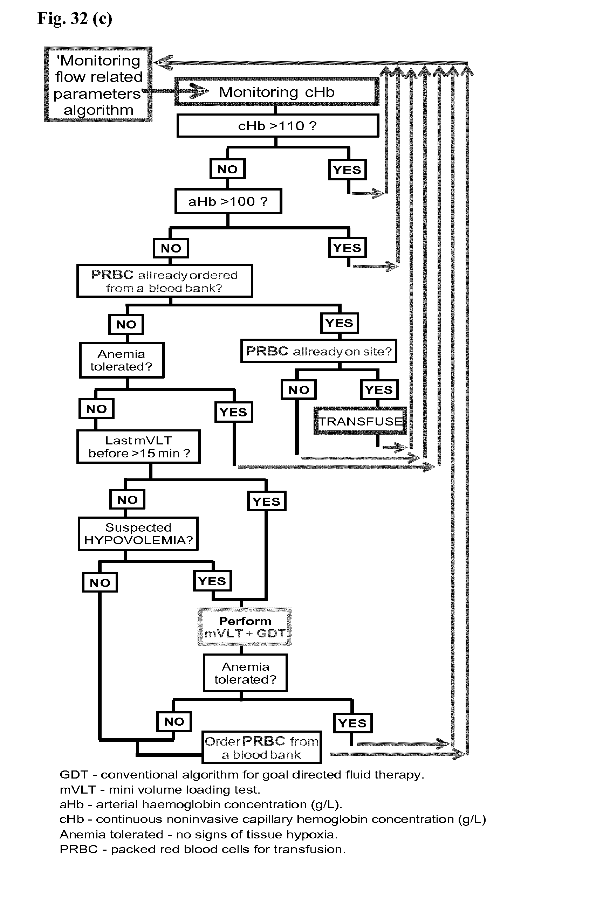

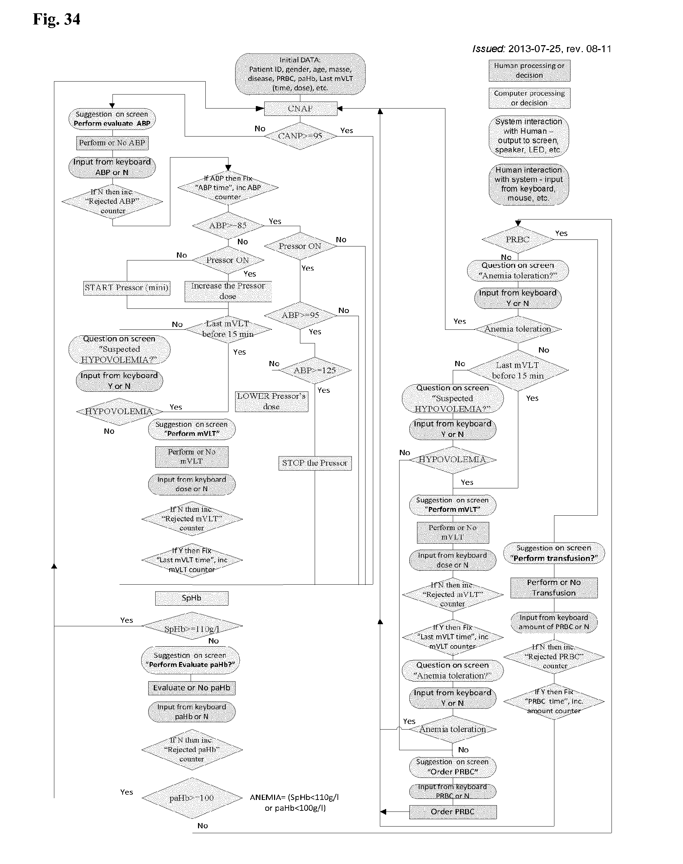

The possible noninvasiveness of the mVLT methodology opens an opportunity for implementing it in decision support systems and even semi-close or closed loop infusion systems. Algorithms ranging from simplistic optimization of hydration status [FIG. 28] to the sophisticated optimization of both hemodynamics and hydration [FIGS. 31-33] can be clinically relevant. Any other target parameter can be used instead of arterial pressure for the same purpose. These algorithms can be used in the decision support systems. A semi closed loop device with an interface of cHb measuring in combination with a high-volume infusion pump guided by a computer would be justified [FIGS. 32, 34]. In a semi-closed loop infusion system, it would permit bidirectional communication with a physician performing the mVLT protocol. A clinician could prompt certain actions such as administer additional mini fluid challenges based on the cHb data analysis described. We suggest that the mVLT can be added to current algorithms of goal-directed fluid therapy. As a supplement, the mVLT would be used to determine the time point during hemodynamic optimization when no more fluid should be given to prevent edema. Moreover, because mVLT requires stepwise fluid infusion similar to what is used in goal-directed fluid therapy, both hemodynamic and plasma dilution evaluations could be performed simultaneously. Clinical applications of closed loop systems for hemodynamic optimization have been suggested and the mVLT could be added and tried in these algorithms. These algorithms can be used in the decision support systems.

Current invention proposes:

1. **Dilution Guided Correction method (DGC method).** It consists of three models and the calibration protocol.

2. **Dynamic gap model** - for the mathematical modelling of changes in GAP, which is the difference between hemoglobin concentration in large blood vessels and capillaries. The dynamic gap model explains the anatomical and physiological origin of the GAP, and explains why there is a significant discrepancy between the invasively and noninvasively measured hemoglobin concentrations (Hb). It is the model for the DGC math equations. Unique model.

3. **Dilution Guided Correction mathematical method (DGC-math)** proposed the calibration protocols for the noninvasive Hb measuring devices. The calibration process and math behind it is unique. The invasively and/or noninvasively measured variables Hb and perfusion index (PI) are required for the calibration. Variables derived during calibration is used for the on-line individual adjustment of noninvasively measured hemoglobin concentration (cHb) values aiming to predict the invasively measured arterial (aHb) or venous (vHb) hemoglobin concentration values from noninvasive measurements. The calibration protocols and equations from 14 to 20 are unique, and to be patented.

4. Applying the DGC-math **has refined mini Volume Loading Test (mVLT)** by enabling its minimally invasive and noninvasive implication. The new variables expanded and refined the list of criteria for the detection of imminent edema during fluid administration, also monitoring the diuretic action and optimization of fluid status and hemodynamics.

5. **The transcapillary fluid reflux model** has proposed the new clinically relevant variables that were developed into SDC-math equations mentioned above. It expands the existing methods to monitor the hydration status and transcapillary fluid movement.

6. The proposed unique **clinical algorithms** can serve as background for the software in clinical decision support systems, and semi-closed or closed loop infusion systems could be patented along with a description of related devices or something.

A. The semi-invasive calibration protocol requires two noninvasive measures of cHb and PI and two invasive arterial or venous blood Hb measures. It implies evaluation of a single simultaneous change in PI, cHb and aHb or vHb that occurs spontaneously or is induced, e.g. by a mini fluid challenge during mVLT. The calibration process begins with obtaining the three measured parameters referred to as **key-3.** That is noninvasively determined cHb and PI, and the invasively measured aHb or vHb when aHb is not available. The key-3 measurements are taken simultaneously. As previously discussed, cHb and PI measurements may be taken by the same sensor or by different sensors examining the same site. In one embodiment, the measurements are taken by the same sensor to ensure that the same microvascular site is the basis for the measurements. Also, the cHb and PI measurements have to be taken simultaneously after the synchronized same duration averaging process. After obtaining the key-3 parameters at the beginning of calibration, another set of key-3 is required for the completion of the calibration protocol. The requirement is that the second set includes at least one variable that has deviated from its baseline value. There are several options to induce a change in cHb or PI, or aHb or vHb or at least one of them: (a) wait until there is a "naturally" occurring change in at least one variable, (b) induce a change in variables by oral fluid intake, (c) induce a change in variables by a relatively small intravenous infusion of a crystalloid, e.g. use the mini fluid challenge of the mVLT algorithms, (d) induce a change in variables by a diuretic, or (e) induce a change in variables by vasoactive agents such as epinephrine. A set of equations from the **DGC-math** is then used to evaluate the actual individual relationship between the deviations of the key-3 parameters during the calibration. That data are used thereafter for the mathematically derived predicted aHb or vHb values also labeled as PI-adjusted cHb value, from the actual measures of cHb and PI in the observation period after the calibration by using equations from the DGC-math model.

**The DGC method with semi-invasive calibration protocol comprises (DGC-1):**

a) quantifying a subject's initial baseline generic variable(s) (key-3 parameters);

b) provoking a deviation of at least one generic variable, e.g by administering an intravenous relatively small test volume load - 2.5 ml per kg of subject's lean body mass of fluid to the subject - an isooncotic, isoosmotic non-cellular liquid is infused at the highest available and clinically appropriate rate with the target of infusing it over a period of 2-5 minutes (*test bolus* in mVLT); alternatively (1) wait until there is a 'naturally' occurring change in key-3 parameters, or (2) induce a change in key-3 parameters by a diuretic, or (3) induce a change in key-3 parameters by vasoactive agents such as epinephrine, or other means of inducing the changes in at least one of the key-3 parameters;

c) quantifying the subject's key-3 parameters after end of step b)-(1) or b)-(2) or b)-(3) or after a period of 5 minutes from the end of step b), but before 6 minutes from the end of step b) without further intravenous administration of a liquid to the subject;

d) referring to the processes in steps a), b) and c) as the calibration protocol;

e) determining by DGC-math the deviations of the respective parameters from the two key-3 sets obtained in steps a) and b);

f) determining the the predicted aHb or vHb values also labeled as PI-adjusted cHb value, from the actual measures of cHb and PI in the observation period after the calibration by using equations from the DGC-math model.

B. For the **noninvasive calibration protocol (DGC-2)** requires the same equations but the GAP needs to be derived by methods of statistical mathematical optimization in a large pool of observations. It requires only two noninvasive measures of cHb and PI. It implies evaluation of a single simultaneous change in PI and cHb that occurs spontaneously or is induced, e.g. by a mini fluid challenge during mVLT. The calibration process begins with obtaining the two measured parameters referred to as **key-2.** That is noninvasively determined cHb and PI. The key-2 measurements are taken simultaneously. As previously discussed, cHb and PI measurements has to be taken by the same sensor or by different sensors examining the same site. In one embodiment, the measurements are taken by the same sensor to ensure that the same microvascular site is the basis for the measurements. Also, the cHb and PI measurements have to be taken simultaneously after the synchronized same duration averaging process. After obtaining the key-2 prameters at the beginning of calibration, another set of key-2 is required for the completion of the calibration protocol. The requirement is that the second set includes at least one variable that has deviated from its baseline value. There are several options to induce a change in cHb or PI, or at least one of them: (a) wait untill there is a "naturally" occuring change in at least one variable, (b) induce a change in variables by oral fluid intake, (c) induce a change in variables by a relatively small intravenous infusion of a crystalloid, e.g. use the mini fluid challenge of the mVLT algorithms, (d) induce a change in variables by a diuretic, or (e) induce a change in variables by vasoactive agents such as epinephrine. A set of equations from the **DGC-math** is then used to evaluate the actual individual relationship between the deviations of the key-2 parameters during the calibration. That data are used thereafter for the mathematically derived PI-adjusted cHb value and the predicted aHb or vHb (paHb and pvHb, accordingly) from the actual noninvasive measures of cHb and PI in the observation period after the calibration.

**The DGC method non-invasive calibration protocol comprises (DGC-2):**

quantifying a subject's initial baseline generic variable(s) (key-2 parameters);

provoking a deviation of at least one generic variable, e.g by administering an intravenous relatively small test volume load - 2.5 ml per kg of subject's lean body mass of fluid to the subject - an isooncotic, isoosmotic non-cellular liquid is infused at the highest available and clinically appropriate rate with the target of infusing it over a period of 2-5 minutes (*test bolus* in mVLT); alternatively (1) wait untill there is a 'naturally' occuring change in key-2 parameters, or (2) induce a change in key-2 parameters by a diuretic, or (3) induce a change in key-2 parameters by vasoactive agents such as epinephrine, or other means of inducing the changes in at leat one of the key-2 parameters;

quantifying the subject's key-2 parameters after end of step b)-(1) or b)-(2) or b)-(3) or after a period of 5 minutes from the end of step b), but before 6 minutes from the end of step b) without further intravenous administration of a liquid to the subject;

referring to the processes in steps a), b) and c) as the calibration protocol;

determining by DGC-math the deviations of the respective parameters from the two key-3 sets obtained in steps a) and b);

f) determining the the predicted aHb or vHb values also labelled as PI-adjusted cHb value, from the actual measures of cHb and PI in the observation period after the calibration by using equations from the DGC-math model.

The current invention **expands the set of diagnostic criteria for the optimisation of body hydration status and imminent edema** defining that state as a setting when at least one of the following criteria is met <u>during any stepwise crystalloid infusion and especially **in the mVLT**</u>: (a) the aPDE or paPDE is negligible (close to zero), also the same for the venous variables when arterial are not available [FIG. 29], (b) the cPDE or predicted arterial PDE (paPDE) is similarly minimized (negligible decrease or increase or unchanged), (c) the acPED changes from positive to negative considering that paHb variable can be used instead of aHb for the calculation [FIG. 30], also venous valriables may be used instead, or (d) the decreasing ROF values during show the decreasing fluid movement from capillaries into tissues (decreasing FAR), and tissues are getting more and more expanded by fluids, and imminent edema may be present when ROF turns from positive to negative. The proposed transcapillary reflux model explains the clinical relevance of the variables. By means of the DGC method the mVLT becomes more specific and sensitive also more clinically applicable. The use of the DGC method expands the applicability of the mVLT method in the automated decision-support systems, also semi-closed or closed loop infusion systems.

**A computing device and application**

The present invention provides a computing device comprising a memory, a processor, and an application stored in the memory that, when executed by the processor, performs a calibration method for real-time adjustment of noninvasive hemoglobin measurements. In some embodiments, the application may further provide for optimization of fluid and transfusion management as a refinement of a Multimodal Feedback Loop (MFL) algorithm based on the simultaneous evaluation of different target parameters as proposed in the mVLT method described in reference {18}.

The Multimodal Feedback Loop (MFL) algorithm may be based on the simultaneous evaluation of simultaneous response of multiple target parameters to the fluid load by simultaneous monitoring of trends described by the BIRD model described in reference {18}. The application may employ mathematical models of the MFL algorithm and mVLT.

The computing device may comprise any type of device. The computing device may include any number of data inputs and dataoutputs. For example, the device may comprise one main input and several optional inputs, and one main output and several optional outputs. In some embodiments, the main input comprises one or more independent modules for entering hemoglobin concentration data. For example, the main input may comprise a manual module and an automated module for entering the hemoglobin concentration data, which may be recorded at specific checkpoints during the processes used in mVLT. Hemoglobin data input may have three arms since hemoglobin concentration can be obtained from arterial and/or venous and/or microvascular blood depending on available and applicable invasive and/or noninvasive methods. In some embodiments, automated hemoglobin data input can be provided via interface with devices that provide an electronic value of the parameter. Otherwise, hemoglobin data can be entered via manual input portal. The application may be configured to adjust any of the hemoglobin inputs according to the procedures described herein. For example, a noninvaseively measured hemoglobin input may be calibrated and adjusted in real time according to equation [7]. Optional inputs are divided into feedback and data input terminals. The feedback input is for the feedback interface with fluid and transfusion pumps. The data input - manual and automated - includes but is not limited to flow-related parameters such as cardiac stroke volume, end diastolic filling ratio (EDFR), global end diastolic volume (GEDV) and systemic vascular resistance (SVR). Main output is for digital outflow of all data that is entering input terminals, and also the data derived from mathematical processing by the processor executing the application of input data according to the MFL algorithm and mVLT. More specifically, the later output provides definition of the suggested preexisting fluid status before and after mVLT, also suggesting further measures targeted to reach and/or maintain the specific fluid status. Optional output is divided into three operational interfaces with the three branches of infusion systems: crystalloid, colloid and blood product infusing pumps. These branches are further divided into specific interfaces according to the type of fluid and blood product. The main purpose of the operational interface is the guidance of isoosmotic crystalloid infusion pumps so that they administer fluid boluses at the rate defined by mVLT protocol and in accordance with the predetermined steady states between boluses.

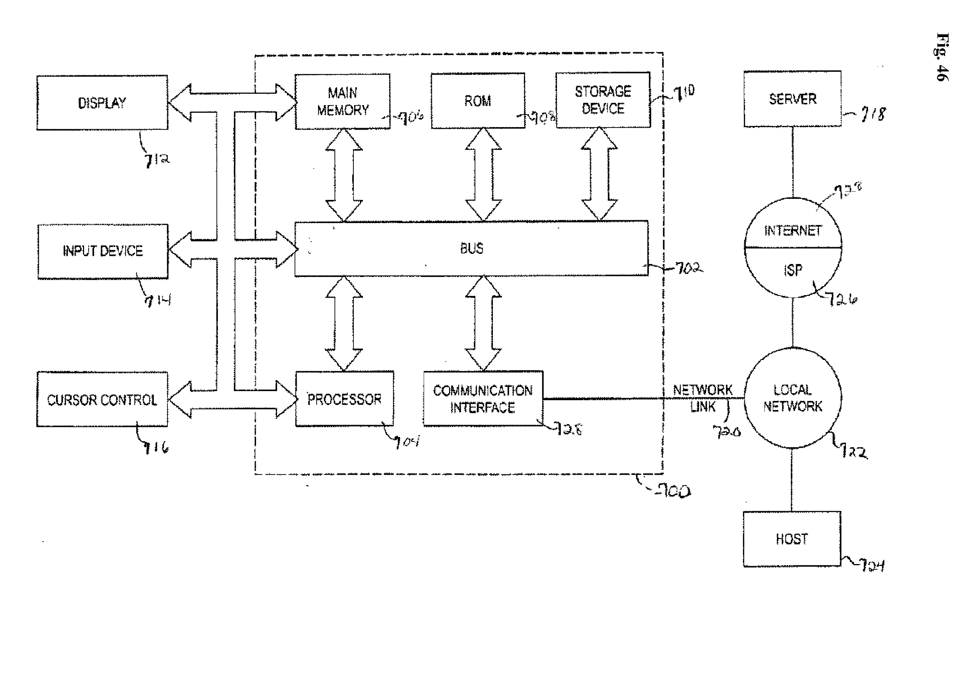

FIG. 46 is a block diagram that illustrates a computer system 700 upon which an embodiment of the invention may be implemented. Computer system 700 includes a bus 702 or other communication mechanism for communicating information, and a processor 704 coupled with bus 702 for processing information. Computer system 700 also includes a main memory 706, such as a random access memory (RAM) or other dynamic storage device, coupled to bus 702 for storing information and instructions to be executed by processor 704. Main memory 706 also may be used for storing temporary variables or other intermediate information during execution of instructions to be executed by processor 704. Computer system 700 further includes a read only memory (ROM) 708 or other static storage device coupled to bus 702 for storing static information and instructions for processor 704. A storage device 710, such as a magnetic disk or optical disk, is provided and coupled to bus 702 for storing information and instructions. Computer system 700 may be coupled via bus 702 to a display 712, such as a cathode ray tube (CRT) or Liquid Crystal Display (LCD), for displaying information to a computer user. An input device 714, including alphanumeric and other keys, is coupled to bus 702 for communicating information and command selections to processor 704. Another type of user input device is cursor control 716, such as a mouse, a trackball, or cursor direction keys for communicating direction information and command selections to processor 704 and for controlling cursor movement on display 712. This input device can have two or more degrees of freedom in two axes, e.g. a first axis (e.g., x) and a second axis (e.g., y), that allows the device to specify positions in a plane.

The invention is related to the use of computer system 700 for determining blood states for patients and their doctors. According to one embodiment of the invention, parameters for determining blood states is provided by computer system 700 in response to processor 704 executing the application contained in main memory 706 and comprising one or more sequences of one or more instructions. Such instructions may be read into main memory 706 from another computer-readable medium, such as storage device 710. Execution of the sequences of instructions contained in main memory 706 causes processor 704 to perform the process steps described herein. One or more processors in a multi-processing arrangement may also be employed to execute the sequences of instructions contained in main memory 706. In alternative embodiments, hardwired circuitry may be used in place of or in combination with software instructions to implement the invention. Thus, embodiments of the invention are not limited to any specific combination of hardware circuitry and software.

The term "computer-readable medium" as used herein refers to any medium that participates in providing instructions to processor 704 for execution. Such a medium may take many forms, including but not limited to, non-volatile media, volatile media, and transmission media. Non- volatile media includes, for example, optical or magnetic disks, such as storage device 710. Volatile media includes dynamic memory, such as main memory 706. Transmission media includes coaxial cables, copper wire and fiber optics, including the wires that comprise bus 702. Transmission media can also take the form of acoustic or light waves, such as those generated during radio wave and infrared data communications.

Common forms of computer-readable media include, for example, a floppy disk, a flexible disk, hard disk, magnetic tape, or any other magnetic medium, a CD-ROM, any other optical medium, punch cards, paper tape, any other physical medium with patterns of holes, a RAM, a PROM, and EPROM, a FLASH-EPROM, any other memory chip or cartridge, a carrier wave as described hereinafter, or any other medium from which a computer can read.

Various forms of computer readable media may be involved in carrying one or more sequences of one or more instructions to processor 704 for execution. For example, the instructions may initially be carried on a magnetic disk of a remote computer. The remote computer can load the instructions into its dynamic memory and send the instructions over a telephone line using a modem. A modem local to computer system 700 can receive the data on the telephone line and use an infrared transmitter to convert the data to an infrared signal. An infrared detector coupled to bus 702 can receive the data carried in the infrared signal and place the data on bus 702. Bus 702 carries the data to main memory 706, from which processor 704 retrieves and executes the instructions. The instructions received by main memory 706 may optionally be stored on storage device 710 either before or after execution by processor 704.

Computer system 700 also includes a communication interface 718 coupled to bus 702. Communication interface 718 provides a two-way data communication coupling to a network link 720 that is connected to a local network 722. For example, communication interface 718 may be an integrated services digital network (ISDN) card or a modem to provide a data communication connection to a corresponding type of telephone line. As another example, communication interface 718 may be a local area network (LAN) card to provide a data communication connection to a compatible LAN. Wireless links may also be implemented. In any such implementation, communication interface 718 sends and receives electrical, electromagnetic or optical signals that carry digital data streams representing various types of information.

Network link 720 typically provides data communication through one or more networks to other data devices. For example, network link 720 may provide a connection through local network 722 to a host computer 724 or to data equipment operated by an Internet Service Provider (ISP) 726. ISP 726 in turn provides data communication services through the world wide packet data communication network now commonly referred to as the "Internet" 728. Local network 722 and Internet 728 both use electrical, electromagnetic or optical signals that carry digital data streams. The signals through the various networks and the signals on network link 720 and through communication interface 718, which carry the digital data to and from computer system 700, are exemplary forms of carrier waves transporting the information.

Computer system 700 can send messages and receive data, including program code, through the network(s), network link 720 and communication interface 718. In the Internet example, a server 730 might transmit a requested code for an application program through Internet 728, ISP 726, local network 722 and communication interface 718. In accordance with the invention, one such downloaded application provides for the calculating of transfusion strategies as described herein.

The received code may be executed by processor 704 as it is received, and/or stored in storage device 710, or other non- volatile storage for later execution. In this manner, computer system 700 may obtain application code in the form of a carrier wave.

While this invention has been described in connection with the best mode presently contemplated by the inventor for carrying out his invention, the preferred embodiments described and shown are for purposes of illustration only, and are not to be construed as constituting any limitations of the invention. Modifications will be obvious to those skilled in the art, and all modifications that do not depart from the spirit of the invention are intended to be included within the scope of the appended claims. Those skilled in the art will appreciate that the conception upon which this disclosure is based, may readily be utilized as a basis for the designing of other structures, methods and systems for carrying out the several purposes of the present invention. It is important, therefore, that the claims be regarded as including such equivalent constructions insofar as they do not depart from the spirit and scope of the present invention. With respect to the above description then, it is to be realized that the optimum dimensional relationships for the parts of the invention, including variations in size, materials, shape, form, function and manner of operation, assembly and use, and all equivalent relationships to those illustrated in the drawings and described in the specification, that would be deemed readily apparent and obvious to one skilled in the art, are intended to be encompassed by the present invention. Therefore, the foregoing is considered as illustrative only of the principles of the invention. Further, since numerous modifications and changes will readily occur to those skilled in the art, it is not desired to limit the invention to the exact construction and operation shown and described, and accordingly, all suitable modifications and equivalents may be resorted to, falling within the scope of the invention.

Despite improvements in techniques and skills, the outcome of treatment after major surgery remains partly dependent on the overall perioperative optimisation of the patient. Optimisation procedures are labour-intensive, stressful to care providers and require simultaneous monitoring of multiple parameters. Nevertheless, optimisation targets may still be missed.

Typical risk reduction measures are not sufficient to reduce the probability of errors resulting from operator errors. Improvement of the device software and hardware, adding more alerts and alarms is not enough to significantly reduce the risk of such types of hazards, so there is a need for new methods to improve the safety of medical devices.

Closed loop systems are the ultimate solution to ensure that optimal therapies are delivered in a timely manner. The objectives such as keeping the perioperative arterial blood pressure (ABP) and optimised haemodilution in the preset safe limits can be achieved semi-automatically. The mathematical modelling cannot account for all the environmental factors, but it may be helpful for clinicians in dealing with the complexity of decision-making and applying treatment in a timely manner. Integrating patient monitoring, fluid and drug infusion devices into one system and using a controller, which would be able to assess the requirements for a specific treatment according to patients' vital signs can make it possible to reduce the probability of hazards arising from the lack of appropriate timely reaction. Controllers such as PID, Fuzzy logic, Artificial neural networks, Rule-based, Model-based and others can be used to fit the specific requirements.

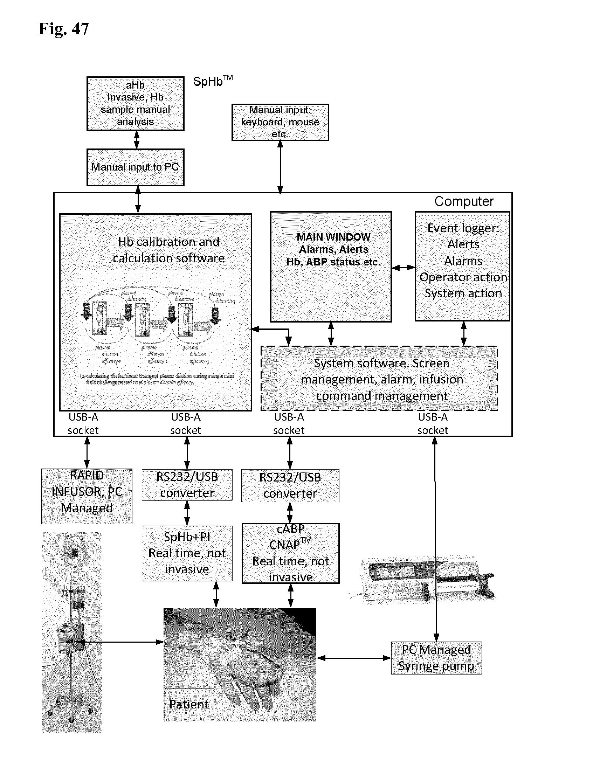

Perioperative patient optimisation at minimum requires maintenence of normal arterial blood pressure (ABP). This can be achieved by applying continuous ABP monitoring and administration of fluid infusion and medication in case of arterial hypotension. A semi-closed loop system could enable administration of treatment in a timely manner and reduce the stress levels of care-givers. Fig. 47 shows a semi-closed loop system infusion system consisting of ABP and Hb measuring devices, algorithmic data analysis and generating the commands for the pumps to perform the vasopressor injection and infuse fluids and blood, according to an embodiment.

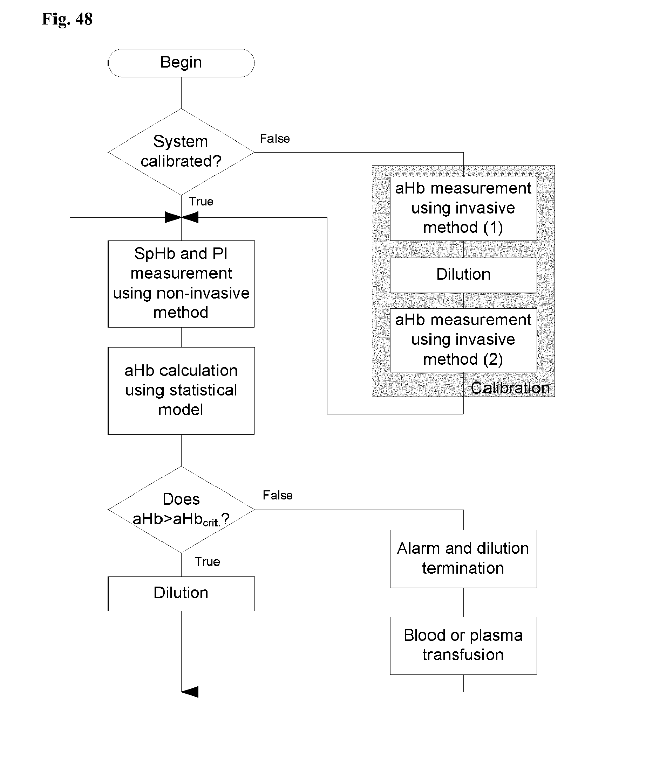

One of the system components - a closed loop vasopressor drug injection system - has been recently put to the test. It used a continuous noninvasive arterial pressure measurement device (CNAPTM; Draeger Medical, Germany) connected to a laptop computer. When CNAP fell below a preset limit, the computer automatically delivered injection commands to the two syringe pumps pre-filled with vasopressors. According to an embodiment, the software in a computer of the semi-closed loop system of the present disclosure implements the extended clinical algorithm for generating the following commands: (1) administer the vasopressor injection (automated, but can be objected), (2) perform the mVLT fluid protocol (semi-automated, may require physician's approval), (3) administer maintenance crystalloid infusion (semi-automated, may require physicians approval), (4) perform the red cell transfusion (semi-automated, may require physician's approval). According to the mVLT methodology, when fluid infusion reaches the point where the ability of an infusion to advance haemodilution is minimised, it functions as confirmation of optimised haemodilution. Fluid boluses are not justified for ABP management after that point. The transfusion is considered if signs of anemia intolerance persist after the optimisation of haemodilution and ABP. To enable the more reliable noninvasive monitoring of anemia, the system implements a calibration protocol according to the statistically biased calibration method, according to an emboidment, which is shown in Fig. 48.

Graphic displays will also help clinicians to better capture and integrate the multivariable clinical information. This may lead to faster and more accurate diagnosis and therapeutic decision-making

In some embodiments, disclosed is a method for adjusting a noninvasively measured total hemoglobin measurement. The method may comprise: (a) noninvasively obtaining a plurality of sets of measurements obtained over a period of time, each of the sets including a total hemoglobin measurement and a perfusion index measurement; (b) determining a plasma dilution based on the noninvasively obtained total hemoglobin measurements; (c) determining a perfusion index deviation based on the noninvaseively obtained perfusion index measurements; (d) determining a plasma dilution efficacy based on the plasma dilution determined in (b); (e) determining a perfusion index efficacy based on the perfusion index deviation determined in (c);

(f) determining a total hemoglobin adjustment coefficient based on the plasma dilution efficacy determined in (d) and the perfusion index efficacy determined in (e); (g) determining an adjusted deviation of total hemoglobin based on a noninvasively obtained total hemoglobin measurement, an invaseively obtained hemoglobin concentration measurement, the plasma dilution efficacy determined in (d) and the total hemoglobin adjustment coefficient determined in (f); and (h) adjusting at least one of the the noninvasively obtained total hemoglobin measurements obtained in (a) based on the adjusted deviation of total hemoglobin determined in (g). In some embodiments, the measurements comprise a set of measurements are obtained simultaneously and from a same sensor. In some embodiments, step (b) comprises determining the plasma dilution according to equation [11]: $cPD_i = (SpHb \cdot SpHb_i - 1) \cdot (1 - Hct)^{-1}$ [11] where $_cPD_i$ is plasma dilution of non-adjusted total hemoglobin (SpHb) at checkpoint $i$ and Hct is an initial baseline value of hematocrit. In some embodiments, step (c) comprises determining the perfusion index deviation calculated according to equation [12]: $PID_i = (PI - PI_i) \cdot PI^{-1}$ [12] where $PID_i$ is the deviation of perfusion index at checkpoint $i$ with respect to an initial measurement of perfusion index (PI). In some embodiments, step (d) comprises determining the plasma dilution efficacy according to equation [13]: $cPDE_i = (cPD_i + 1) \cdot (cPD_{i-1} + 1)^{-1} - 1$ [13] where $cPDE_i$ is plasma dilution efficacy of an observation period $i$; $cPD_i$ is plasma dilution after observation period $i$, and $cPD_{i-1}$ is plasma dilution after a preceding observation period. In ome embodiments, step (e) comprises determining the perfusion index efficacy according to equation [4]: $PIE_i = (PID_i + 1) \cdot (PID_{i-1} + 1)^{-1} - 1$ [14] where $PIE_i$ is plasma dilution efficacy of an observation period $i$; $PID_i$ is perfusion index deviation after observation period number $i$; and $PID_{i-1}$ is perfusion index deviation after a preceding observation period. In some embodiments, if $cPDE_i$ is greater than $PIE_i$, the step (f) comprises determining the total hemoglobin adjustment coefficient according to equation [15]: $k_i = (cPD_i + 1) \cdot (PIE_i + cPDE_i + 1)^{-1} - 1$ [15] where $k_i$ is the total hemoglobin adjustement coefficient $k$ for observation period $i$; $cPD_i$ is plasma dilution after observation period $i$, $PIE_i$ is perfusion index deviation efficacy in the observation period $i$; and $PDE_i$ is plasma dilution efficacy in the observation period $i$. In some embodiments, if $PIE_i$ is greater than $cPDE_i$, then step (f) comprises determining the total hemoglobin adjustment coefficient according to equation [16]: $k_i = (cPD_i + 1) \cdot (PIE_i - cPDE_i + 1)^{-1} - 1$ [16] where $k_i$ is the total hemoglobin adjustement coefficient $k$ for observation period $i$; $cPD_i$ is plasma dilution after observation period $i$, $PIE_i$ is perfusion index deviation efficacy in the observation period $i$; and $PDE_i$ is plasma dilution efficacy in the observation period $i$. In some embodiments, step (g) comprises determining the adjusted deviation of total hemoglobin according to equation [17]: $corPD_i = cPD_i - k_i \cdot cPD_i + SpHb_i \cdot Hb_0^{-1} - 1$ [17] where $corPD_i$ is adjusted deviation of a noninvasively obtained total hemoglobin measurement (SpHb) during observation period $i$; $cPD_i$ is a deviation of non-adjusted total hemoglobin in observation period $i$; $k_i$ is a total hemoglobin adjustement coefficient $k$ of the observation period $i$, $SpHb_i$ is a non-adjusted total hemoglobin measurement after observation period $i$; and $Hb_0$ is a baseline hemoglobin value obtained before the observation period $i$ by one of (i) invasively measuring one of arterial and venous hemoglobin concentration and (ii) noninvasively measuring total hemoglobin. In some embodiments, step (h) comprises adjusting at least one of the the noninvasively obtained total hemoglobin measurements obtained in (a) according to equation [18]: $corSpHb_i = SpHb_0 \cdot ((corPD_i \cdot (1 - Hct_0) + 1))^{-1}$ [18] where $corSpHb_i$ is an adjusted total hemoglobin value for observation period $i$; $SpHb_0$ is a total hemoglobin rmeasurement obtained at an initial baseline; $corPD_i$ is an adjusted deviation of SpHb during observation period $i$; and $Hct_0$ is a baseline hematocrit value. In some embodiments, $corPD_i$ is an adjusted deviation of SpHb during a calibration protocol. In some embodiments, $corPD_i$ is an adjusted deviation of SpHb during a calibration protocol. In some embodiments, the adjusted deviation of total hemoglobin determined in step (g) is determined for a period during a previousl calibration protocol when a subject is in a steady-state condition. In some embodiments, the invaseively obtained hemoglobin concentration measurement is one of an arterial and venous hemoglobin concentration measurement.

In some embodiments, disclosed is a method for adjusting a noninvasively measured total hemoglobin measurement, the method comprising: (a) noninvasively obtaining a plurality of sets of measurements obtained over a period of time, each of the sets including a total hemoglobin measurement and a perfusion index measurement; (b) determining a plasma dilution based on the noninvasively obtained total hemoglobin measurements; (c) determining a perfusion index deviation based on the noninvaseively obtained perfusion index measurements; (d) determining a predicted arterial plasma dilution based on the plasma dilution determined in (b) and the perfusion index deviation determined in (c); (e) adjusting at least one of the the noninvasively obtained total hemoglobin measurements obtained in (a) based on the predicted arterial plasma dilution determined in (d). In some embodiments, step (d) comprises determining a predicted arterial plasma dilution based on the plasma dilution determined in (b) and the perfusion index deviation determined in (c) according to equation [19]: $aPD_i = (2 \cdot cPD_i + 1) \cdot (PID_i + 2)^{-1}$ [19] where $aPD_i$ is a predicted arterial plasma dilution for observation period $i$ with respect to an invasively determined initial baseline arterial hemoblogin concentration value; $cPD_i$ is a deviation of non-adjusted total hemoglobin in observation period $i$; and $PID_i$ is perfusion index deviation during observation period number $i$. In some embodiments, step (e) comprises adjusting at least one of the the noninvasively obtained total hemoglobin measurements obtained in (a) according to equation [20]: $corSpHb_i = SpHb_0 \cdot ((aPD_i \cdot (1 - Hct_0) + 1))^{-1}$ [20] where $corSpHb_i$ is an adjusted total hemoglobin value for observation period $i$; $SpHb_0$ is a total hemoglobin rmeasurement obtained at an initial baseline; $aPD_i$ is a predicted arterial plasma dilution for observation period $i$ with respect to an invasively determined initial baseline arterial hemoblogin concentration value; and $Hct_0$ is a baseline hematocrit value.

In some embodiments, disclosed is a method for determining the baseline state of the whole-body interstitial hydration (diagnostic mVLT or DmVLT) of a subject comprising: (a) quantifying the subject's initial baseline generic target parameters, wherein the derivative target parameter(s) are determined from the generic target parameter(s) by the Bolus Induced Response of Deviations (BIRD) mathematical model set forth in the specification and figures of reference {18}, the baseline generic target parameters including a noninvasively obtained total hemoglobin measurement adjusted according to claim 1; (b) intravenously administering to the subject an iso-oncotic, iso-osmotic non-cellular liquid at the highest safe rate over a period of 2-5 minutes, wherein the total volume administered is 1.5 to 2.5 ml per kg of the subject's lean body mass; (c) quantifying the subject's generic target parameter(s) after a period of 5 minutes from the end of step (b), but before 6 minutes from the end of step (b) without further intravenous administration of a liquid to the subject; (d) determining by formulae the derivative target parameter(s) for the respective generic target parameter(s) obtained in steps (a), and (c); (e) intravenously administering to the subject an iso-oncotic, iso-osmotic non-cellular liquid at the highest safe rate over a period of 2-5 minutes, wherein the total volume administered is 1.5 to 2.5 ml per kg of the subject's lean body mass; (f) quantifying the subject's acute residual generic target parameter(s) after a period of 5 minutes from the end of step (b), but before 6 minutes from the end of step (e), without further administration of liquid to the subject; (g) determining by formulae the derivative target parameter(s) of the respective generic target parameter(s) obtained in steps (e) and (g); (h) comparing the derivative target parameter(s) determined in step (d) and step (g) to a specific pattem(s) of the diagnostic criteria set forth in the specification; and (i) iteratively repeating steps (e) through (h) until the diagnosis of baseline interstitial hydration status is derived by fitting the dynamics of the derivative target parameter(s) to a specific pattem(s) of the diagnostic criteria set forth in the specification.

In some embodiments, disclosed is a device comprising: a noninvasive blood hemoglobin concentration sensor attached to a computing apparatus so as to provide blood hemoglobin concentration input to the computing apparatus; the computing apparatus; an intravenous fluid pump controller which is attached to, and controlled by output from, the computing apparatus, wherein total hemoglobin measurements obtained by the noninvasive blood hemoglobin concentration sensor are adjusted by the computing apparatus according to the method of claim 1, and wherein the output is related to the input by the BIRD algorithm set forth in the specification and figures.

In some embodiments, disclosed is a device comprising: a noninvasive blood hemoglobin concentration sensor attached to a computing apparatus comprising a memory so as to provide total hemoglobin measurements input to the computing apparatus; the computing apparatus comprising the memory, which memory is communicatively coupled to one or more processors, the memory comprising at least one sequence of instructions which when executed by the processor causes the processor to perform the method of claim 1 so as to provide an output to an intravenous fluid pump controller; an intravenous fluid pump controller which is attached to, and controlled by output from, the computing apparatus, wherein total hemoglobin measurements obtained by the noninvasive blood hemoglobin concentration sensor are adjusted by the computing apparatus according to the method of claim 1.

In some embodiments, disclosed is a method for adjusting a haemoglobin measurement, comprising: (a) receiving, by a processor, a pluraltiy of arterial hemoglobin input data relating to arterial hemoglobin concentrations and a plurality of capillary hemoglobin input data relating to capillary hemoglobin concentrations; (b) calculating, by the processor, a statistical bias based on the pluraltiy of arterial hemoglobin input data and the plurality of capillary hemoglobin input data; and (c) performing, by the processor, a calibration to calculate a predicted arterial hemoglobin measurement based on the capillary hemoglobin input data. In some embodiments, the pluraltiy of arterial hemoglobin input data comprise arterial hemoglobin concetration measurements and the pluraltiy of capilary hemoglobin input data comprise capilary hemoglobin concentraion measurements, and wherein the calculation comprises calculating a plurality of GAP values, wherein each of the plurlaity of GAP values is equal to a difference between an arterial hemoglobin concentration measurement and a capillary hemoglobin concentration measurement. In some embodiments, the method comprises a dilution guided correction method (DGC method) comprising of three models and the calibration protocol. In some embodiments one of the three models comprises a dynamic gap model for a mathematical modelling of changes in a plurality of GAP values, wherein each of the plurality of GAP values is equal to a difference between an arterial hemoglobin concentration measurement and a capillary hemoglobin concentration measurement. In some embodiments, one of the three models comprises a transcapillary fluid reflux model configured to monitor a hydration status and a transcapillary fluid movement. In some embodiments, the calibration comprises determining a ratio of GAPs (ROG) based on a first GAP values and a second GAP value, wherein each of the first and second GAP values is equal to a difference between an arterial hemoglobin concentration measurement and a capillary hemoglobin concentration measurement, and wherein the ROG equals the ratio of the first GAP value to the second GAP value. In some embodiments, the calibration comprises determining a ratio of perfusion indexes equal to the ratio of a first perfusion index value to a second index value. In some embodiments, the calibration comprises determining a ratio of FAR values equal to a first FAR value after a deviationof parameters in the calibration protocol to a baseline FAR value in the calibration protocol. In some embodiments, the method comprises calculating a PI-adjustment coefficient based on a ratio of GAPs and on a ratio of perfusion indexes.

In some embodiments, disclosed is a method for adjusting a haemoglobin measurement, comprising: (a) receiving, by a processor, a pluraltiy of arterial hemoglobin input data comprising a pluraltiy of arterial hemoglobin concentration measurements and a plurality of capillary hemoglobin input data comprising a plurality of capillary hemoglobin concentration measurements; (b) receiving, by the processor, a plurality of perfusion index (PI) data comprising PI measurements; (c) calculating, by the processor, a plurality of GAP values, wherein each of the plurlaity of GAP values is equal to a difference between one of the pluraltiy of arterial hemoglobin concentration measurements and one of the plurality of capillary hemoglobin concentration measurements; (d) performing, by the processor, a calibration based on the pluraltiy of arterial hemoglobin concentration measurements, the plurality of capillary hemoglobin concentration measurements, and the pluraltiy of PI measurements; and (e) calculating, by the processor, a predicted arterial hemoglobin measurement using the calibration. In some embodiments, each of the plurality of GAP values comprises a Fahraeus effect related deviation (tGAP) and a transcapillary fluid filtration-absorbtion ratio (FAR) related GAP (fGAP), wherein tGAP related deviations in capillary hemoglobin are reflected in the plurality of perfusion index measurements. In some embodiments, performing the calibration comprises: determining a ratio of GAPs (ROG), wherein ROG equals a ratio of a GAP value determined after a deviation of parameters in the calibration protocol to a baseline GAP value in the calibration protocol; determining a ratio of PIs (ROP), wherein ROP equals a ratio of a PI value after the deviation of parameters in the calibration protocol to a baseline PI value in the calibration protocol; and determining a ratio of transcapillary fluid filtration-absorbtion ratio (FAR) values (ROF), wherein ROF equals a ratio of a FAR value after the deviation of parameters in the calibration protocol to a baseline FAR value in the calibration protocol. In some embodiments, performing the calibration further comprises: determining ROF based on subtracting the absolute value of ROP from the absolute value of ROG. In some embodiment,s the method comprising: determining a plurality of ratio of transcapillary fluid filtration-absorbtion ratio (FAR) values (ROF), wherein each of the plurality of ROF values equals a ratio of a FAR value after a deviation of parameters in the calibration protocol to a baseline FAR value in the calibration protocol; determining an increase of ROF; and based on determining an increase of ROF, determining an increase in a ratio of an amount of fluid entering tissues from capillaries to an amount of fluid that returns to the capillaries. In some embodiments, performing the calibration further comprises determining a PI adjustment-coefficient ($k$), wherein $k$ is equal to the ratio of ROG to ROP. In some embodiments, calculating the predicted arterial hemoglobin measurement using the calibration comprises determining a predicted GAP (pGAP) value. In some embodiments, calculating the predicted aerterial hemoglobin measurement is performed when invasive measurements are not available. In some embodiments, receiving a PI value after a parameter shift; determining a predicted arterial hemoglobin concentration (paHb) value after the parameter shift; determining a GAPi-1 based on subtracting a capillary hemoglobin concentration measurement from the paHb value; determining a ratio of perfusion indexes (ROPi), wherein ROPi is equal to a ratio of a PI value after the parameter shift and a PI value before the parameter shift; and calculating a predicted GAP (pGAPi) based on adding GAPi-1 and the product of $k$ and ROPi. In some embodiment, at least one of the plurality of capillary hemoglobin concentration measurements, at least one of the plurality of arterial hemoglobin concentration measurements, and at least one of the PI measurements are measured substantially simultaneously by at least one sensor. In some embodiments, the deviation of parameters occurs when at least one of the capillary hemoglobin concentration value, the arterial hemoglobin concentration value, and the PI value has deviated from its baseline value. In some embodiments, the deviation of parameters is one of spontaneous or induced. In some embodiments, the inducement is produced by way of a mini volume loading test method. In some embodiments, pGAP comprises a predicted difference between an arterial hemoglobin concentration and a capillary hemoglobin concentration when invasive measurements are not available.

In some embodiments, a system is disclosed comprising: at least one sensor; a computing apparatus communicatively coupled to the at least one sensor, the computing apparatus comprising a memory, a processor, and an application stored in the memory that, when executed by the processor, performs the method of claim 1, wherein the processor receives the pluraltiy of arterial hemoglobin input data, the plurality of capillary hemoglobin input data, and the plurality of perfusion index data from the at least one sensor.

## CLINICAL CASE EXAMPLES

### Investigation n.1

The accuracy and precision of noninvasively measured haemoglobin is affected by fluid administration and anaemia. The impact of single fluid boluses was previously investigated, but the effect of stepwise infusion is unknown. **Objectives:** To study the accuracy and precision of noninvasive haemoglobin measurements during stepwise crystalloid infusion before and after perioperative haemorrhage. **Design:** Open intervention study. Trial registration: Clinicaltrials.gov identifier: NCT01355900. **Setting:** Single university hospital. **Patients:** Forty-three patients scheduled for total knee arthroplasty surgery were enrolled. Thirty-six (31 females and 5 males aged 68.2 $\pm$ 1.8 years) patients completed the study. **Interventions:** Each patient underwent two infusion sessions - one before the start of anaesthesia and one 24 hours later. In both sessions the patients received three mini fluid challenges consisting of a 5 ml kg$^{-1}$ bolus of Ringer's acetate infused over 3-5 min followed by a 5 min period without fluid. The noninvasive and invasive arterial haemoglobin concentrations were determined before and after each mini fluid challenge [FIG.X]. **Main outcome measures:** The accuracy and precision of noninvasive haemoglobin measurements using laboratory arterial haemoglobin measurement as the reference. **Results:** In Bland Altman plots the average bias and precision of 288 paired measurements obtained during the two fluid sessions were 5.0 g l$^{-1}$ and 18.3 g l$^{-1}$ and the lower and upper limits of agreement were -41.5 g l$^{-1}$ and 31.5 g l$^{-1}$, respectively [FIG.X]. Overall, 81 of 144 (56%) postoperative noninvasive readings were within 10 g l$^{-1}$ of the laboratory values compared with 102 of 144 (71%) preoperative measurements ($P$ = 0.014 for the difference) [FIG.X].

Only 33 of 81 (40.7%) noninvasive readings were within 10 g l$^{-1}$ of the laboratory values when they were <100 g l$^{-1}$. The PI did not correlate with changes in Hb gap [FIG.X]. **Conclusions:** Noninvasive haemoglobin measurements are acceptably accurate but lack precision and are not suitable for the monitoring of anaemia or for transfusion decision-making. The stepwise crystalloid infusion decreased accuracy only in the setting of postoperative anaemia. **Discussion:** These findings are in accordance with previous studies where an increased accuracy of SpHb measurement was associated with invasive Hb values below 100 g l$^{-1}$ {4}. It remains unclear why the net impact of our entire postoperative fluid protocol was a decrease in accuracy, but this might be due to the previously suggested{47} possibility of net fluid accumulation in the interstitium because this can reduce the quality of the noninvasive signal. The net increase in bias was associated with a greater decrease in SpHb compared to aHb in both fluid sessions. These findings are in accordance with a previous study in which a 20 ml kg$^{-1}$ bolus infusion of Ringer's acetate significantly increased the bias in healthy volunteers {18}. The similar tendency of a more pronounced haemodilution calculated from noninvasive measures was reported in 40 infusions of 5 or 10 ml kg$^{-1}$ of Ringer's acetate given over 15 min in healthy volunteers {47}. There were speculations that this was attributed to the Fahraeus effect and the transcapillary fluxes of fluid because Pulse CO-oximetry evaluates a mean value of Hb as a function of signals from a network of capillaries whereas an invasive sample is based on the more predictable blood flow in large vessels {13} {21} {26}. The transcapillary reflux of excessive interstitial fluid during overloading infusion of crystalloid serves as an explanation of why SpHb decreased more than large-vessel Hb during stepwise fluid infusion. These findings are not in accordance with previously reported decrease in PI that was associated with an increasing Hb gap during lactated Ringer's infusion in healthy volunteers {18} {47}.

## Investigation n.2

According to the transcapillary reflux model, edema is imminent when at least one of the following three criteria is met during a stepwise crystalloid infusion: the aPDE is negligible (close to zero), the cPDE is minimized (negligible decrease or increase or unchanged), or the acPED changes from positive to negative. **Objectives:** To study the accuracy and precision of noninvasive haemoglobin measurements during stepwise crystalloid infusion before and after perioperative haemorrhage. **Design:** Retrospective observation study. **Setting:** Single university hospital.

**Patients:** Forty-three patients scheduled for total knee arthroplasty surger y were enrolled. Thirty-six (31 females and 5 males) patients who completed the study. The patients were 68.2 ± 1.8 years of age and weighed 31.8 ± 1.2 kg. **Interventions:** Each patient underwent one preoperative infusion session before the start of anaesthesia and surgery. The patients received three mini fluid challenges consisting of a 5 ml kg$^{-1}$ bolus of Ringer's acetate infused over 3-5 min followed by a 5 min period without fluid. The noninvasive and invasive arterial hemoglobin concentrations were determined before and after each mini fluid challenge [FIG.X]. **Main outcome measures:** The aPD, cPD, aPDE, cPDE and acPED. **Results:** We fitted the transcapillary reflux model to the Hb data. The 144 simultaneous measurements of the aHb and SpHb are shown in [FIG.X]. The mean of the aHb was higher than the mean of the SpHb (120.3 ± 0.7 vs. 114.2 ± 1.3, g/L, $p$ = 0.001). The 108 estimates of the aPD and cPD (Fig.5b) were calculated after each of the three mini fluid challenges. The 108 estimates of the aPDE and cPDE (Fig.5c) were calculated after each of the three mini fluid challenges from 108 estimates of the aPD and cPD, respectively. The 108 estimates of the acPED in three consecutive mini fluid challenges (Fig.5d) were calculated from 108 estimates of the aPDE and cPDE. An important observation was that the median acPED was positive in the first and second mini fluid challenges, but became negative in the third. It significantly decreased from positive to negative between the first and third mini fluid challenges (0.042 [-0.025; 0.095] *vs.* - 0.022 [-0.059; 0.023], $p$ = 0.031). The cPDE was considered as minimized, because, in contrast to the aPDE, it did not decrease significantly. **Conclusions:** We have obtained preliminary data to validate the transcapillary reflux model. **Discussion:** The main finding was that, in a preoperative mVLT protocol, it was possible to identify such a point after three relatively small crystalloid boluses separated by 5 minute periods without fluid. We used a non invasive Hb analyzing device, the Radical 7 (Masimo Corporation, Irvine, CA). It measures the SpHb (SpHb® is a registered trademark of Masimo Inc., Irvine, CA) in capillaries of derma under the finger nail. Thus, we hypothesized that the SpHb is equivalent to the capillary Hb suitable for our method. Thus, the SpHb was used as a surrogate for the cHb in calculating the cPD, cPDE and acPED for the detection of imminent edema during the mVLT fluid protocol. The results identified such a point after three mini fluid challenges. Most importantly, that status could be completely detected noninvasively by using the minimization of the cPDE as a marker of capillary reflux. However, the present study has several limitations. It is based on physiological reasoning without any validation of what actually happens in the pertinent tissues. We did not measure interstitial pressures or volumes. In the transcapillary reflux model, we use capillary measurements from a segment of derma under a fingernail. However, we postulate that the derma and the skeletal muscles constitute a major part of body tissue volume and show similar interstitial fluid compliance properties {31}. This approach is in accordance with pharmaco-kinetic and volume kinetic models that also are macro models based on the analysis of arterial or venous samples from a specific site {48}. The previously reported relationship between changes of PI and Hb gap {18} {47} may have had impact on the results. The PI adjustment described by the DGC method described in the present invention could probably have given more focused look at changes in transcapillary fluid movement.

**REFERENCES**

1. Aoyagi T, Fuse M, Kobayashi N, Machida K, Miyasaka K. Multiwavelength pulse oximetry: theory for the future. Anesth Analg 2007; 105:S53-8.

2. Goldman JM, Petterson MT, Kopotic RJ, Barker SJ. Masimo signal extraction pulse oximetry. J Clin Monit Comput 2000; 16:475-83.

3. Mannheimer PD. The light tissue interaction of pulseoximetry. Anesth Analg 2007;105:10-7.

4. Shamir MY, Avramovich A, Smaka T. The Current Status of Continuous Noninvasive Measurement of Total, Carboxy, and Methemoglobin Concentration. Anesth Analg 2012; 114:972-8.

5. Causey MW, Miller S, Foster A, Beekley A, Zenger D, Martin M. Validation of noninvasive hemoglobin measurements using the Masimo Radical-7 SpHb Station. Am J Surg 2011; 201:590-6.

6. Gayat E, Aulagnier J, Matthieu E, Boisson M, Fischler M (2012) Non-invasive measurement of hemoglobin: assessment of two different point-of-care technologies. PLoS ONE 7(1): e30065. doi:10.1371/journal.pone.0030065.

7. Crowley C., Montenegro-Bethancourt G., Solomons N.W., Schumann K. Validity and correspondence of non-invasively determined hemoglobin concentrations by two trans-cutaneous digital measuring devices. Asia Pac J Clin Nutr. 2012; 21(2):191-200.

8. Perfusion Index. The whitepaper from Masimo Corp. http://www.masimo.com/pdf/whitepaper/LAB3410F.pdf

9. Hales JR, Stephens FR, Fawcett AA, et al. Observations on a new non-invasive monitor of skin blood flow. Clinical and experimental pharmacology and physiology. 1989;16:403-415.

10. Applegate RL 2nd, Barr SJ, Collier CE, Rook JL, Mangus DB, Allard MW. Evaluation of pulse cooximetry in patients undergoing abdominal or pelvic surgery. Anesthesiology 2012; 116(1):65-72.

11. Butwick A, Hilton G, Carvalho B. Non-invasive haemoglobin measurement in patients undergoing elective Caesarean section. British Journal of Anaesthesia 2012; 108 (2):271-7.

12. Gayat E, Bodin A, Fischler M. Instability in non-invasive haemoglobin measurement: a possible influence of oxygen administration. Acta Anaesthesiol Scand 2011; 55(7):902.

13. Naftalovich R, Naftalovich D. Error in noninvasive spectrophotometric measurement of blood hemoglobin concentration under conditions of blood loss. Med Hypotheses 2011;77(4):665-7.

14. Goldsmith LH, Cokelet GR, Gaehtgens P. Robin Fahraeus: evolution of his concepts in cardiovascular physiology. Am J Physiol 1989; 257: H1005-15.

15. Fahraeus R. The suspension stability of the blood. Physiol Rev 1929; 9: 241-4.

16. O'Reilly M. E-Letter published January 26, 2012. Response to: Non-invasive haemoglobin measurement in patients undergoing elective Caesarean section. British Journal of Anaesthesia 2012; 108 (2):271-7.

17. Gomez Diago L., Soliveres J., Balaguer J., Hernández M.J., Sánchez A., Solaz C. Masimo Radical-7 Continuous Hemoglobin Measurement Error Grid Approach Results. EJA Supplement. 50, 2012; 29: 47.

18. Bergek C, Zdolsek JH, Hahn RG. Accuracy of noninvasive haemoglobin measurement by pulse oximetry depends on the type of infusion fluid. Eur J Anaesthesiol. 2012; 29: 586-592.

19. PCT International Application No. PCT/US2011/057362, filed October 21, 2011 and titled "METHOD FOR EVALUATING AND MODIFYING THE STATE OF HYDRATION OF A SUBJECT" NEEDS UPDATE SINCE US NONPROVISIONAL WAS FILED: Non-provisional US patent application: Method for evaluating state of hydration of a subject. Dkt. No. 81281-A-PCT.

20. Andrijauskas A, Svensen CH, Ivaskevicius J, Porvaneckas N, Kvederas G, Andrijauskas P. Clinical Interpretation of Noninvasive Hemoglobin (SpHb) Revised: Single Capillary-Bed rather than Arterial Hemoglobin. EJA 2012; 29: May 2012 Supplement: 47 (Abs 3AP4-5).

21. Sjostrand F, Rodhe P, Berglund E, Lundstrom N, Svensen C. The use of a noninvasive hemoglobin monitor for volume kinetic analysis in an emergency room setting. Anesth Analg. 2013; 116: 337-342.

22. Svensen CH, Rodhe PM, Olsson J, Borsheim E, Aarsland A, Hahn RG. Arteriovenous differences in plasma dilution and the distribution kinetics of lactated Ringer's solution. Anesth Analg 2009; 108:128 -33.

23. Mokken FC, et al. Differences in peripheral arterial and venous hemorheologic parameters. Ann Hematol 1996; 73:135-137.

24. Yang ZW, et al. Comparison of blood counts in venous, fingertip, and arterial blood and their measurement variation. Clin. Lab. Haem. 2001;23:155-159.

25. Markevicius V, Andrijauskas A, Navikas D, Svensen CH, Porvaneckas N, Andriukaitis D, Kvederas G, Cincikas D, Andrijauskas P. Statistically biased calibration method for the real-time adjustment of noninvasive haemoglobin measures in semi-automated infusion system. Electronics and Electrical Engineering; [In Press] 2013.

26. Andrijauskas A, Svensen C, Ivaskevicius J, Porvaneckas N, Kvederas G, Marmaite U. Goal directed fluid therapy revised: indirect monitoring of interstitial fluid accumulation during mini fluid challenges with crystalloids. The Open Conference Proceedings Journal 3: 42-51, 2012.

27. Hamilton MA, Cecconi M, Rhodes A. A systematic review and meta-analysis on the use of preemptive hemodynamic intervention to improve postoperative outcomes in moderate and high-risk surgical patients. Anesth Analg 2011; 112:1392-402.

28. Zander, R. Infusion fluids: why should they be balanced solutions? E. J. P. Practice., 2006, 12, 60-62.

29. Boulpaep, E.L. In: Medical physiology; Boron, W.F.; Boulpaep, E.L., Eds.; Saunders: Philadelphia, 2003; pp. 463-475.

30. Aukland, K.; Reed, R.K. Interstitial lymphatic mechanisms in the control of extracellular fluid. Physiol. Rev., 1993, 73(1), 1-78.

31. Wiig, H.; Rubin, K.; Reed, R.K. New active role of the interstitium in control of interstitial fluid pressure: potential therapeutic consequences. Acta. Anaesthesiol. Scand., 2003; 47(2):111-121.

32. Boulpaep, E.L. In: Medical physiology; Boron, W.F.; Boulpaep, E.L., Eds.; Saunders: Philadelphia, 2003; pp. 475-477.

33. Boulpaep, E.L. In: Medical physiology; Boron, W.F.; Boulpaep, E.L., Eds.; Saunders: Philadelphia, 2003; pp. 425-426

34. Holte, K.; Sharrock, N.E.; Kehlet, H. Pathophysiology and clinical implications of perioperative fluid excess. Br. J. Anaesth., 2002; 89:622-32.

35. Arieff, A.I. Fatal postoperative pulmonary edema. Pathogenesis and literature review. Chest., 1999; 115: 1371-1377.

36. US Patent: A.Andrijauskas. Systems and method for homeostatic blood states. Issued on 2010-08-31. Patent number US 7,788,045 B2.

37. Hahn RG, Andrijauskas A, Drobin D, Svensen C, Ivaskevicius J. A volume loading test for the detection of hypovolaemia and dehydration. Medicina (Kaunas) 2008; 44(12):953-959.

38. Hamilton MA, Cecconi M, Rhodes A. A systematic review and meta-analysis on the use of preemptive hemodynamic intervention to improve postoperative outcomes in moderate and high-risk surgical patients. Anesth Analg 2011; 112:1392-402.

39. Andrijauskas A, Svensen CH, Ivaskevicius J. Minimum volume loading test to evaluate hydration in healthy volunteers. Annual Meeting of International Anesthesia Research Society (IARS) in Vancouver, Canada, May 21-23, 2011. Final Supplement to Anaesth Analg 2011; 112(5): S-234. http://www.iars.org/abstracts/11_Abstract_ Supplement_FINAL.pdf

40. Andrijauskas A, Svensen CH, Ivaskevicius J. Minimum volume loading test to evaluate hydration in patients. Annual Meeting of International Anesthesia Research Society (IARS) in Vancouver, Canada, May 21-23, 2011. Final Supplement to Anaesth Analg 2011; 112(5): S-232. http://www.iars.org/abstracts/11_Abstract_Supplement_ FINAL.pdf

41. Andrijauskas A, Svensen CH, Ivaskevicius J, Porvaneckas N, Kvederas G, Andrijauskas P. Clinical Interpretation of Noninvasive Hemoglobin (SpHb) Revised: Single Capillary-Bed rather than Arterial Hemoglobin. EJA 2012; 29: May 2012 Supplement: 47 (Abstract 3AP4-5). http://www .euroanaesthesia.org/sitecore/content/Publications/~/media/Files/Publications/Abstract%20Books/ESA2012 PRIN T.ashx

42. Cannesson M, Pestel G, Ricks C, Hoeft A, Perel A. Hemodynamic monitoring and management in patients undergoing high risk surgery: a survey among North American and European anesthesiologists. Crit Care 15: R197, 2011.

43. Rinehart J, Liu N, Alexander B, Cannesson M. Closed-Loop Systems in Anesthesia: Is There a Potential for Closed-Loop Fluid Management and Hemodynamic Optimization? Anesth Analg, 2012; 114:130-143.

44. Hamilton MA, Cecconi M, Rhodes A. A systematic review and meta-analysis on the use of preemptive hemodynamic intervention to improve postoperative outcomes in moderate and high-risk surgical patients. Anesth Analg 112: 1392-1402, 2011.

45. Decision Support for Hemodynamic Management: From Graphical Displays to Closed Loop Systems. Michard F. Anesth Analg. 2013 Feb 28. [Epub ahead of print]

46. Sia ATH, Tan HS, Sng BL. Closed-loop double-vasopressor automated system to treat hypotension during spinal anaesthesia for caesarean section: a preliminary study. Anaesthesia 2012; 67:1348-55.

47. Hahn RG, Li Y, Zdolsek J. Non-invasive monitoring of blood haemoglobin for analysis of fluid volume kinetics. Acta Anaesthesiol Scand. 2010; 54: 1233-1240.

48. Hahn RG (2009) Volume kinetics for infusion fluids. Anesthesiology 113:470-481.

**Claims**

1.  A method for adjusting a haemoglobin measurement, comprising:

    (a) receiving, by a processor, a plurality of arterial hemoglobin input data comprising a plurality of arterial hemoglobin concentration measurements and a plurality of capillary hemoglobin input data comprising a plurality of capillary hemoglobin concentration measurements;
    (b) receiving, by the processor, a plurality of perfusion index (PI) data comprising PI measurements;
    (c) calculating, by the processor, a plurality of GAP values, wherein each of the plurality of GAP values is equal to a difference between one of the plurality of arterial hemoglobin concentration measurements and one of the plurality of capillary hemoglobin concentration measurements;
    (d) performing, by the processor, a calibration based on the plurality of arterial hemoglobin concentration measurements, the plurality of capillary hemoglobin concentration measurements, and the plurality of PI measurements; and
    (e) calculating, by the processor, a predicted arterial hemoglobin measurement using the calibration.

2.  The method of claim 1, wherein each of the plurality of GAP values comprises a Fahraeus effect related deviation (tGAP) and a transcapillary fluid filtration-absorbtion ratio (FAR) related GAP (fGAP), wherein tGAP related deviations in capillary hemoglobin are reflected in the plurality of perfusion index measurements.

3.  The method of claim 1, wherein performing the calibration comprises:

    determining a ratio of GAPs (ROG), wherein ROG equals a ratio of a GAP value determined after a deviation of parameters in the calibration protocol to a baseline GAP value in the calibration protocol;
    determining a ratio of PIs (ROP), wherein ROP equals a ratio of a PI value after the deviation of parameters in the calibration protocol to a baseline PI value in the calibration protocol; and
    determining a ratio of transcapillary fluid filtration-absorption ratio (FAR) values (ROF), wherein ROF equals a ratio of a FAR value after the deviation of parameters in the calibration protocol to a baseline FAR value in the calibration protocol.

4.  The method of claim 3, wherein performing the calibration further comprises:

    determining ROF based on subtracting the absolute value of ROP from the absolute value of ROG.

5.  The method of claim 1, further comprising:

    determining a plurality of ratio of transcapillary fluid filtration-absorption ratio (FAR) values (ROF), wherein each of the plurality of ROF values equals a ratio of a FAR value after a deviation of parameters in the calibration protocol to a baseline FAR value in the calibration protocol;
    determining an increase of ROF; and
    based on determining an increase of ROF, determining an increase in a ratio of an amount of fluid entering tissues from capillaries to an amount of fluid that returns to the capillaries.

6.  The method of claim 3, wherein performing the calibration further comprises determining a PI adjustment-coefficient (*k*), wherein *k* is equal to the ratio of ROG to ROP.

7.  The method of claim 1, wherein calculating the predicted arterial hemoglobin measurement using the calibration comprises determining a predicted GAP (pGAP) value.

8.  The method of claim 1, wherein calculating the predicted aerterial hemoglobin measurement is performed when invasive measurements are not available.

9.  The method of claim 6, further comprising:

    receiving a PI value after a parameter shift;
    determining a predicted arterial hemoglobin concentration (paHb) value after the parameter shift;
    determining a GAPi-1 based on subtracting a capillary hemoglobin concentration measurement from the paHb value;

determining a ratio of perfusion indexes (ROPi), wherein ROPi is equal to a ratio of a PI value after the parameter shift and a PI value before the parameter shift; and

calculating a predicted GAP (pGAPi) based on adding GAPi-1 and the product of $k$ and ROPi.

10. The method of claim 1, wherein at least one of the plurality of capillary hemoglobin concentration measurements, at least one of the plurality of arterial hemoglobin concentration measurements, and at least one of the PI measurements are measured substantially simultaneously by at least one sensor.

11. The method of claim 3, wherein the deviation of parameters occurs when at least one of the capillary hemoglobin concentration value, the arterial hemoglobin concentration value, and the PI value has deviated from its baseline value.

12. The method of claim 11, wherein the deviation of parameters is one of spontaneous or induced.

13. The method of claim 12, wherein the inducement is produced by way of a mini volume loading test method.

14. The method of claim 7, wherein pGAP comprises a predicted difference between an arterial hemoglobin concentration and a capillary hemoglobin concentration when invasive measurements are not available.

15. A system comprising:

at least one sensor;
a computing apparatus communicatively coupled to the at least one sensor, the computing apparatus comprising a memory, a processor, and an application stored in the memory that, when executed by the processor, performs the method of claim 1, wherein the processor receives the plurality of arterial hemoglobin input data, the plurality of capillary hemoglobin input data, and the plurality of perfusion index data from the at least one sensor.

## FIG. 1

**Rainbow® Adhesive Sensors** (R25; for >30kg adults)

**Rainbow® ReSposable Sensors** (for >30kg adults)

## FIG. 2

## FIG. 3

A          B          C

## FIG. 4

**FIG. 5**

**FIG. 6**

Blood vessels with radius > 500 micrometers

f-ratio 1  f-ratio 2  f-ratio 3  f-ratio 4
f-ratio 5

Arteries  Capillaries  Veins

Capillaries r = 3 µm

First order arteriole r = 30 µm
Second order arteriole
Third order arteriole
Fourth order arteriole r = 5 µm

Filtration-absorption ratio (FAR)

First order venule r = 30 µm
Second order venule
Third order venule
Fourth order venule r = 5 µm

Capillary bed

f-ratio 1 > f-ratio 2 > f-ratio 3 > f-ratio 4 > f-ratio 5

**Fig. 7**

(a)

(b)

(c)

(d)

(e)

(f)

**Fig. 8**

○ --- end of a brisk isoosmotic crystalloid infusion

☐ --- after 5 min period without fluids that follows the end of a crystalloid infusion

● --- after 20 min period without fluids that follows the end of a crystalloid infusion (every infusion in VLT or last infusion in mVLT)

**(b)**

**p.d.u.** -- procedure defined unit (Confirmed minutes for the meeting in Uppsala 2008-10-23 – 25 (Report). Committee and Subcommittee on Nomenclature, Properties and Units (SC-C-NPU), IUPAC–IFCC. 2008.)

**Figure 8**. A fluid challenge is defined as a fluid bolus followed by its corresponding time without fluid. Thick lines are showing plasma dilution during bolus infusions and thin lines are showing plasma dilution during periods without fluid infusion. Solid lines reflect dehydrated subjects while broken lines reflect normohydrated subjects. The VLT test will show similar plasma dilution peaks at the end of infusion in both hydrated states but show a difference twenty minutes after infusion. The mVLT test, however, will distinguish between these two states earlier, after two challenges in the dehydrated state and after three fluid challenges in the normohydrated state. The dilution plateau is a state where plasma dilution is equal after two consecutive mini fluid challenges.

**Fig. 9**

(a)

Patterns of radius difference

● Mean radius difference between a large artery and a mixture of *macro-* capillaries
○ Mean radius difference between a large artery and a mixture of *micro* -capillaries
Large artery is any artery on the way from the heart to a capillary tree.
Capillaries are large (macro-) and small (micro-) capillaries.
━━ Macro-capillaries (venule, arteriole and metarteriole)
━━ Micro-capillaries (true capillaries)

(b)

Fig. 10

Positive sGAP

Positive mGAP

Hb difference (GAP)

0

Relative number of capillaries

0    1    2    3    4    5    6    7    8

Patterns of radius difference

△ mGAP -- mean Hb difference between a large artery and a mixture of *macro* -capillaries

▲ sGAP -- mean Hb difference between a large artery and a mixture of *micro* -capillaries

● Mean radius difference between a large artery and a mixture of *macro-* capillaries

○ Mean radius difference between a large artery and a mixture of *micro* -capillaries

━ Macro-capillaries (venule, arteriole and metarteriole do not participate in transcapillary fluid exchange; the mGAP is constant for a specific radius difference and dependent solely on Fahraeus effect)

── Micro-capillaries ( these 'true capillaries' participate in transcapillary fluid exchange; the sGAP is dynamic - it changes with shifts in transcapillary filtation absorption ratio while being independent from Fahraeus effect)

**Fig. 11**

Mean radius difference between a large artery and
a mixture of all types of capillaries

Relative number of capillaries

Patterns of radius difference

● Mean radius difference between a large artery and a mixture of micro-capillaries
○ Mean radius difference between a large artery and a mixture of micro-capillaries
▬ Macro-capillaries (venule, arteriole and metarteriole)
━ Micro-capillaries (true capillaries)

Fig. 12

☐ cGAP -- the Hb difference between a large artery (aHb) and a mixture of all types of capillaries (SpHb), where SpHb = 0.5*(sHb + mHb) and cGAP = 0.5*(sGAP + mGAP)

△ mGAP -- mean Hb difference between a large artery and a mixture of *macro* -capillaries

▲ sGAP -- mean Hb difference between a large artery and a mixture of *micro* -capillaries

● Mean radius difference between a large artery and a mixture of micro-capillaries

○ Mean radius difference between a large artery and a mixture of micro-capillaries

▬ Macro-capillaries (venule, arteriole and metarteriole do not participate in transcapillary fluid exchange; the mGAP is constant for a specific radius difference and dependent solely on Fahraeus effect)

▬ Micro-capillaries (these 'true capillaries' participate in transcapillary fluid exchange; the sGAP is dynamic - it changes with shifts in transcapillary filtation absorption ratio while being independent from Fahraeus effect)

**Fig. 13**

Light absorbed by haemoglobin in macro-capillaries (mHb)

Light absorbed by haemoglobin in micro-capillaries (sHb)

**Fig. 14**

☐   cGAP -- the Hb difference between a large artery (aHb) and a mixture of all types of capillaries (SpHb), where SpHb = 0.5*(sHb + mHb) and cGAP = 0.5*(sGAP + mGAP)

△   mGAP -- mean Hb difference between a large artery and a mixture of *macro* -capillaries

▲   sGAP -- mean Hb difference between a large artery and a mixture of *micro* -capillaries

●   Mean radius difference between a large artery and a mixture of micro-capillaries

○   Mean radius difference between a large artery and a mixture of micro-capillaries

▬   Macro-capillaries (venule, arteriole and metarteriole do not participate in transcapillary fluid exchange; the mGAP is constant for a specific radius difference and dependent solely on Fahraeus effect)

━   Micro-capillaries (these 'true capillaries' participate in transcapillary fluid exchange; the sGAP is dynamic - it changes with shifts in transcapillary filtation absorption ratio while being independent from Fahraeus effect)

Fig. 15

☐ cGAP -- the Hb difference between a large artery (aHb) and a mixture of all types of capillaries (SpHb), where SpHb = 0.5*(sHb + mHb) and cGAP = 0.5*(sGAP + mGAP)

△ mGAP -- mean Hb difference between a large artery and a mixture of *macro* -capillaries

▲ sGAP -- mean Hb difference between a large artery and a mixture of *micro* -capillaries

▬▬▬ Macro-capillaries (venule, arteriole and metarteriole do not participate in transcapillary fluid exchange; the mGAP is constant for a specific radius difference and dependent solely on Fahraeus effect)

━━━ Micro-capillaries (these 'true capillaries' participate in transcapillary fluid exchange; the sGAP is dynamic - it changes with shifts in transcapillary filtation absorption ratio while being independent from Fahraeus effect)

**FAR** -- transcapillary filtration absorption ratio.

Fig. 16

cGAP -- the Hb difference between a large artery (aHb) and a mixture of all types of capillaries (SpHb), where SpHb = 0.5*(sHb + mHb) and cGAP = 0.5*(sGAP + mGAP)

mGAP -- mean Hb difference between a large artery and a mixture of *macro* -capillaries

sGAP -- mean Hb difference between a large artery and a mixture of *micro* -capillaries

Macro-capillaries (these are micro-vessels that do not participate in transcapillary fluid

Micro-capillaries (or 'true capillaries'; these are micro-vessels that participate in transcapillary

**FAR** -- transcapillary filtration absorption ratio.

**Fig. 17**

Gap between arterial Hb and SpHb (cGAP) is equal to zero

cGAP↓↓↓    cGAP↓  cGAP↓

Negative **sGAP**    Positive **mGAP**

aHb↓↓↓ ≈ sGAP↓↓↓    aHb↓ ≈ sGAP↓

Relative number of micro vessels

-4    -3    -2    -1    0    1    2    3    4

Hb difference (GAP)

▨ cGAP -- the Hb difference between a large artery (aHb) and a mixture of all types of capillaries (SpHb), where SpHb = 0.5*(sHb + mHb) and cGAP = 0.5*(sGAP + mGAP)

△ mGAP -- mean Hb difference between a large artery and a mixture of *macro* -capillaries

▲ sGAP -- mean Hb difference between a large artery and a mixture of *micro* -capillaries

▬ Macro-capillaries (these are micro-vessels that do not participate in transcapillary fluid exchange - venule, arteriole and metarteriole)

— Micro-capillaries (or 'true capillaries'; these are micro-vessels that participate in transcapillary fluid exchange)

**Fig. 18**

Gap between arterial Hb and SpHb (cGAP) is negative

Negative **sGAP**

mGAP↓
≈ PI↑

Relative number of micro vessels

-4   -3   -2   -1   0   1   2   3   4

Hb difference (GAP)

▨ cGAP -- the Hb difference between a large artery (aHb) and a mixture of all types of capillaries (SpHb), where SpHb = 0.5*(sHb + mHb) and cGAP = 0.5*(sGAP + mGAP)

△ mGAP -- mean Hb difference between a large artery and a mixture of *macro* -capillaries

▲ sGAP -- mean Hb difference between a large artery and a mixture of *micro* -capillaries

▬ Macro-capillaries (these are micro-vessels that do not participate in transcapillary fluid exchange - venule, arteriole and metarteriole)

— Micro-capillaries (or 'true capillaries'; these are micro-vessels that participate in transcapillary fluid exchange)

**PI** - perfusion index.

**Fig. 19**

**Fig. 20**

GAP = aHb - cHb    $\Delta GAP_1 = GAP_1 - GAP_0 = 0$    GAP = aHb - cHb    $\Delta GAP_1 = GAP_1 - GAP_0 = 0$

**POSITIVE GAP**                              **NEGATIVE GAP**

(a)                                          (b)

Fig. 21

$$GAP = aHb - cHb \qquad \textbf{POSITIVE GAP}$$

$$\Delta cHb_1 = cHb_1 - cHb_0 = 2 - 7 = (-5)$$

$$\Delta aHb_1 = aHb_1 - aHb_0 = 18 - 20 = (-2)$$

$$\Delta GAP_1 = GAP_1 - GAP_0 = \Delta aHb_1 - \Delta cHb_1 = (-2) - (-5) = 3$$

$$\boxed{GAP_1 = GAP_0 + \Delta aHb_1 - \Delta cHb_1 = 13 + (-2) - (-5) = 16}$$

(a)

$$GAP = aHb - cHb \qquad \textbf{NEGATIVE GAP}$$

$$\Delta cHb_1 = cHb_1 - cHb_0 = 5 - 7 = (-2)$$

$$\Delta aHb_1 = aHb_1 - aHb_0 = 15 - 20 = (-5)$$

$$\Delta GAP_1 = GAP_0 - GAP_1 = \Delta aHb_1 - \Delta cHb_1 = (-5) - (-2) = 3$$

$$\boxed{GAP_1 = GAP_0 + \Delta aHb_1 - \Delta cHb_1 = (-13) + (-2) - (-5) = (-10)}$$

(b)

55

**Fig. 22**

$$GAP_1 = tGAP_1 + fGAP_1$$

**Fig. 23**

$$\Delta GAP_1 = \Delta t GAP_1 + \Delta f GAP_1$$

Fig. 24

POSITIVE GAP

NEGATIVE GAP

**Fig. 25**

Fluid shift: 'IN' -- into the blood stream,
'OUT' -- out of the blood stream.

**A** -- net arterial blood flow of the whole-body,
**V** -- net venous blood flow of the whole-body,
**C** -- net capillary flow of the whole body capillary beds.

**Fig. 26**

Basal renal elimination

OUT

A

IN

Lymph

V

OUT

OUT

OUT

Arteriole

Capillaries

Metarteriole

**Arterio-capillary** dilution difference acDD>0

C

IN

Thirst guided fluid intake

Infusion

Interstitium

Fluid accumulation (compliance ↑)

IN

**Arterio-venous** dilution difference avDD>0

Venule

IN

IN

| Net transcapillary **FAR$_2$** = 3 : 1 | acDD > avDD > 0 |
|---|---|

Fluid shift: 'IN' -- into the blood stream, 'OUT' -- out of the blood stream.

**A** -- net arterial blood flow of the whole-body,
**V** -- net venous blood flow of the whole-body,
**C** -- net capillary flow of the whole body capillary beds.

**Fig. 27**

Net transcapillary **FAR₃ = 1 : 2**    acDD < avDD < 0

--Whole-body inter-compartment fluid shift ('IN' -- into blood stream, 'OUT' -- out of blood stream).

A -- net arterial blood flow of the whole-body,
V -- net venous blood flow of the whole-body,
C -- net capillary flow of the whole body capillary beds.

**Fig. 28**

mini VLT for optimization of hemodilution and hydration status

1st mini fluid challenge

BASELINE: cHb and paHb or aHb

BOLUS infusion: 1.5-5.0 ml/kg over 3 min

5 min steady state without fluids

T1 checkpoint: cHb and paHb or aHb

2nd mini fluid challenge

BOLUS infusion: 1.5-5.0 ml/kg over 3 min

5 min steady state without fluids

T2 checkpoint: cHb and paHb or aHb

acPED<0 and/or other criteria for optimization?

YES

NO

Optimized hemodilution and hydration status

mVLT - mini volume loading test.
cHb - capillary hemoglobin concentration.
aHb - arterial hemoglobin concentration.
paHb - predicted arterial hemoglobin concentration.
acPED - arteriocapillary plasmadilution efficacy difference.

Fig. 29

Mini fluid challenges

■●▬ Arterial plasma dilution (aPD)
■○ ■ ■ ▪ Arterial plasma dilution efficacy (aPDE)

**p.d.u.** -- procedure defined unit (Confirmed minutes for the meeting in Uppsala 2008-10-23 – 25 (Report). Committee and Subcommittee on Nomenclature, Properties and Units (SC-C-NPU), IUPAC–IFCC. 2008.)

**Fig. 30**

p.d.u. -- procedure defined unit (Confirmed minutes for the meeting in Uppsala 2008-10-23 – 25 (Report). Committee and Subcommittee on Nomenclature, Properties and Units (SC-C-NPU), IUPAC–IFCC. 2008.)

## Fig. 31

mVLT+GDT for optimization of circulation, hemodilution and hydration status

FTP<TPA ?

NO — Pressor ON? — YES / NO
YES — FTP>TPH ? — NO / YES → LOWER pressor's dose / STOP pressor

YES — Pressor ON? — YES / NO
FTP<TPL ? — NO Continue pressor / YES START pressor
FTP<TPL ? — NO / YES START pressor

EVALUATE AND ADJUST PRESSOR SUPPORT

BASELINE: FTP, cHb and paHb or aHb

1st mini fluid challenge:
BOLUS infusion: 1.5-5.0 ml/kg over 3 min
5 min steady state without fluids
T1 checkpoint: FTP, cHb and paHb or aHb

EVALUATE AND ADJUST THE PRESSOR SUPPORT

2nd mini fluid challenge:
BOLUS infusion: 1.5-5.0 ml/kg over 3 min
5 min steady state without fluids
T2 checkpoint: FTP, cHb and paHb or aHb

acPED<0 and/or other criteria for optimized hemodilition ?

YES / NO

Presumably optimized hemodilution and hydration status. Continue with pressor support if the optimization of target parameter is not reached. Consider transfusion in case of anemia with signs of tissue hypoxia (anemia intolerance)

mVLT - mini volume loading test.
GDT - conventional algorithm for goal directed fluid therapy.
cHb - capillary hemoglobin concentration.
aHb - invasively measured arterial hemoglobin concentration.
paHb - noninvasively predicted arterial hemoglobin concentration.
acPED - arteriocapillary plasmadilution efficacy difference.
FRP - flow related parameter.
TPA-- target parameter acceptable (normal value)
TPL -- target parameter low (critical low value)
TPH -- target parameter high (critical high value)
Pressor - intravenous infusion of vasoactive drug and/or innotrope.

Fig. 32 (a)

EP 2 700 358 A2

66

aHb - arterial haemoglobin concentration (g/L).
cHb - continuous noninvasively measured capillary hemoglobin concentration (g/L).
Pressor - intravenous infusion of vasoactive drug and/or innotrope.
GDT - conventional algorithm for goal directed fluid therapy.
mVLT - mini volume loading test.
TPA -- target parameter acceptable (normal value)
TPL -- target parameter low (critical low value)
TPH -- target parameter high (critical high value)
PRBC - packed red blood cells for transfusion.
Anemia tolerated - no signs of tissue hypoxia.

Order PRBC from a blood bank

Anemia tolerated? YES / NO

Perform mVLT

Suspected HYPOVOLEMIA? YES / NO

Last mVLT before >15 min ? YES / NO

Anemia tolerated? YES / NO

TRANSFUSE

PRBC allready on site? YES / NO

PRBC allready ordered from a blood bank? YES / NO

aHb >100 ? YES / NO

cHb >110 ? YES / NO

Monitoring cHb

ANEMIA (cHb ≤110 or aHb ≤100) ? YES / NO

Perform mVLT + GDT

Suspected HYPOVOLEMIA? YES / NO

Last mVLT + GDT before >15 min ? YES / NO

START pressor

Increase pressor's DOSE

Pressor ON? YES / NO

TPL? YES / NO

STOP the pressor

LOWER pressor's dose

TPH? YES / NO

Value >TPA? YES / NO

Pressor ON? YES / NO

Perform mVLT + GDT

Need

TPA? YES / NO

Monitoring flow related parameters

**Fig. 32 (b)**

GDT - conventional algorithm for goal directed fluid therapy.

mVLT - mini volume loading test.

TPA-- target parameter acceptable (normal value)

TPL -- target parameter low (critical low value)

TPH -- target parameter high (critical high value)

Pressor - intravenous infusion of vasoactive drug and/or innotrope.

## Fig. 32 (c)

GDT - conventional algorithm for goal directed fluid therapy.
mVLT - mini volume loading test.
aHb - arterial haemoglobin concentration (g/L).
cHb - continuous noninvasive capillary hemoglobin concentration (g/L)
Anemia tolerated - no signs of tissue hypoxia.
PRBC - packed red blood cells for transfusion.

**Fig. 33**

START mini VLT

Baeline SpHb and aHb

1st mini fluid challenge

Bolus infusion 5ml/kg 0.9% NaCl over 3..5 min

5 min steady state without fluids

SpHb and aHb

2nd mini fluid challenge

Bolus infusion 5ml/kg 0.9% NaCl over 3..5 min

5 min steady state without fluids

SpHb and aHb

acPED<=0 ?

No

Yes

IMMINENT EDEMA

aHb - invasively measured arterial hemoglobin concentration
SpHb - noninvasively measured hemoglobin concentration in capillaries
acPED - arterio-capillary plasma diliution efficacy difference

# Fig. 34

*Issued:* 2013-07-25, rev. 08-11

**Fig. 35**

(a)

(b)

**Fig. 36**

(a)

(b)

(c)

**Fig. 37**

(a)

(b)

(c)

**Fig. 38**

**Fig. 39**

(a)

(b)

(c)

**Fig. 40**

Invasive arterial hemoglobin concentration (aHb) and non-invasive capillary hemoglobin (cHb)

**Fig. 41**

Invasive (arterial) and non-invasive arterial (capillary) plasma dilution

**Fig. 42**

**Fig. 43**

Fig. 44

Arterial hemoglobin concentration (aHb) and
Total hemoglobin (SpHb) --
PERI-OPERATIVE (24 HRS)

(1-3) 1st mVLT - mini fluid challenges,
(4-6) 2nd mVLT - mini fluid challenges

A

Arterial hemoglobin concentration (aHb) and
1T CORRECTED Total hemoglobin (SpHb) --
PERI-OPERATIVE (24 HRS)

(1-3) 1st mVLT - mini fluid challenges,
(4-6) 2nd mVLT - mini fluid challenges,

B

**Fig. 45**

The GAP between Arterial hemoglobin concentration (aHb) and Total hemoglobin (SpHb) -- PERI-OPERATIVE (24 HRS)

(1-3) 1st mVLT - mini fluid challenges,
(4-6) 2nd mVLT - mini fluid challenges

**Fig. 46**

EP 2 700 358 A2

**Fig. 47**

86

**Fig. 48**

```
                    ┌──────────────────────┐
                    │        Begin         │
                    └──────────┬───────────┘
                               │
                          ╱─────────╲       False
                         ╱  System   ╲──────────────────────────┐
                         ╲ calibrated?╱                          │
                          ╲─────────╱                            │
                               │                    ┌─────────────────────────────┐
                             True                   │  ┌────────────────────────┐ │
                               │                    │  │    aHb measurement     │ │
        ┌──────────────────►──┤                    │  │    using invasive      │ │
        │                      │                    │  │      method (1)        │ │
        │           ┌──────────────────────┐        │  └────────────────────────┘ │
        │           │    SpHb and PI       │        │  ┌────────────────────────┐ │
        │           │    measurement       │        │  │       Dilution         │ │
        │           │  using non-invasive  │        │  └────────────────────────┘ │
        │           │      method          │        │  ┌────────────────────────┐ │
        │           └──────────┬───────────┘        │  │    aHb measurement     │ │
        │                      │                    │  │    using invasive      │ │
        │           ┌──────────────────────┐        │  │      method (2)        │ │
        │           │   aHb calculation    │        │  └────────────────────────┘ │
        │           │  using statistical   │        │                 Calibration │
        │           │       model          │        └─────────────────────────────┘
        │           └──────────┬───────────┘
        │                      │
        │                 ╱─────────╲       False
        │                ╱   Does    ╲──────────────────────────┐
        │                ╲ aHb>aHb   ╱                          │
        │                 ╲   crit.? ╱                          │
        │                  ╲───────╱                            │
        │                      │                    ┌──────────────────────┐
        │                    True                   │  Alarm and dilution  │
        │           ┌──────────────────────┐        │    termination       │
        │           │      Dilution        │        └──────────┬───────────┘
        │           └──────────┬───────────┘                   │
        │                      │                    ┌──────────────────────┐
        │                      │                    │  Blood or plasma      │
        │                      │                    │  transfusion          │
        │                      │                    └──────────┬───────────┘
        └──────────────────◄──┴───────────────────────────────┘
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61692904 A
- US 2011057362 W
- US 7788045 B2

**Non-patent literature cited in the description**

- **AOYAGI T ; FUSE M ; KOBAYASHI N ; MACHIDA K ; MIYASAKA K.** Multiwavelength pulse oximetry: theory for the future. *Anesth Analg,* 2007, vol. 105, 53-8
- **GOLDMAN JM ; PETTERSON MT ; KOPOTIC RJ ; BARKER SJ.** Masimo signal extraction pulse oximetry. *J Clin Monit Comput,* 2000, vol. 16, 475-83
- **MANNHEIMER PD.** The light tissue interaction of pulseoximetry. *Anesth Analg,* 2007, vol. 105, 10-7
- **SHAMIR MY ; AVRAMOVICH A ; SMAKA T.** The Current Status of Continuous Noninvasive Measurement of Total, Carboxy, and Methemoglobin Concentration. *Anesth Analg,* 2012, vol. 114, 972-8
- **CAUSEY MW ; MILLER S ; FOSTER A ; BEEKLEY A ; ZENGER D ; MARTIN M.** Validation of noninvasive hemoglobin measurements using the Masimo Radical-7 SpHb Station. *Am J Surg,* 2011, vol. 201, 590-6
- **GAYAT E ; AULAGNIER J ; MATTHIEU E ; BOISSON M ; FISCHLER M.** Non-invasive measurement of hemoglobin: assessment of two different point-of-care technologies. *PLoS ONE,* 2012, vol. 7 (1), e30065
- **CROWLEY C. ; MONTENEGRO-BETHANCOURT G. ; SOLOMONS N.W. ; SCHUMANN K.** Validity and correspondence of non-invasively determined hemoglobin concentrations by two trans-cutaneous digital measuring devices. *Asia Pac J Clin Nutr.,* 2012, vol. 21 (2), 191-200
- **HALES JR ; STEPHENS FR ; FAWCETT AA et al.** Observations on a new non-invasive monitor of skin blood flow. *Clinical and experimental pharmacology and physiology,* 1989, vol. 16, 403-415
- **APPLEGATE RL 2ND ; BARR SJ ; COLLIER CE ; ROOK JL ; MANGUS DB ; ALLARD MW.** Evaluation of pulse cooximetry in patients undergoing abdominal or pelvic surgery. *Anesthesiology,* 2012, vol. 116 (1), 65-72
- **BUTWICK A ; HILTON G ; CARVALHO B.** Non-invasive haemoglobin measurement in patients undergoing elective Caesarean section. *British Journal of Anaesthesia,* 2012, vol. 108 (2), 271-7
- **GAYAT E ; BODIN A ; FISCHLER M.** Instability in non-invasive haemoglobin measurement: a possible influence of oxygen administration. *Acta Anaesthesiol Scand,* 2011, vol. 55 (7), 902
- **NAFTALOVICH R ; NAFTALOVICH D.** Error in non-invasive spectrophotometric measurement of blood hemoglobin concentration under conditions of blood loss. *Med Hypotheses,* 2011, vol. 77 (4), 665-7
- **GOLDSMITH LH ; COKELET GR ; GAEHTGENS P.** Robin Fahraeus: evolution of his concepts in cardiovascular physiology. *Am J Physiol,* 1989, vol. 257, H1005-15
- **FAHRAEUS R.** The suspension stability of the blood. *Physiol Rev,* 1929, vol. 9, 241-4
- **O'REILLY M.** Response to: Non-invasive haemoglobin measurement in patients undergoing elective Caesarean section. *British Journal of Anaesthesia,* 26 January 2012, vol. 108 (2), 271-7
- **GOMEZ DIAGO L. ; SOLIVERES J. ; BALAGUER J. ; HERNÁNDEZ M.J. ; SÁNCHEZ A. ; SOLAZ C.** Masimo Radical-7 Continuous Hemoglobin Measurement Error Grid Approach Results. *EJA Supplement,* 2012, vol. 50 (29), 47
- **BERGEK C ; ZDOLSEK JH ; HAHN RG.** Accuracy of noninvasive haemoglobin measurement by pulse oximetry depends on the type of infusion fluid. *Eur J Anaesthesiol.,* 2012, vol. 29, 586-592
- **ANDRIJAUSKAS A ; SVENSEN CH ; IVASKEVICIUS J ; PORVANECKAS N ; KVEDERAS G ; ANDRIJAUSKAS P.** Clinical Interpretation of Noninvasive Hemoglobin (SpHb) Revised: Single Capillary-Bed rather than Arterial Hemoglobin. *EJA,* May 2012, vol. 29, 47
- **SJOSTRAND F ; RODHE P ; BERGLUND E ; LUNDSTROM N ; SVENSEN C.** The use of a noninvasive hemoglobin monitor for volume kinetic analysis in an emergency room setting. *Anesth Analg.,* 2013, vol. 116, 337-342
- **SVENSEN CH ; RODHE PM ; OLSSON J ; BORSHEIM E ; AARSLAND A ; HAHN RG.** Arteriovenous differences in plasma dilution and the distribution kinetics of lactated Ringer's solution. *Anesth Analg,* 2009, vol. 108, 128-33

- **MOKKEN FC et al.** Differences in peripheral arterial and venous hemorheologic parameters. *Ann Hematol,* 1996, vol. 73, 135-137
- **YANG ZW et al.** Comparison of blood counts in venous, fingertip, and arterial blood and their measurement variation. *Clin. Lab. Haem.,* 2001, vol. 23, 155-159
- **MARKEVICIUS V ; ANDRIJAUSKAS A ; NAVIKAS D ; SVENSEN CH ; PORVANECKAS N ; ANDRIUKAITIS D ; KVEDERAS G ; CINCIKAS D ; ANDRIJAUSKAS P.** Statistically biased calibration method for the real-time adjustment of noninvasive haemoglobin measures in semi-automated infusion system. *Electronics and Electrical Engineering,* 2013
- **ANDRIJAUSKAS A ; SVENSEN C ; IVASKEVICIUS J ; PORVANECKAS N ; KVEDERAS G ; MARMAITE U.** Goal directed fluid therapy revised: indirect monitoring of interstitial fluid accumulation during mini fluid challenges with crystalloids. *The Open Conference Proceedings Journal,* 2012, vol. 3, 42-51
- **HAMILTON MA ; CECCONI M ; RHODES A.** A systematic review and meta-analysis on the use of preemptive hemodynamic intervention to improve postoperative outcomes in moderate and high-risk surgical patients. *Anesth Analg,* 2011, vol. 112, 1392-402
- **ZANDER, R.** Infusion fluids: why should they be balanced solutions?. *E. J. P. Practice.,* 2006, vol. 12, 60-62
- **BOULPAEP, E.L.** Medical physiology. Saunders, 2003, 463-475
- **AUKLAND, K. ; REED, R.K.** Interstitial lymphatic mechanisms in the control of extracellular fluid. *Physiol. Rev.,* 1993, vol. 73 (1), 1-78
- **WIIG, H. ; RUBIN, K. ; REED, R.K.** New active role of the interstitium in control of interstitial fluid pressure: potential therapeutic consequences. *Acta. Anaesthesiol. Scand.,* 2003, vol. 47 (2), 111-121
- **BOULPAEP, E.L.** Medical physiology. Saunders, 2003, 475-477
- **BOULPAEP, E.L.** Medical physiology. Saunders, 2003, 425-426
- **HOLTE, K. ; SHARROCK, N.E. ; KEHLET, H.** Pathophysiology and clinical implications of perioperative fluid excess. *Br. J. Anaesth.,* 2002, vol. 89, 622-32
- **ARIEFF, A.I.** Fatal postoperative pulmonary edema. Pathogenesis and literature review. *Chest,* 1999, vol. 115, 1371-1377
- **HAHN RG ; ANDRIJAUSKAS A ; DROBIN D ; SVENSEN C ; IVASKEVICIUS J.** A volume loading test for the detection of hypovolaemia and dehydration. *Medicina,* 2008, vol. 44 (12), 953-959
- **ANDRIJAUSKAS A ; SVENSEN CH ; IVASKEVICIUS J.** Minimum volume loading test to evaluate hydration in healthy volunteers. *Anaesth Analg,* 21 May 2011, vol. 112 (5), S-234, http://www.iars.org/abstracts/11_Abstract_Supplement_FINAL.pdf
- **ANDRIJAUSKAS A ; SVENSEN CH ; IVASKEVICIUS J.** Minimum volume loading test to evaluate hydration in patients. *Anaesth Analg,* 21 May 2011, vol. 112 (5), S-232, http://www.iars.org/abstracts/11_Abstract_Supplement_FINAL.pdf
- **CANNESSON M ; PESTEL G ; RICKS C ; HOEFT A ; PEREL A.** Hemodynamic monitoring and management in patients undergoing high risk surgery: a survey among North American and European anesthesiologists. *Crit Care,* 2011, vol. 15, R197
- **RINEHART J ; LIU N ; ALEXANDER B ; CANNESSON M.** Closed-Loop Systems in Anesthesia: Is There a Potential for Closed-Loop Fluid Management and Hemodynamic Optimization?. *Anesth Analg,* 2012, vol. 114, 130-143
- **HAMILTON MA ; CECCONI M ; RHODES A.** A systematic review and meta-analysis on the use of preemptive hemodynamic intervention to improve postoperative outcomes in moderate and high-risk surgical patients. *Anesth Analg,* 2011, vol. 112, 1392-1402
- **MICHARD F.** Decision Support for Hemodynamic Management: From Graphical Displays to Closed Loop Systems. *Anesth Analg.,* 28 February 2013
- ATH, Tan HS, Sng BL. Closed-loop double-vasopressor automated system to treat hypotension during spinal anaesthesia for caesarean section: a preliminary study. *Anaesthesia,* 2012, vol. 67, 1348-55
- **HAHN RG ; LI Y ; ZDOLSEK J.** Non-invasive monitoring of blood haemoglobin for analysis of fluid volume kinetics. *Acta Anaesthesiol Scand.,* 2010, vol. 54, 1233-1240
- **HAHN RG.** Volume kinetics for infusion fluids. *Anesthesiology,* 2009, vol. 113, 470-481